(19) Europäisches Patentamt / European Patent Office / Office européen des brevets

(11) **EP 4 069 663 B1**

(12) **EUROPEAN PATENT SPECIFICATION**

(45) Date of publication and mention of the grant of the patent:
**13.12.2023 Bulletin 2023/50**

(21) Application number: **20815830.3**

(22) Date of filing: **02.12.2020**

(51) International Patent Classification (IPC):
***C07C 13/54*** *(2006.01)* ***C09K 11/06*** *(2006.01)*
***C07C 13/573*** *(2006.01)* ***H10K 101/20*** *(2023.01)*
***C07C 25/24*** *(2006.01)* ***C07C 211/61*** *(2006.01)*
***H10K 85/60*** *(2023.01)*

(52) Cooperative Patent Classification (CPC):
**C07C 25/24; C07C 13/573; C07C 211/61; H10K 85/615; H10K 85/633;** C07C 2603/18; C07C 2603/24; C07C 2603/94; H10K 50/11; H10K 50/12; H10K 85/1135; H10K 85/654; H10K 85/6572; H10K 2101/20; Y02E 10/549

(86) International application number:
**PCT/EP2020/084257**

(87) International publication number:
**WO 2021/110741 (10.06.2021 Gazette 2021/23)**

(54) **MATERIALS FOR ORGANIC ELECTROLUMINESCENT DEVICES**

MATERIALIEN FÜR ORGANISCHE ELEKTROLUMINESZENTE VORRICHTUNGEN

MATÉRIAUX POUR DISPOSITIFS ÉLECTROLUMINESCENTS ORGANIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.12.2019 EP 19213426**

(43) Date of publication of application:
**12.10.2022 Bulletin 2022/41**

(73) Proprietor: **Merck Patent GmbH**
**64293 Darmstadt (DE)**

(72) Inventors:
- **STENGEL, Ilona**
  **64293 DARMSTADT (DE)**
- **PFLUMM, Christof**
  **64293 DARMSTADT (DE)**
- **MEKIC, Amel**
  **9471 BUCHS (CH)**

(56) References cited:
**WO-A1-2007/105917**
**WO-A2-03/060956**
**US-A1- 2009 256 468**
**US-A1- 2017 200 905**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 069 663 B1

## Description

The present invention relates to a compound of the formula (1), to the use of the compound in an electronic device, and to an electronic device comprising a compound of the formula (1).

The development of functional compounds for use in electronic devices is currently the subject of intensive research. The aim is, in particular, the development of compounds with which improved properties of electronic devices in one or more relevant points can be achieved, such as, for example, power efficiency and lifetime of the device as well as colour coordinates of the emitted light.

In accordance with the present invention, the term electronic device is taken to mean, inter alia, organic integrated circuits (OICs), organic field-effect transistors (OFETs), organic thin-film transistors (OTFTs), organic light-emitting transistors (OLETs), organic solar cells (OSCs), organic optical detectors, organic photoreceptors, organic field-quench devices (OFQDs), organic light-emitting electrochemical cells (OLECs), organic laser diodes (O-lasers) and organic electroluminescent devices (OLEDs).

Of particular interest is the provision of compounds for use in the last-mentioned electronic devices called OLEDs. The general structure and the functional principle of OLEDs are known to the person skilled in the art and are described, for example, in US 4539507.

Further improvements are still necessary with respect to the performance data of OLEDs, in particular with a view to broad commercial use, for example in display devices or as light sources. Of particular importance in this connection are the lifetime, the efficiency and the operating voltage of the OLEDs and also the colour values achieved. In particular, in case of blue-emitting OLEDs, there is potential for improvement with respect to the lifetime, the efficiency of the devices and the colour purity of the emitters.

An important starting point for achieving the said improvements is the choice of the emitter compound and of the host compound employed in the electronic device.

Blue-fluorescent emitters known from the prior art are a multiplicity of compounds. Arylamines containing one or more condensed aryl are known from the prior art. Anthracenes and aminoanthracenes are also well-known materials in the field of OLED. Anthracenes are mostly used as host materials for blue fluorescent OLEDS. Applications of anthracenes as blue emitters are also known, especially derivatives of 9,10-diphenylanthracene as disclosed, for example in J. Mater. Chem. C, 2015, 3, 913. Further anthracene derivatives suitable as OLED materials are disclosed in WO 2007/105917 A1, US 2009/256468 A1 and US 2017/200905 A1.

However, there is still a need for further fluorescent emitters, especially blue-fluorescent emitters, which may be employed in OLEDs and lead to OLEDs having very good properties in terms of lifetime, colour emission and efficiency. More particularly, there is a need for blue-fluorescent emitters combining very high efficiencies, very good life time and suitable colour coordinates as well as high colour purity.

Recently, organic electroluminescent devices having, in the emitting layer, a TADF compound (compounds which exhibit thermally activated delayed fluorescence) or a phosphorescent compound as a sensitizer and a fluorescent compound having high steric shielding with respect to its environment as an emitter have been described (for example in WO2015/135624). This device construction makes it possible to provide organic electroluminescent devices which emit in all emission colours, so that it is possible to use the base structures of known fluorescent emitters which nevertheless exhibit the high efficiency of electroluminescent devices with TADF or phosphorescent compounds. This is also known as hyperfluorescence, when the sensitizer is a TADF compound, and hyperphosphorescence, when the sensitizer is a phosphorescent compound.

TADF compounds are described, for example, in H. Uoyama et al., Nature 2012, vol. 492, 234. TADF compounds are, in general, organic materials in which the energy gap between the lowest triplet state $T_1$ and the first excited singlet state $S_1$ is sufficiently small so that the $S_1$ state is thermally accessible from the $T_1$ state. For quantum-statistical reasons, on electronic excitation in the OLED, 75% of the excited states are in the triplet state and 25% in the singlet state. Since purely organic molecules cannot usually emit efficiently from the triplet state, 75% of the excited states cannot be utilized for emission, which means that it is possible in principle to convert only 25% of the excitation energy to light. If, however, the energy gap between the lowest triplet state and the lowest excited singlet state is sufficiently small, the first excited singlet state of the molecule is accessible from the triplet state by thermal excitation and can be populated thermally. Since this singlet state is an emissive state from which fluorescence is possible, this state can be used to generate light. Thus, in principle, the conversion of up to 100% of the electrical energy to light is possible when purely organic materials are used as emitter.

Concerning hyperphosphorescence, the prior art describes organic electroluminescent devices comprising, in the emitting layer, a phosphorescent organometallic complex as a sensitizer, which shows mixing of S1 and T1 states due to the large spin-orbit coupling, and a fluorescent compound as an emitter, so that the emission decay time can significantly be shortened.

Hyperfluorescence and hyperphosphorescence are promising techniques to improve OLEDs properties, especially in terms of deep blue emission.

However, further improvements are still necessary with respect to the performance data of OLEDs, in particular with a view to broad commercial use, for example in display devices or as light sources. Of particular importance in this connection are the lifetime, the efficiency, the operating voltage of the OLEDs and the colour values achieved, in particular colour purity.

An important starting point for achieving the said improvements in hyperfluorescent and hyperphosphorescent systems is the choice of the sterically hindered fluorescent emitter compound. The steric shielding of the fluorescent emitter is accomplished by electronically inert, sterically demanding substituents, which surround the electronically active core of the fluorescent compound and thus shield it substantially from contact with adjacent molecules in the layer.

In WO 2015/135624, sterically hindered fluorescent emitters based on rubrene are described. However, there is still a need for further sterically hindered fluorescent emitters, especially sterically hindered blue-fluorescent emitters, which lead to OLEDs having very good properties in terms of efficiency and colour emission. More particularly, there is a need for deep blue-fluorescent emitters combining very high efficiency, very good life time and suitable colour coordinates as well as high colour purity.

Furthermore, it is known that an OLED may comprise different layers, which may be applied either by vapour deposition in a vacuum chamber or by processing from a solution. The processes based on vapour deposition lead to good results, but such processes are complex and expensive. Therefore, there is also a need for OLED materials that can be easily and reliably processed from solution. In this case, the materials should have good solubility properties in the solution that comprises them. Additionally, the OLED materials that are processed from a solution should be able to orientate themselves in the deposited film to improve the overall efficiency of the OLED. The term orientation means here the horizontal molecular orientation of the compounds, as explained in Zhao et al., Horizontal molecular orientation in solution-processed organic light-emitting diodes, Appl. Phys. Lett. 106063301, 2015.

Thus, the present invention is based on the technical object of providing sterically hindered fluorescent emitters, which can be used in combination with a sensitizer compound in a hyperfluorescent or hyperphosphorescent system. More particularly, there is a need for sterically hindered deep blue-fluorescent emitters combining very high efficiency, very good life time and suitable colour coordinates as well as high colour purity.

The present invention is also based on the technical object of providing compounds which are suitable for use in electronic devices, such as OLEDs, more particularly as emitters and, which are suitable for vacuum processing or for solution processing.

In investigations on novel compounds for use in electronic devices, it has now been found, that compounds of formula (1) as defined below are eminently suitable for use in electronic devices. In particular, they achieve one or more, preferably all, of the above-mentioned technical objects.

The invention thus relates to compounds of the formula (1),

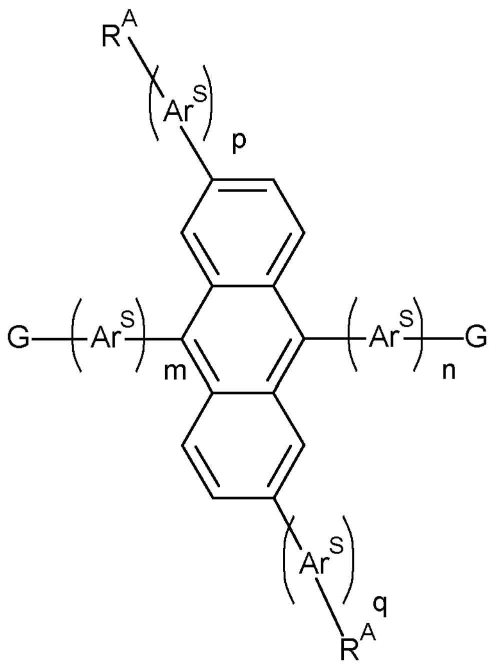

formula (1)

where the following applies to the symbols and indices used:

G is a group of formula (G-1),

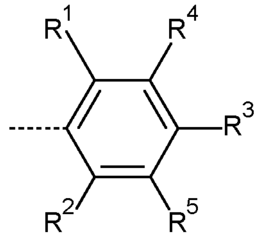

(G-1)

where the dashed bond indicates the bonding to $Ar^S$ or, if $Ar^S$ is absent, to the central benzene of the anthracene as depicted in formula (1);

$R^1$, $R^2$ stand, on each occurrence, identically or differently, for an aromatic or heteroaromatic ring system having 5 to 24 aromatic ring atoms, which may in each case also be substituted by one or more radicals R;

$R^3$, $R^4$, $R^5$ stand, on each occurrence, identically or differently, for H, D, F, Cl, Br, I, CHO, CN, $N(Ar)_2$, C(=O)Ar, $P(=O)(Ar)_2$, S(=O)Ar, $S(=O)_2Ar$, $NO_2$, $Si(R)_3$, $B(OR)_2$, $OSO_2R$, a straight-chain alkyl, alkoxy or thioalkyl group having 1 to 40 C atoms or a branched or cyclic alkyl, alkoxy or thioalkyl group having 3 to 40 C atoms, each of which may be substituted by one or more radicals R, where in each case one or more non-adjacent $CH_2$ groups may be replaced by RC=CR, C≡C, $Si(R)_2$, $Ge(R)_2$, $Sn(R)_2$, C=O, C=S, C=Se, P(=O)(R), SO, $SO_2$, O, S or CONR and where one or more H atoms may be replaced by D, F, Cl, Br, I, CN or $NO_2$, an aromatic or heteroaromatic ring system having 5 to 60 aromatic ring atoms, which may in each case be substituted by one or more radicals R, an aryloxy group having 5 to 60 aromatic ring atoms, which may be substituted by one or more radicals R, or an aralkyl or heteroaralkyl group which has 5 to 60 aromatic ring atoms, which may be substituted by one or more R radicals; where two adjacent radicals selected from $R^3$, $R^4$, $R^5$ may form a mono- or polycyclic, aliphatic ring system or aromatic ring system, which may be substituted by one or more radicals R;

$R^A$ stands, on each occurrence, identically or differently, for F, CN, $N(Ar^N)_2$, C(=O)Ar, $P(=O)(Ar)_2$, S(=O)Ar, $S(=O)_2Ar$, $Si(R)_3$, $B(OR)_2$, $OSO_2R$, a straight-chain alkyl, alkoxy or thioalkyl group having 1 to 40 C atoms or a branched or cyclic alkyl, alkoxy or thioalkyl group having 3 to 40 C atoms, each of which may be substituted by one or more radicals R, where in each case one or more non-adjacent $CH_2$ groups may be replaced by RC=CR, C=C, $Si(R)_2$, $Ge(R)_2$, $Sn(R)_2$, C=O, C=S, C=Se, P(=O)(R), SO, $SO_2$, O, S or CONR and where one or more H atoms may be replaced by D, F, Cl, Br, I, CN or $NO_2$, an aromatic or heteroaromatic ring system having 5 to 60 aromatic ring atoms, which may in each case be substituted by one or more radicals R, an aryloxy group having 5 to 60 aromatic ring atoms, which may be substituted by one or more radicals R, or an aralkyl or heteroaralkyl group which has 5 to 60 aromatic ring atoms, which may be substituted by one or more R radicals;

$Ar^N$ stands, on each occurrence, identically or differently, for a single bond or for an aromatic or heteroaromatic ring system having 5 to 60 aromatic ring atoms, which may in each case also be substituted by one or more radicals R, and wherein two groups $Ar^N$ may be connected with one another by a single bond or by a divalent bridge selected from N(R), O, S, $C(R)_2$, $C(R)_2$-$C(R)_2$, $Si(R)_2$ or B(R);

$Ar^S$ stands, on each occurrence, identically or differently, for a single bond or for an aromatic or heteroaromatic ring system having 5 to 40 aromatic ring atoms, which may in each case also be substituted by one or more radicals R;

R stands on each occurrence, identically or differently, for H, D, F, Cl, Br, I, CHO, CN, $N(Ar)_2$, C(=O)Ar, $P(=O)(Ar)_2$, S(=O)Ar, $S(=O)_2Ar$, $NO_2$, $Si(R')_3$, $B(OR')_2$, $OSO_2R'$, a straight-chain alkyl, alkoxy or thioalkyl group having 1 to 40 C atoms or a branched or cyclic alkyl, alkoxy or thioalkyl group having 3 to 40 C atoms, each of which may be substituted by one or more radicals R', where in each case one or more non-adjacent $CH_2$ groups may be replaced by R'C=CR', C≡C, $Si(R')_2$, $Ge(R')_2$, $Sn(R')_2$, C=O, C=S, C=Se, P(=O)(R'), SO, $SO_2$, O, S or CONR' and where one or more H atoms may be replaced by D, F, Cl, Br, I, CN or $NO_2$, an aromatic or heteroaromatic ring system having

5 to 60 aromatic ring atoms, which may in each case be substituted by one or more radicals R', or an aryloxy group having 5 to 60 aromatic ring atoms, which may be substituted by one or more radicals R', where two adjacent radicals R may form a mono- or polycyclic, aliphatic ring system or aromatic ring system, which may be substituted by one or more radicals R';

Ar is on each occurrence, identically or differently, an aromatic or heteroaromatic ring system having 5 to 24 aromatic ring atoms, which may in each case also be substituted by one or more radicals R';

R' stands on each occurrence, identically or differently, for H, D, F, Cl, Br, I, CN, a straight-chain alkyl, alkoxy or thioalkyl group having 1 to 20 C atoms or a branched or cyclic alkyl, alkoxy or thioalkyl group having 3 to 20 C atoms, where in each case one or more non-adjacent $CH_2$ groups may be replaced by SO, $SO_2$, O, S and where one or more H atoms may be replaced by D, F, Cl, Br or I, or an aromatic or heteroaromatic ring system having 5 to 24 C atoms; and

n, m, p, q are, identically or differently, 0,1 or 2.

Adjacent substituents in the sense of the present invention are substituents which are bonded to atoms which are linked directly to one another or which are bonded to the same atom.

Furthermore, the following definitions of chemical groups apply for the purposes of the present application: An aryl group in the sense of this invention contains 6 to 60 aromatic ring atoms, preferably 6 to 40 aromatic ring atoms, more preferably 6 to 20 aromatic ring atoms; a heteroaryl group in the sense of this invention contains 5 to 60 aromatic ring atoms, preferably 5 to 40 aromatic ring atoms, more preferably 5 to 20 aromatic ring atoms, at least one of which is a heteroatom. The heteroatoms are preferably selected from N, O and S. This represents the basic definition. If other preferences are indicated in the description of the present invention, for example with respect to the number of aromatic ring atoms or the heteroatoms present, these apply.

An aryl group or heteroaryl group here is taken to mean either a simple aromatic ring, i.e. benzene, or a simple heteroaromatic ring, for example pyridine, pyrimidine or thiophene, or a condensed (annellated) aromatic or heteroaromatic polycycle, for example naphthalene, phenanthrene, quinoline or carbazole. A condensed (annellated) aromatic or heteroaromatic polycycle in the sense of the present application consists of two or more simple aromatic or heteroaromatic rings condensed with one another.

An aryl or heteroaryl group, which may in each case be substituted by the above-mentioned radicals and which may be linked to the aromatic or heteroaromatic ring system via any desired positions, is taken to mean, in particular, groups derived from benzene, naphthalene, anthracene, phenanthrene, pyrene, dihydropyrene, chrysene, perylene, fluoranthene, benzanthracene, benzophenanthrene, tetracene, pentacene, benzopyrene, furan, benzofuran, isobenzofuran, dibenzofuran, thiophene, benzothiophene, isobenzothiophene, dibenzothiophene, pyrrole, indole, isoindole, carbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthimidazole, phenanthrimidazole, pyridimidazole, pyrazinimidazole, quinoxalinimidazole, oxazole, benzoxazole, naphthoxazole, anthroxazole, phenanthroxazole, isoxazole, 1,2-thiazole, 1,3-thiazole, benzothiazole, pyridazine, benzopyridazine, pyrimidine, benzopyrimidine, quinoxaline, pyrazine, phenazine, naphthyridine, azacarbazole, benzocarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine and benzothiadiazole.

An aryloxy group in accordance with the definition of the present invention is taken to mean an aryl group, as defined above, which is bonded via an oxygen atom. An analogous definition applies to heteroaryloxy groups.

An aralkyl group in accordance with the definition of the present invention is taken to mean an alkyl group, where at least one hydrogen atom is replaced by an aryl group. An analogous definition applies to heteroaralkyl groups.

An aromatic ring system in the sense of this invention contains 6 to 60 C atoms in the ring system, preferably 6 to 40 C atoms, more preferably 6 to 20 C atoms. A heteroaromatic ring system in the sense of this invention contains 5 to 60 aromatic ring atoms, preferably 5 to 40 aromatic ring atoms, more preferably 5 to 20 aromatic ring atoms, at least one of which is a heteroatom. The heteroatoms are preferably selected from N, O and/or S. An aromatic or heteroaromatic ring system in the sense of this invention is intended to be taken to mean a system which does not necessarily contain only aryl or heteroaryl groups, but instead in which, in addition, a plurality of aryl or heteroaryl groups may be connected by a non-aromatic unit (preferably less than 10% of the atoms other than H), such as, for example, an $sp^3$-hybridised C, Si, N or O atom, an $sp^2$-hybridised C or N atom or an sp-hybridised C atom. Thus, for example, systems such as 9,9'-spirobifluorene, 9,9'-diaryl-fluorene, triarylamine, diaryl ether, stilbene, etc., are also intended to be taken to be aromatic ring systems in the sense of this invention, as are systems in which two or more aryl groups are connected, for example, by a linear or cyclic alkyl, alkenyl or alkynyl group or by a silyl group. Furthermore, systems

in which two or more aryl or heteroaryl groups are linked to one another via single bonds are also taken to be aromatic or heteroaromatic ring systems in the sense of this invention, such as, for example, systems such as biphenyl, terphenyl or diphenyltriazine.

An aromatic or heteroaromatic ring system having 5-60 aromatic ring atoms, which may in each case also be substituted by radicals as defined above and which may be linked to the aromatic or heteroaromatic group via any desired positions, is taken to mean, in particular, groups derived from benzene, naphthalene, anthracene, benzanthracene, phenanthrene, benzophenanthrene, pyrene, chrysene, perylene, fluoranthene, naphthacene, pentacene, benzopyrene, biphenyl, biphenylene, terphenyl, terphenylene, quaterphenyl, fluorene, spirobifluorene, dihydrophenanthrene, dihydropyrene, tetrahydropyrene, cis- or trans-indenofluorene, truxene, isotruxene, spirotruxene, spiroisotruxene, furan, benzofuran, isobenzofuran, dibenzofuran, thiophene, benzothiophene, isobenzothiophene, dibenzothiophene, pyrrole, indole, isoindole, carbazole, indolocarbazole, indenocarbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthimidazole, phenanthrimidazole, pyridimidazole, pyrazinimidazole, quinoxalinimidazole, oxazole, benzoxazole, naphthoxazole, anthroxazole, phenanthroxazole, isoxazole, 1,2-thiazole, 1,3-thiazole, benzothiazole, pyridazine, benzopyridazine, pyrimidine, benzopyrimidine, quinoxaline, 1,5-diazaanthracene, 2,7-diazapyrene, 2,3-diazapyrene, 1,6-diazapyrene, 1,8-diazapyrene, 4,5-diazapyrene, 4,5,9,10-tetraazaperylene, pyrazine, phenazine, phenoxazine, phenothiazine, fluorubin, naphthyridine, azacarbazole, benzocarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine and benzothiadiazole, or combinations of these groups.

For the purposes of the present invention, a straight-chain alkyl group having 1 to 40 C atoms or a branched or cyclic alkyl group having 3 to 40 C atoms or an alkenyl or alkynyl group having 2 to 40 C atoms, in which, in addition, individual H atoms or $CH_2$ groups may be substituted by the groups mentioned above under the definition of the radicals, is preferably taken to mean the radicals methyl, ethyl, n-propyl, i-propyl, n-butyl, i-butyl, s-butyl, t-butyl, 2-methylbutyl, n-pentyl, s-pentyl, cyclopentyl, neopentyl, n-hexyl, cyclohexyl, neohexyl, n-heptyl, cycloheptyl, n-octyl, cyclooctyl, 2-ethylhexyl, trifluoromethyl, pentafluoroethyl, 2,2,2-trifluoroethyl, ethenyl, propenyl, butenyl, pentenyl, cyclopentenyl, hexenyl, cyclohexenyl, heptenyl, cycloheptenyl, octenyl, cyclooctenyl, ethynyl, propynyl, butynyl, pentynyl, hexynyl or octynyl. An alkoxy or thioalkyl group having 1 to 40 C atoms is preferably taken to mean methoxy, trifluoromethoxy, ethoxy, n-propoxy, i-propoxy, n-butoxy, i-butoxy, s-butoxy, t-butoxy, n-pentoxy, s-pentoxy, 2-methylbutoxy, n-hexoxy, cyclohexyloxy, n-heptoxy, cycloheptyloxy, n-octyloxy, cyclooctyloxy, 2-ethylhexyloxy, pentafluoroethoxy, 2,2,2-trifluoroethoxy, methylthio, ethylthio, n-propylthio, i-propylthio, n-butylthio, i-butylthio, s-butylthio, t-butylthio, n-pentylthio, s-pentylthio, n-hexylthio, cyclohexylthio, n-heptylthio, cycloheptylthio, n-octylthio, cyclooctylthio, 2-ethylhexylthio, trifluoromethylthio, pentafluoroethylthio, 2,2,2-trifluoroethylthio, ethenylthio, propenylthio, butenylthio, pentenylthio, cyclopentenylthio, hexenylthio, cyclohexenylthio, heptenylthio, cycloheptenylthio, octenylthio, cyclooctenylthio, ethynylthio, propynylthio, butynylthio, pentynylthio, hexynylthio, heptynylthio or octynylthio.

The formulation that two or more radicals may form a ring with one another is, for the purposes of the present application, intended to be taken to mean, inter alia, that the two radicals are linked to one another by a chemical bond. This is illustrated by the following schemes:

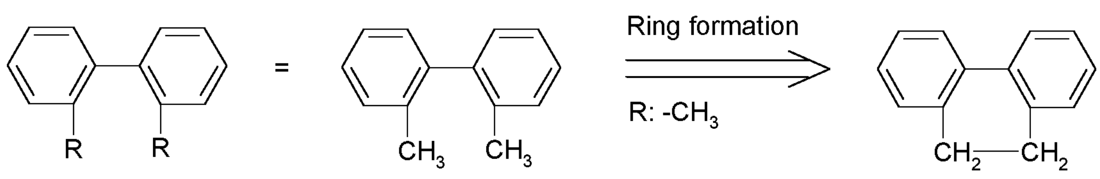


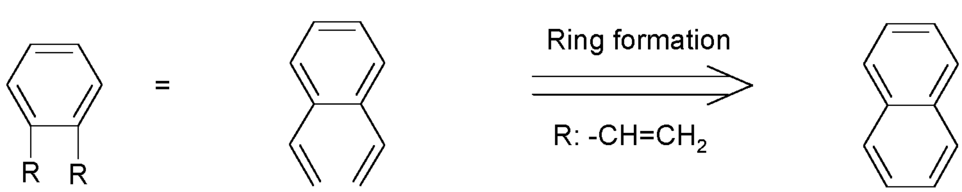


Furthermore, however, the above-mentioned formulation is also intended to be taken to mean that, in the case where one of the two radicals represents hydrogen, the second radical is bonded at the position to which the hydrogen atom was bonded, with formation of a ring. This is illustrated by the following scheme:

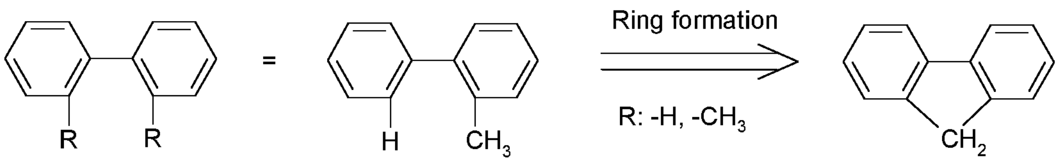


Preferably, the radicals $R^1$ and $R^2$ stand, on each occurrence, identically or differently, for an aromatic or heteroaromatic ring system having 6 to 24 aromatic ring atoms, which may in each case also be substituted by one or more radicals R.

Preferably, the radicals $R^1$ and $R^2$ stand, on each occurrence, identically or differently, for an aromatic or heteroaromatic ring system selected from benzene, naphthalene, biphenyl, fluorene, dibenzofuran, dibenzothiophene, carbazole, pyridine, pyrimidine, pyrazine or triazine, which may in each case also be substituted by one or more radicals R.

More preferably, the radicals $R^1$ and $R^2$ stand, on each occurrence, identically or differently, for an aromatic or heteroaromatic ring system selected from benzene, biphenyl, pyridine, pyrimidine, pyrazine or triazine, which may in each case also be substituted by one or more radicals R.

Particularly preferably, the radicals $R^1$ and $R^2$ stand, on each occurrence, identically or differently, for a benzene or biphenyl ring, which may in each case also be substituted by one or more radicals R.

Preferably, the radicals $R^3$, $R^4$, $R^5$ stand, on each occurrence, identically or differently, for a straight-chain alkyl or alkoxy group having 1 to 10 C atoms or a branched or cyclic alkyl or alkoxy group having 3 to 40 C atoms, each of which may be substituted by one or more radicals R, where in each case one or more non-adjacent $CH_2$ groups may be replaced by RC=CR, C≡C, $Si(R)_2$, $Ge(R)_2$, $Sn(R)_2$, C=O, C=S, C=Se, P(=O)(R), SO, $SO_2$, O, S or CONR and where one or more H atoms may be replaced by D, F, Cl, Br, I, CN or $NO_2$, an aromatic or heteroaromatic ring system having 5 to 40 aromatic ring atoms, which may in each case be substituted by one or more radicals R, an aryloxy group having 5 to 40 aromatic ring atoms, which may be substituted by one or more radicals R, or an aralkyl or heteroaralkyl group which has 5 to 40 aromatic ring atoms, which may be substituted by one or more R radicals.

More preferably, the radicals $R^3$, $R^4$, $R^5$ stand, on each occurrence, identically or differently, for an aromatic or heteroaromatic ring system having 5 to 24 aromatic ring atoms, which may in each case also be substituted by one or more radicals R.

In accordance with a preferred embodiment, the group G stands for a group of formula (G-2), (G-3) or (G-4),

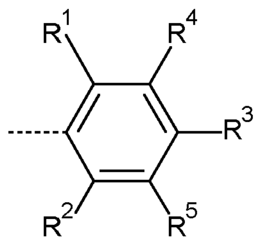
(G-2)

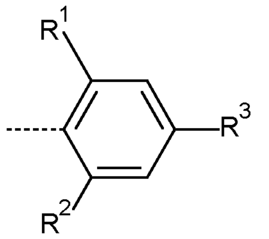
(G-3)

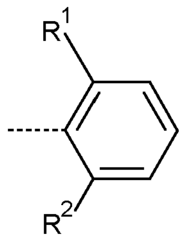
(G-4)

where $R^1$ and $R^2$ have the same definition as above; and

$R^3$, $R^4$, $R^5$ stand on each occurrence, identically or differently for a straight-chain alkyl, alkoxy or thioalkyl group having 1 to 40 C atoms or a branched or cyclic alkyl, alkoxy or thioalkyl group having 3 to 40 C atoms, each of which may be substituted by one or more radicals R, where in each case one or more non-adjacent $CH^2$ groups may be replaced by RC=CR, C≡C, $Si(R)^2$, $Ge(R)^2$, $Sn(R)^2$, C=O, C=S, C=Se, P(=O)(R), SO, $SO_2$, O, S or CONR and where one or more H atoms may be replaced by D, F, Cl, Br, I, CN or $NO_2$, an aromatic or heteroaromatic ring system having 5 to 60 aromatic ring atoms, which may in each case be substituted by one or more radicals R, an aryloxy group having 5 to 60 aromatic ring atoms, which may be substituted by one or more radicals R, or an aralkyl or heteroaralkyl group which has 5 to 60 aromatic ring atoms, which may be substituted by one or more R radicals.

In accordance with a very preferred embodiment, the group G stands for a group of formula (G-2-1), (G-3-1) or (G-4-1),

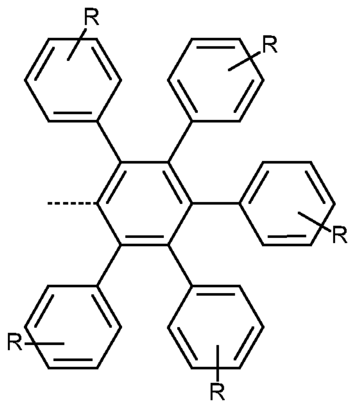

(G-2-1)

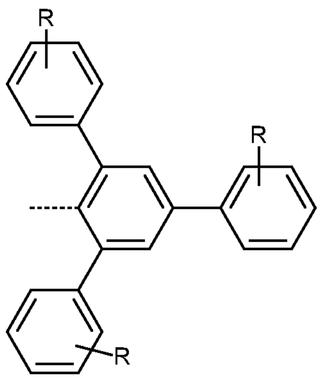

(G-3-1)

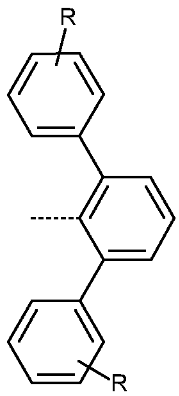

(G-4-1)

where R has the same definition as above.

In accordance with a preferred embodiment, the radical $R^A$ stands, on each occurrence, identically or differently, for F, CN, $N(Ar^N)_2$, C(=O)Ar, $P(=O)(Ar)_2$, S(=O)Ar, $S(=O)_2Ar$, $Si(R)_3$, $B(OR)_2$, $OSO_2R$, a straight-chain alkyl, alkoxy or thioalkyl group having 1 to 40 C atoms or a branched or cyclic alkyl, alkoxy or thioalkyl group having 3 to 40 C atoms, each of which may be substituted by one or more radicals R, where in each case one or more non-adjacent $CH_2$ groups may be replaced by RC=CR, C≡C, $Si(R)_2$, $Ge(R)_2$, $Sn(R)_2$, C=O, C=S, C=Se, P(=O)(R), SO, $SO_2$, O, S or CONR and where one or more H atoms may be replaced by D, F, Cl, Br, I, CN or $NO_2$, an aromatic or heteroaromatic ring system having 5 to 60 aromatic ring atoms, which may in each case be substituted by one or more radicals R, an aryloxy group having 5 to 60 aromatic ring atoms, which may be substituted by one or more radicals R, or an aralkyl or heteroaralkyl group which has 5 to 60 aromatic ring atoms, which may be substituted by one or more R radicals.

In accordance with a very preferred embodiment, the radical $R^A$ is selected on each occurrence, identically or differently,

- from $N(Ar^N)_2$; where $Ar^N$ has the same definition as above;
- from branched or cyclic alkyl groups represented by the general following formula (RS-a)

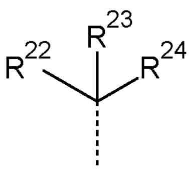

(RS-a)

wherein

$R^{22}$, $R^{23}$, $R^{24}$ are at each occurrence, identically or differently, selected from H, a straight-chain alkyl group having 1 to 10 carbon atoms, or a branched or cyclic alkyl group having 3 to 10 carbon atoms, where the above-mentioned groups may each be substituted by one or more radicals $R^{25}$, and where two of radicals $R^{22}$, $R^{23}$, $R^{24}$ or all radicals $R^{22}$, $R^{23}$, $R^{24}$ may be joined to form a (poly)cyclic alkyl group, which may be substituted by one or more radicals $R^{25}$;

$R^{25}$ is at each occurrence, identically or differently, selected from a straight-chain alkyl group having 1 to 10 carbon atoms, or a branched or cyclic alkyl group having 3 to 10 carbon atoms;

with the proviso that at each occurrence at least one of radicals $R^{22}$, $R^{23}$ and $R^{24}$ is other than H, with the proviso that at each occurrence all of radicals $R^{22}$, $R^{23}$ and $R^{24}$ together have at least 4 carbon atoms and with the proviso that at each occurrence, if two of radicals $R^{22}$, $R^{23}$, $R^{24}$ are H, the remaining radical is not a straight-chain;

- or from branched or cyclic alkoxy groups represented by the general following formula (RS-b)

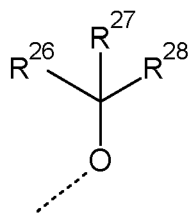

(RS-b)

wherein

$R^{26}$, $R^{27}$, $R^{28}$ are at each occurrence, identically or differently, selected from H, a straight-chain alkyl group having 1 to 10 carbon atoms, or a branched or cyclic alkyl group having 3 to 10 carbon atoms, where the above-mentioned groups may each be substituted by one or more radicals $R^{25}$ as defined above, and where two of radicals $R^{26}$, $R^{27}$, $R^{28}$ or all radicals $R^{26}$, $R^{27}$, $R^{28}$ may be joined to form a (poly)cyclic alkyl group, which may be substituted by one or more radicals $R^{25}$ as defined above;
with the proviso that at each occurrence only one of radicals $R^{26}$, $R^{27}$ and $R^{28}$ may be H;

- or from aralkyl groups represented by the general following formula (RS-c)

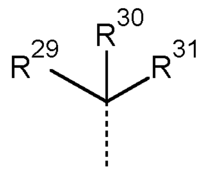

(RS-c)

wherein

$R^{29}$, $R^{30}$, $R^{31}$ are at each occurrence, identically or differently, selected from H, a straight-chain alkyl group having 1 to 10 carbon atoms, or a branched or cyclic alkyl group having 3 to 10 carbon atoms, where the above-mentioned groups may each be substituted by one or more radicals $R^{32}$, or an aromatic ring system having 6 to 30 aromatic ring atoms, which may in each case be substituted by one or more radicals $R^{32}$, and where two or all of radicals $R^{29}$, $R^{30}$, $R^{31}$ may be joined to form a (poly)cyclic alkyl group or an aromatic ring system, each of which may be substituted by one or more radicals $R^{32}$;
$R^{32}$ is at each occurrence, identically or differently, selected from a straight-chain alkyl group having 1 to 10 carbon atoms, or a branched or cyclic alkyl group having 3 to 10 carbon atoms, or an aromatic ring system having 6 to 24 aromatic ring atoms;
with the proviso that at each occurrence at least one of radicals $R^{29}$, $R^{30}$ and $R^{31}$ is other than H and that at each occurrence at least one of radicals $R^{29}$, $R^{30}$ and $R^{31}$ is or contains an aromatic ring system having at least 6 aromatic ring atoms;

- or from aromatic ring systems represented by the general following formula (RS-d)

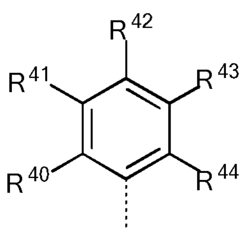

(RS-d)

wherein

$R^{40}$ to $R^{44}$ is at each occurrence, identically or differently, selected from H, a straight-chain alkyl group having 1 to 10 carbon atoms, or a branched or cyclic alkyl group having 3 to 10 carbon atoms, where the above-mentioned groups may each be substituted by one or more radicals $R^{32}$, or an aromatic ring system having 6 to 30 aromatic ring atoms, which may in each case be substituted by one or more radicals $R^{32}$, and where two or more of radicals $R^{40}$ to $R^{44}$ may be joined to form a (poly)cyclic alkyl group or an aromatic ring system, each of which may be substituted by one or more radicals $R^{32}$ as defined above.

Examples of suitable groups of formulae (RS-a) to (RS-d) are the groups (RS-1) to (RS-78):

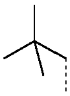
(RS-1)
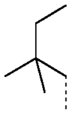
(RS-2)
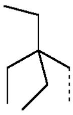
(RS-3)
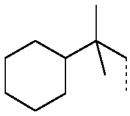
(RS-4)
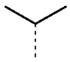
(RS-5)
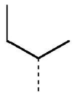
(RS-6)
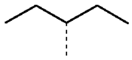
(RS-7)
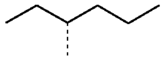
(RS-8)

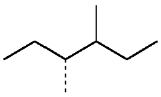
(RS-9)
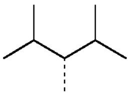
(RS-10)
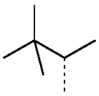
(RS-11)
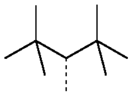
(RS-12)
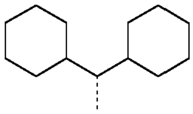
(RS-13)
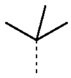
(RS-14)
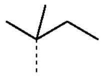
(RS-15)
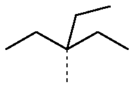
(RS-16)

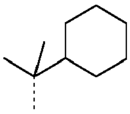
(RS-17)
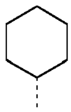
(RS-18)
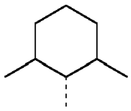
(RS-19)
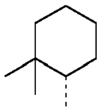
(RS-20)
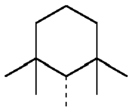
(RS-21)
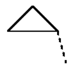
(RS-22)

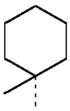
(RS-23)
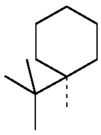
(RS-24)
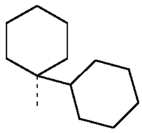
(RS-25)
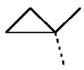
(RS-26)
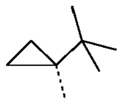
(RS-27)

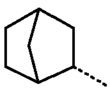
(RS-28)
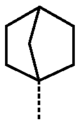
(RS-29)
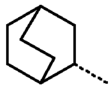
(RS-30)
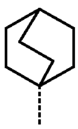
(RS-31)
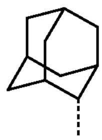
(RS-32)
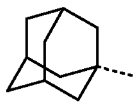
(RS-33)

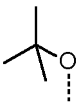
(RS-34)
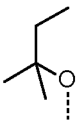
(RS-35)
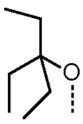
(RS-36)
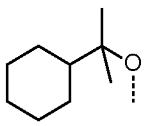
(RS-37)
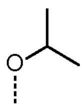
(RS-38)
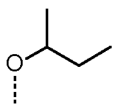
(RS-39)
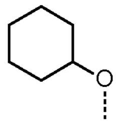
(RS-40)

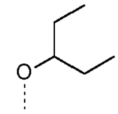
(RS-41)

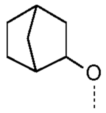
(RS-42)

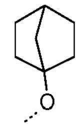
(RS-43)

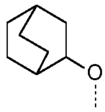
(RS-44)

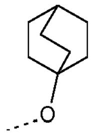
(RS-45)

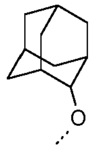
(RS-46)

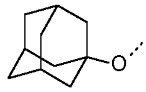
(RS-47)

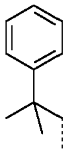
(RS-48)

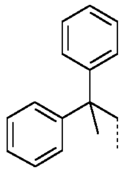
(RS-49)

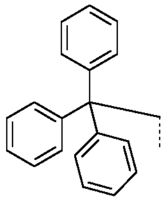
(RS-50)

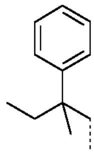
(RS-51)

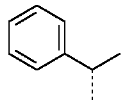
(RS-52)

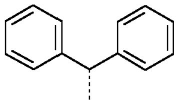
(RS-53)

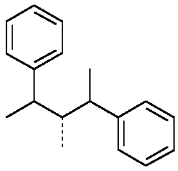
(RS-54)

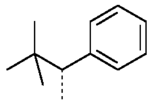
(RS-55)

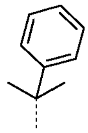
(RS-56)

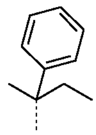
(RS-57)

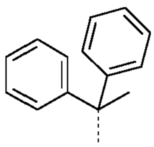
(RS-58)

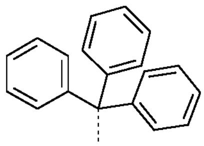
(RS-59)

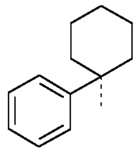
(RS-60)

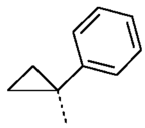
(RS-61)

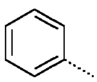
(RS-62)

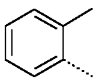
(RS-63)

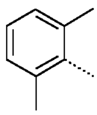
(RS-64)

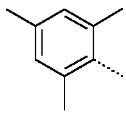
(RS-65)

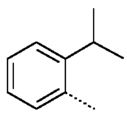
(RS-66)

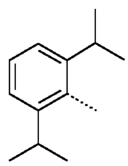
(RS-67)

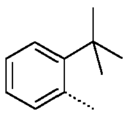
(RS-68)

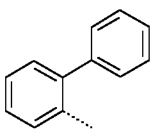
(RS-69)

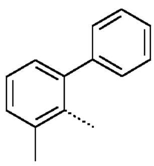
(RS-70)

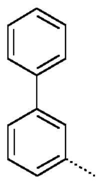
(RS-71)

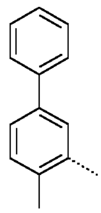
(RS-72)

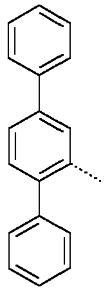

(RS-73)

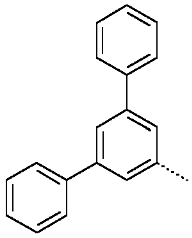

(RS-74)

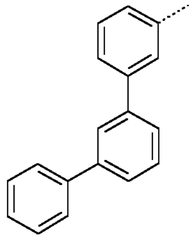

(RS-75)

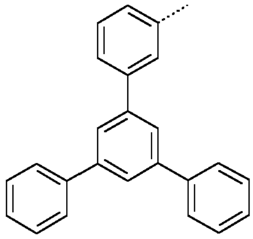

(RS-76)

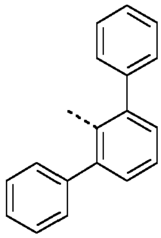

(RS-77)

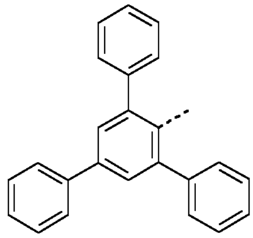

(RS-78)

where the dashed bond indicates the bonding of these groups to the structure of formula (1) and where the groups of formulae (RS-1) to (RS-47) may further be substituted by a least one group $R^{25}$ as defined above and groups (RS-48) to (RS-78) may further be substituted by a least one group $R^{32}$ as defined above.

Preferably, the group $Ar^S$ stands for benzene, biphenyl, terphenyl, naphthalene, fluorene, indenofluorene, spirobifluorene, triazine, benzoquinoline, benzoquinazoline, dibenzofuran, dibenzothiophene, and carbazole, where each of the above-mentioned groups may be substituted by one or more radicals R. More preferably, the group $Ar^S$ stands for benzene, biphenyl, terphenyl, naphthalene, fluorene, indenofluorene or spirobifluorene, where each of the above-mentioned groups may be substituted by one or more radicals R. Particularly preferably, the group $Ar^S$ stands for benzene, which may be substituted by one or more radicals R.

It is preferred that m = n = 0.

It is also preferred that p = q = 0.

Even more preferably, m = n = 0 and p = q = 0. When m = n = p = q = 0, then the corresponding groups $Ar^S$ are absent and the compounds of formula (1) correspond to compounds of formula (1-A),

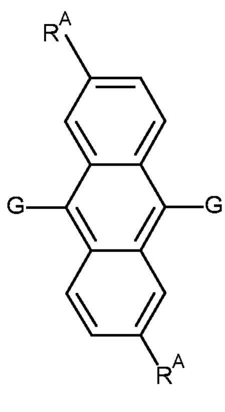

formula (1-A)

In accordance with a preferred embodiment, the compounds of formula (1) are selected from the compounds of formulae (2), (3) or (4),

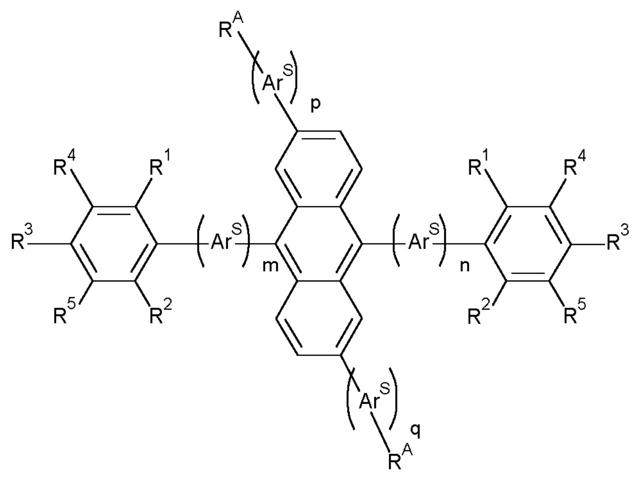

formula (2)

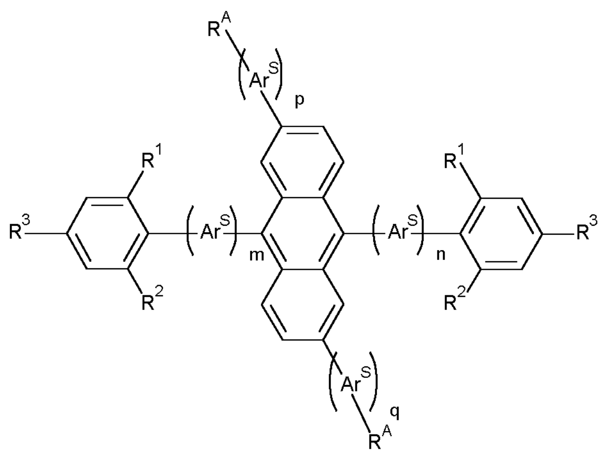

formula (3)

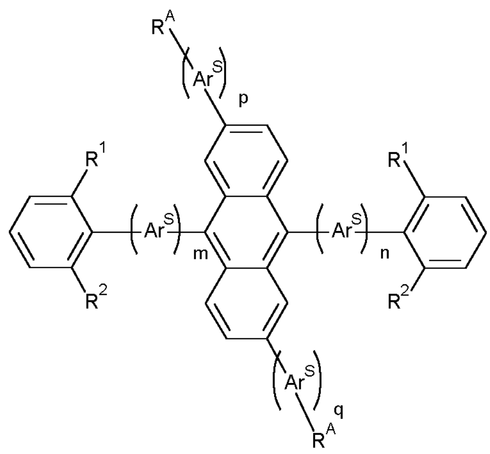

formula (4)

where $R^3$, $R^4$, $R^5$ stand on each occurrence, identically or differently for a straight-chain alkyl, alkoxy or thioalkyl group having 1 to 40 C atoms or a branched or cyclic alkyl, alkoxy or thioalkyl group having 3 to 40 C atoms, each of which may be substituted by one or more radicals R, where in each case one or more non-adjacent $CH^2$ groups may be replaced by RC=CR, C=C, $Si(R)^2$, $Ge(R)^2$, $Sn(R)^2$, C=O, C=S, C=Se, P(=O)(R), SO, $SO_2$, O, S or CONR and where one or more H atoms may be replaced by D, F, Cl, Br, I, CN or $NO_2$, an aromatic or heteroaromatic ring system having 5 to 60 aromatic ring atoms, which may in each case be substituted by one or more radicals R, an aryloxy group having 5 to 60 aromatic ring atoms, which may be substituted by one or more radicals R, or an aralkyl or heteroaralkyl group which has 5 to 60 aromatic ring atoms, which may be substituted by one or more R radicals; and where the other symbols and indices have the same meaning as above.

In accordance with a preferred embodiment, the compounds of formula (1) are selected from the compounds of formulae (2-1a) to (2-5a), (3-1a) to (3-5a) and (4-1a) to (4-5a),

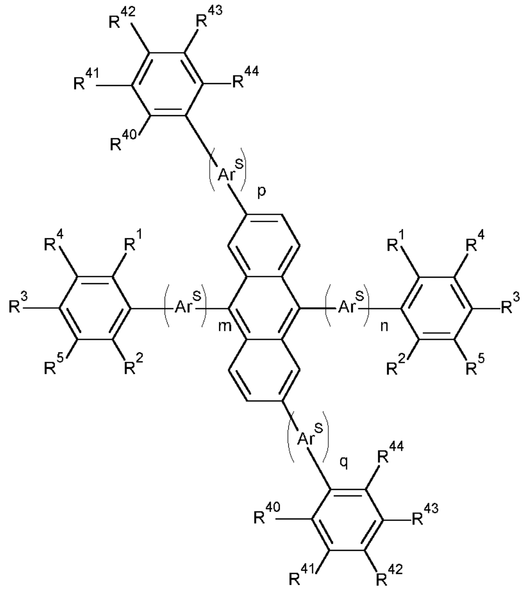

formula (2-1-a)

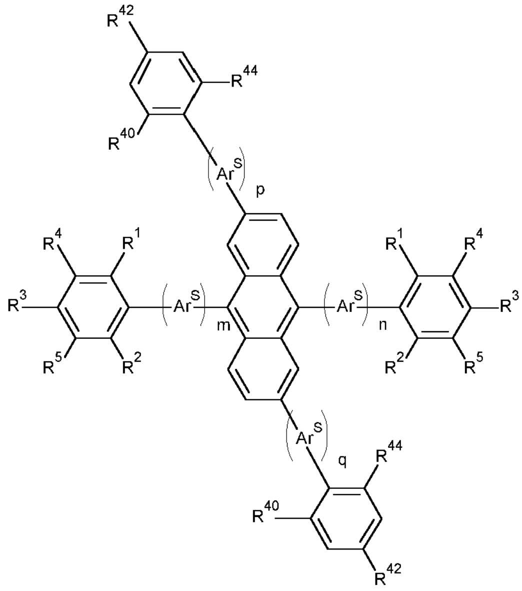

formula (2-2-a)

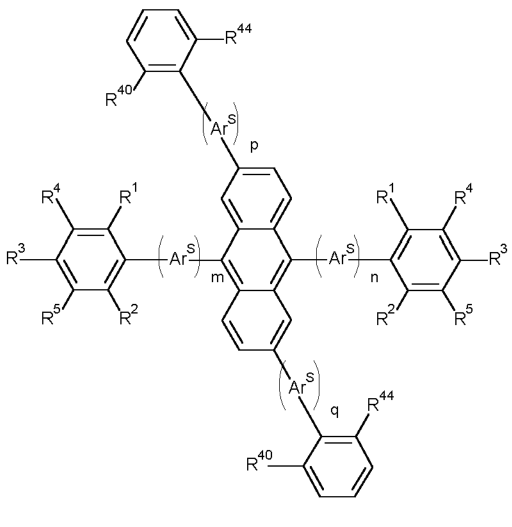

formula (2-3-a)

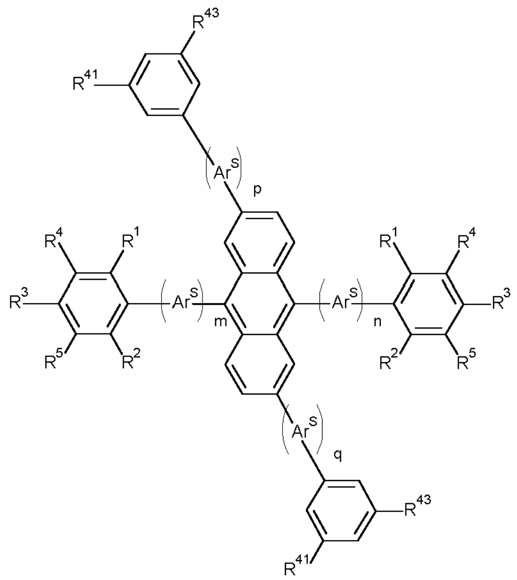

formula (2-4-a)

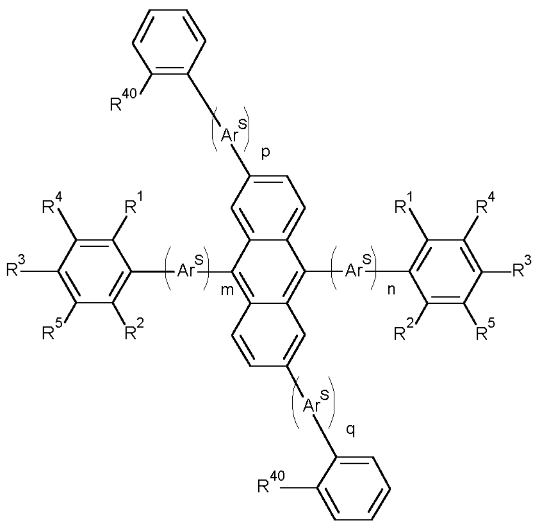

formula (2-5-a)

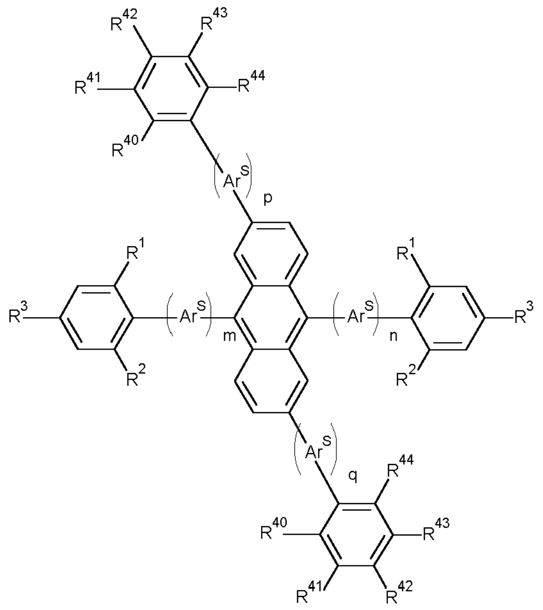

formula (3-1-a)

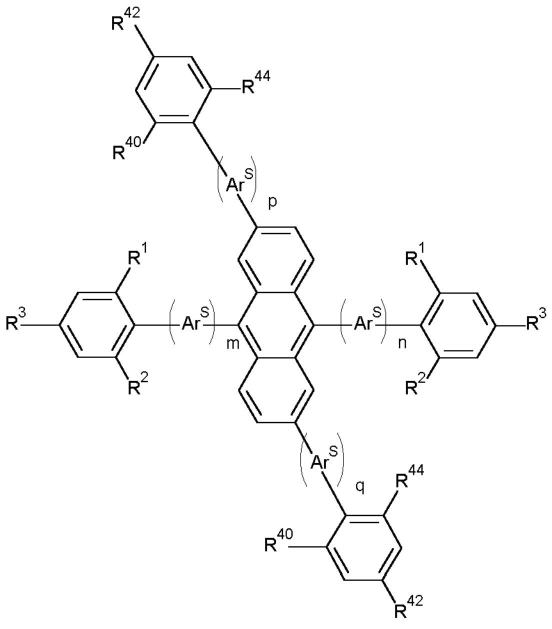

formula (3-2-a)

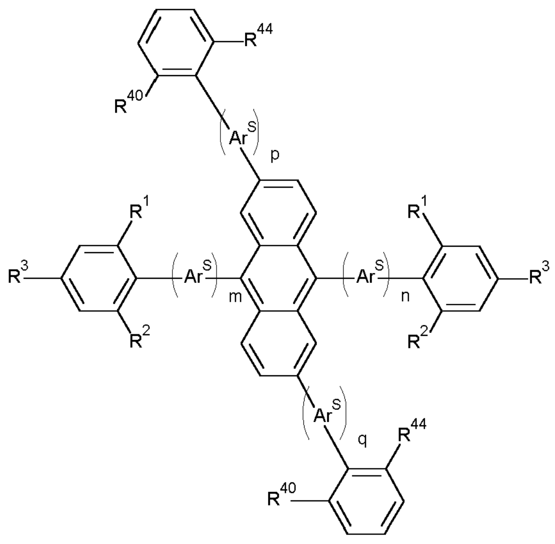

formula (3-3-a)

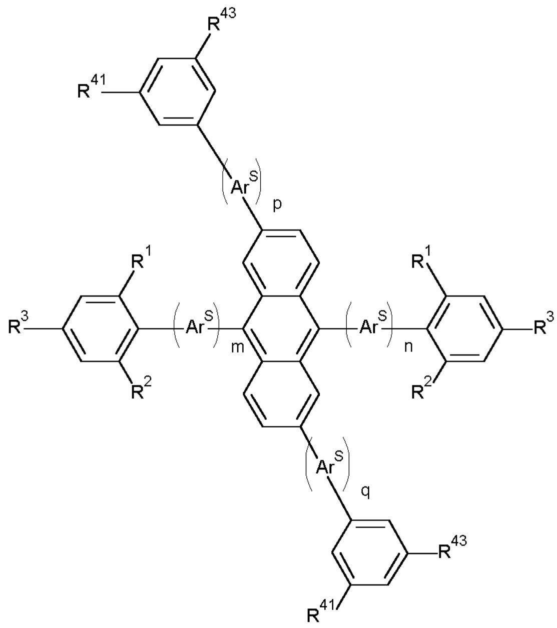

formula (3-4-a)

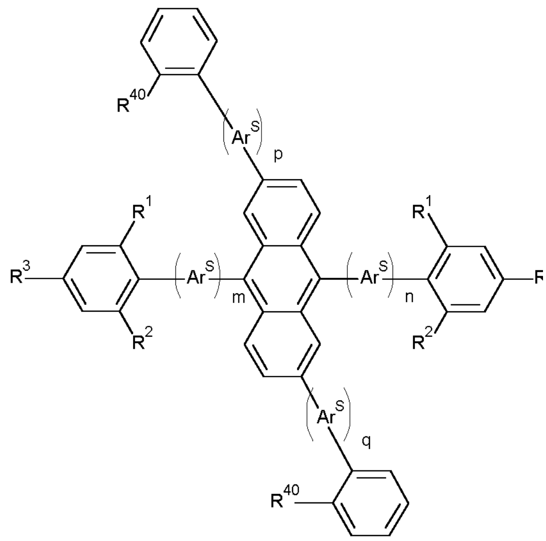

formula (3-5-a)

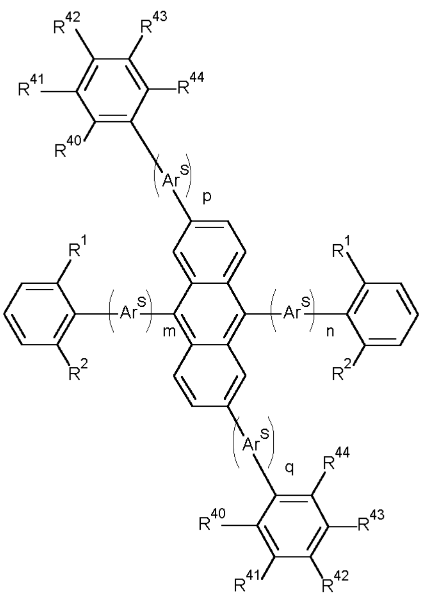

formula (4-1-a)

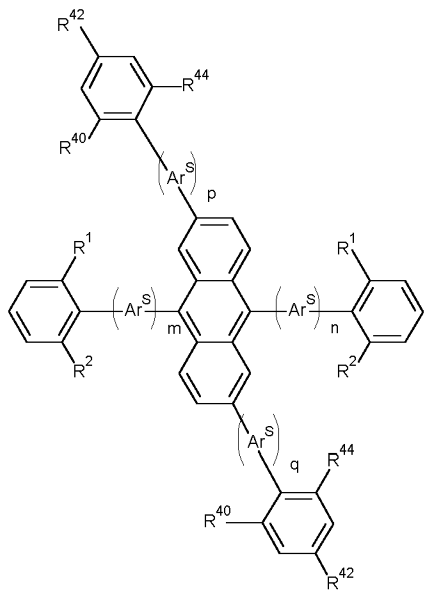

formula (4-2-a)

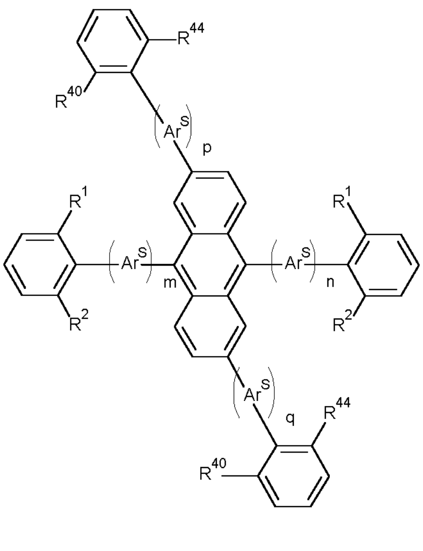

formula (4-3-a)

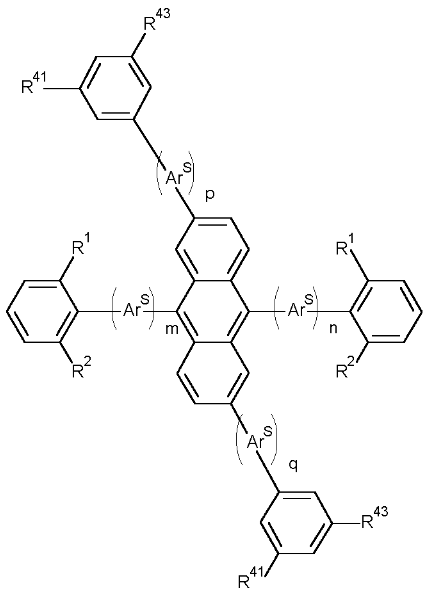

formula (4-4-a)

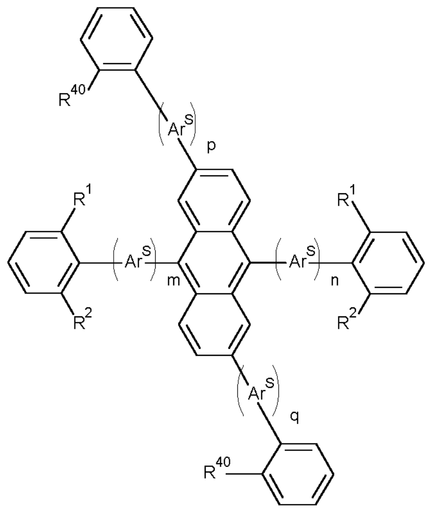

formula (4-5-a)

where

$R^3$, $R^4$, $R^5$ stand on each occurrence, identically or differently for a straight-chain alkyl, alkoxy or thioalkyl group having 1 to 40 C atoms or a branched or cyclic alkyl, alkoxy or thioalkyl group having 3 to 40 C atoms, each of which may be substituted by one or more radicals R, where in each case one or more non-adjacent $CH^2$ groups may be replaced by RC=CR, C≡C, $Si(R)^2$, $Ge(R)^2$, $Sn(R)^2$, C=O, C=S, C=Se, P(=O)(R), SO, $SO_2$, O, S or CONR and where one or more H atoms may be replaced by D, F, Cl, Br, I, CN or $NO_2$, an aromatic or heteroaromatic ring system having 5 to 60 aromatic ring atoms, which may in each case be substituted by one or more radicals R, an aryloxy group having 5 to 60 aromatic ring atoms, which may be substituted by one or more radicals R, or an aralkyl or heteroaralkyl group which has 5 to 60 aromatic ring atoms, which may be substituted by one or more R radicals;

$R^{40}$ to $R^{44}$ are on each occurrence, identically or differently, selected from a straight-chain alkyl group having 1 to 10 carbon atoms, or a branched or cyclic alkyl group having 3 to 10 carbon atoms, where the above-mentioned groups may each be substituted by one or more radicals $R^{32}$, or an aromatic ring system having 6 to 30 aromatic ring atoms, which may in each case be substituted by one or more radicals $R^{32}$; where $R^{32}$ is as defined as above; and where $R^1$, $R^2$, R, m, n, p and q have the same definition as above.

Preferably, the radicals $R^{40}$ to $R^{44}$ are at each occurrence, identically or differently, selected from an aromatic

ring system having 6 to 30 aromatic ring atoms, which may in each case be substituted by one or more radicals $R^{32}$; where $R^{32}$ is as defined as above.

In accordance with a particularly preferred embodiment, the compounds of formula (1) are selected from the compounds of formulae (2-1-a') to (4-5-a'),

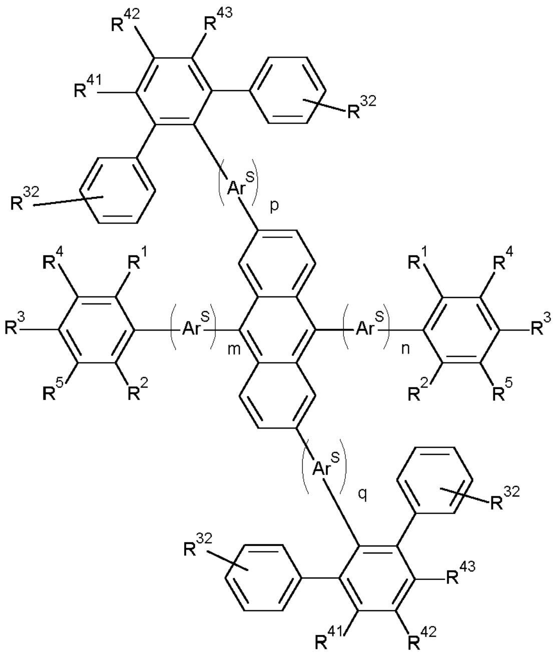

formula (2-1-a´)

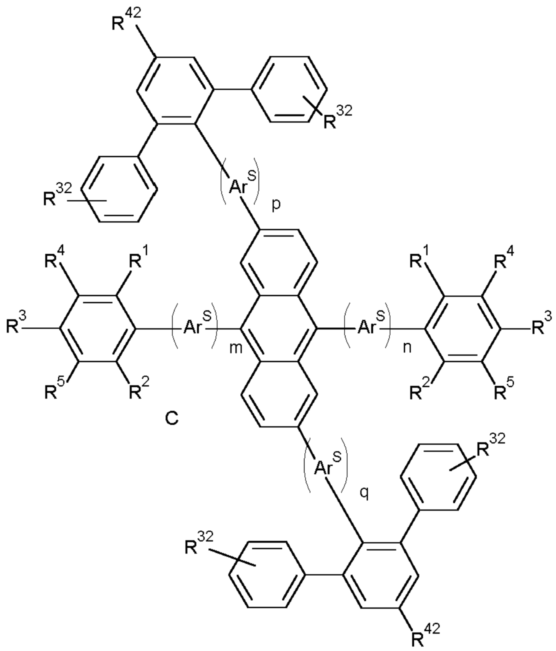

formula (2-2-a´)

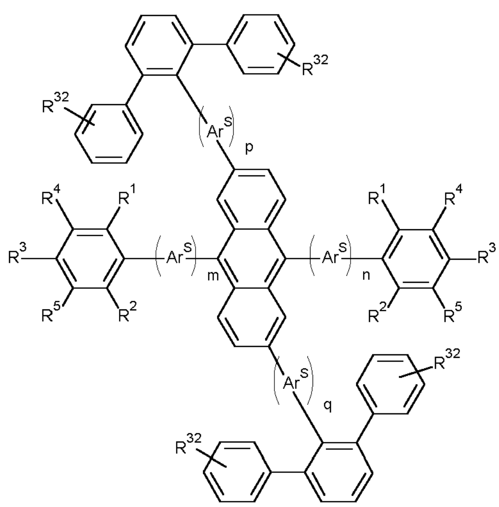

formula (2-3-a´)

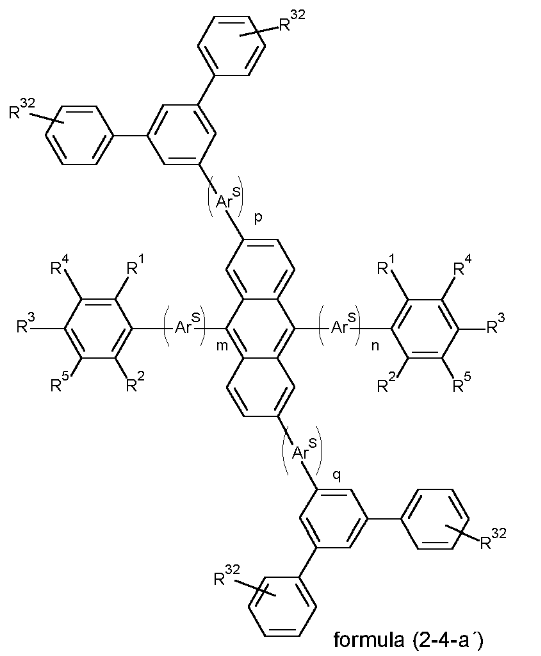

formula (2-4-a´)

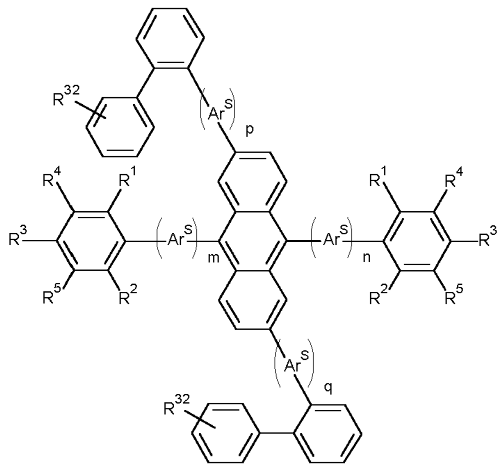

formula (2-5-a´)

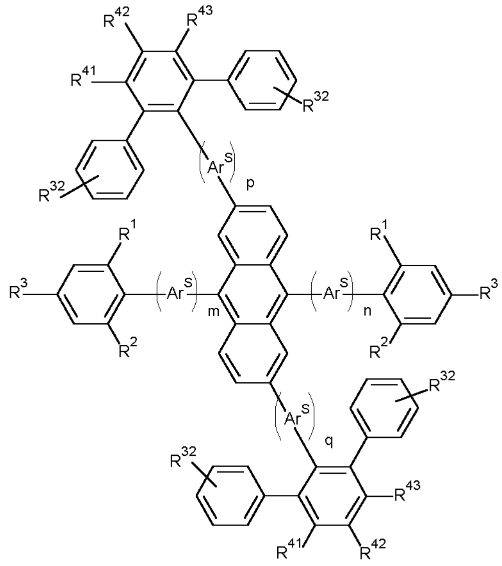

formula (3-1-a´)

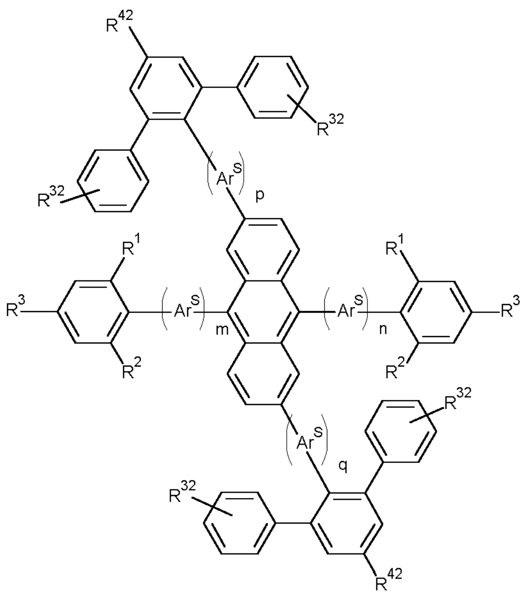

formula (3-2-a´)

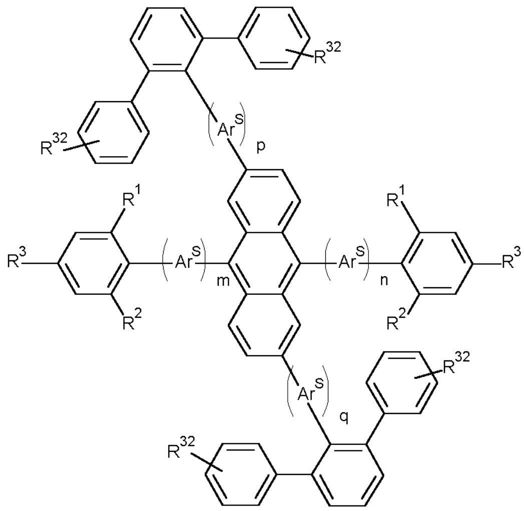

formula (3-3-a´)

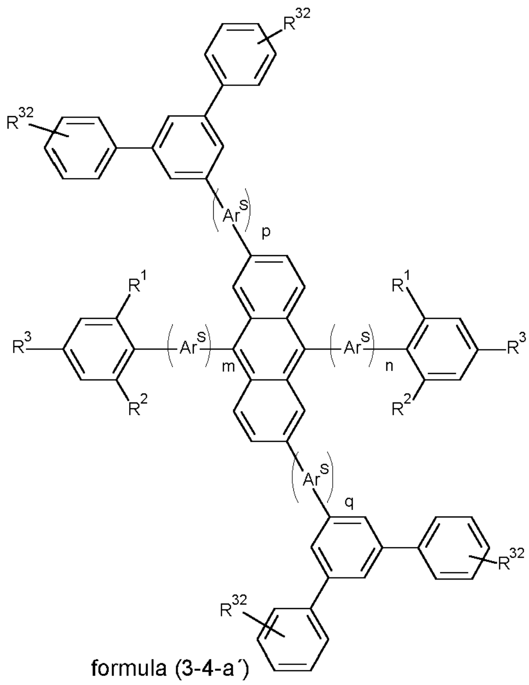

formula (3-4-a´)

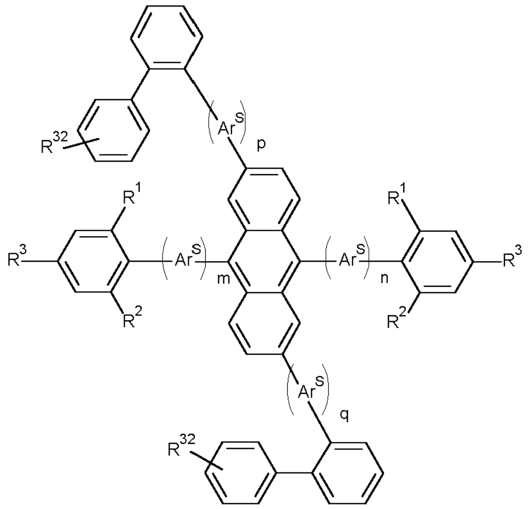

formula (3-5-a´)

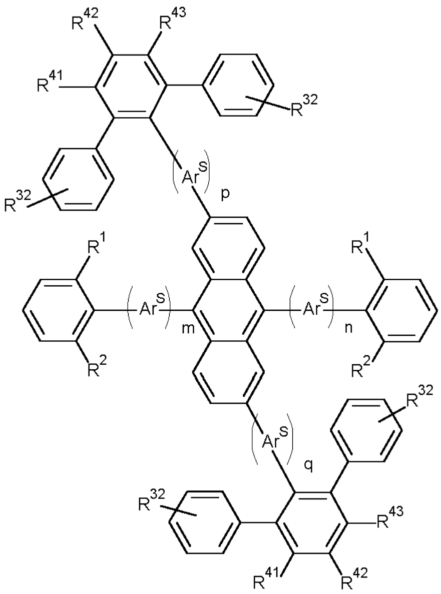

formula (4-1-a´)

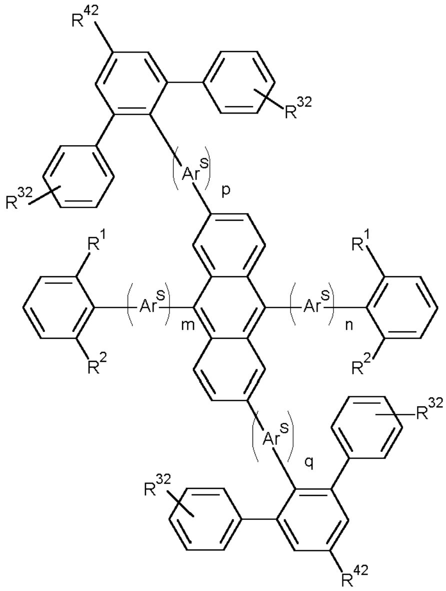

formula (4-2-a´)

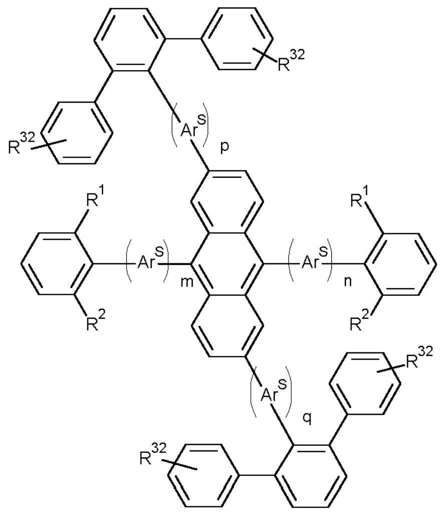

formula (4-3-a´)

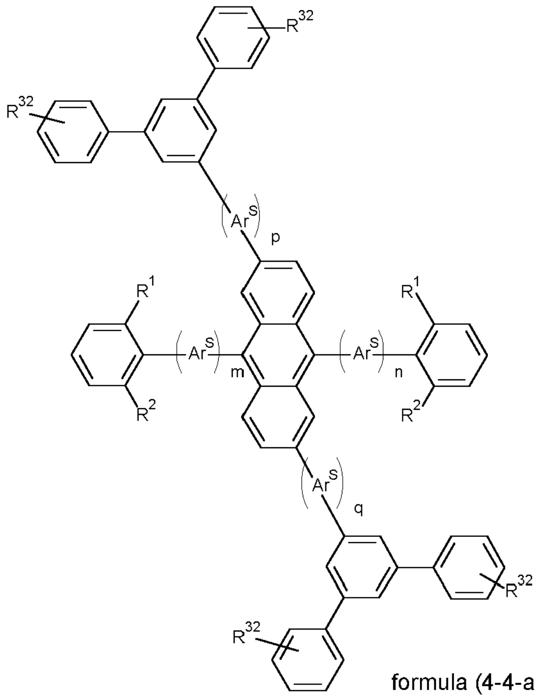

formula (4-4-a´)

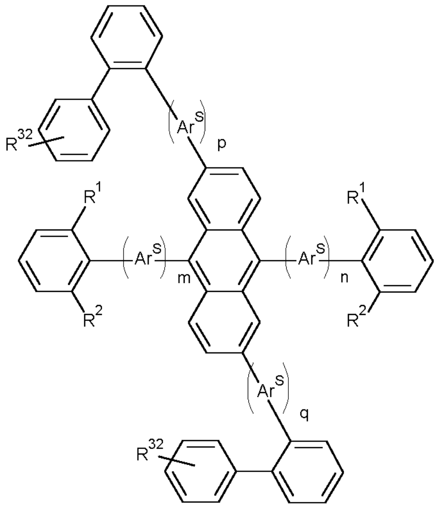

formula (4-5-a´)

where the symbols and indices have the same meaning as above.

In accordance with a very particularly preferred embodiment, the compounds of formula (1) are selected from the compounds of formulae (2-b) to (4-b),

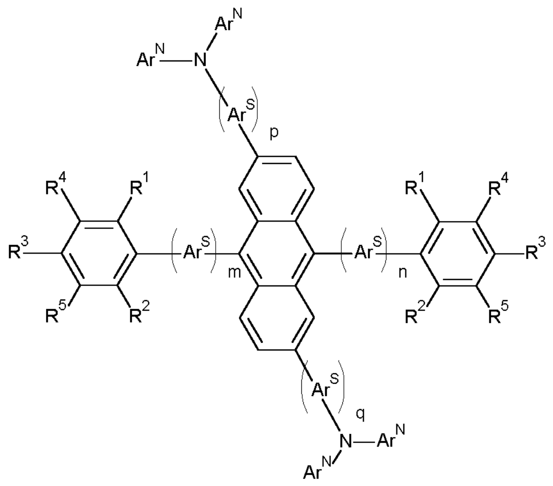

formula (2-b)

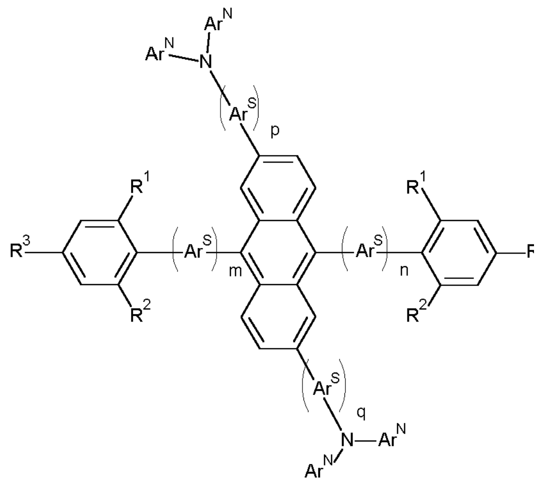

formula (3-b)

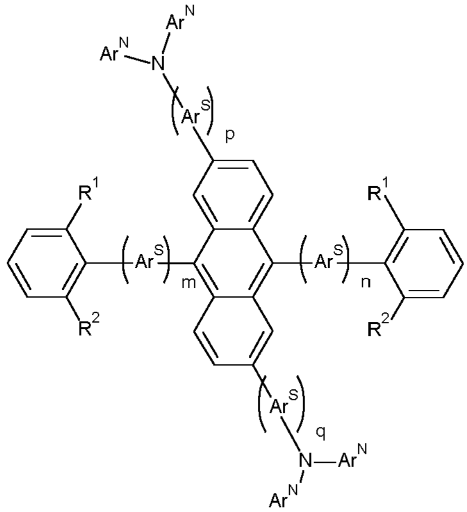

formula (4-b)

where the symbols and indices have the same meaning as above.

Preferably, $Ar^N$ stands, on each occurrence, identically or differently, for an aromatic or heteroaromatic ring system having 5 to 40, preferably 5 to 30, more preferably 6 to 25, particularly preferably from 6 to 18 aromatic ring atoms, which may in each case also be substituted by one or more radicals R, and wherein two groups $Ar^N$ may be connected with one another by a single bond or by a divalent bridge selected from N(R), O, S, $C(R)_2$, $C(R)_2$-$C(R)_2$, $Si(R)_2$ or B(R).

When two groups $Ar^N$ are connected with one another, then the group $N(Ar^N)_2$ preferably has the structure of one of the following formulae (E1) to (E7),

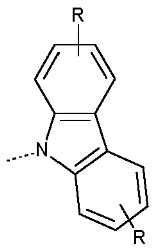

(E-1)

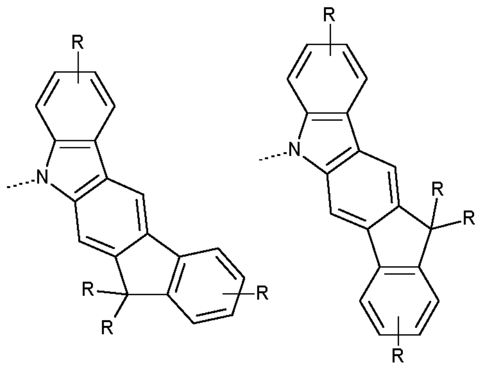

(E-2) (E-3)

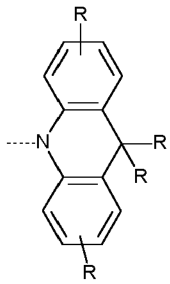

(E-4)

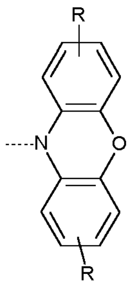

(E-5)

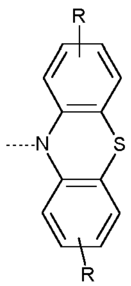

(E-6)

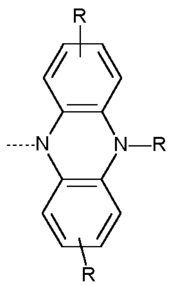

(E-7)

where the dashed bond indicates the bonding to the structure in formula (1) and where the radical R has the same meaning as above.

More preferably, when $Ar^N$ is an aromatic or heteroaromatic ring system, then $Ar^N$ stands on each occurrence, identically or differently, for an aromatic or heteroaromatic ring system selected from benzene, naphthalene, anthracene, phenanthrene, biphenyl, terphenyl, quaterphenyl, fluorene, spirobifluorene, dibenzofuran, dibenzothiophene, carbazole, indolocarbazole or indenocarbazole, which may in each case be substituted by one or more radicals R, or combinations of these groups.

Particularly preferably, when $Ar^N$ is an aromatic or heteroaromatic ring system, then $Ar^N$ stands on each occurrence, identically or differently, for an aromatic or heteroaromatic ring system selected from benzene, naphthalene, biphenyl, terphenyl, fluorene or spirobifluorene, which may in each case be substituted by one or more radicals R, or combinations of these groups.

Preferably, the group R stands on each occurrence, identically or differently, for H, D, F, Cl, Br, I, CHO, CN, $N(Ar)_2$, $Si(R')_3$, a straight-chain alkyl, alkoxy or thioalkyl group having 1 to 40, preferably 1 to 20, more preferably 1 to 10 C atoms or branched or cyclic alkyl, alkoxy or thioalkyl groups having 3 to 40, preferably 3 to 20, more preferably 3 to 10 C atoms, each of which may be substituted by one or more radicals R', where in each case one or more non-adjacent $CH_2$ groups may be replaced by R'C=CR', O or S and where one or more H atoms may be replaced by D, F or CN, an aromatic or heteroaromatic ring system having 5 to 60 aromatic ring atoms, which may in each case be substituted by one or more radicals R', or an aryloxy group having 5 to 60, preferably 5 to 40, more preferably 5 to 30, very preferably 5 to 18 aromatic ring atoms, which may be substituted by one or more radicals R', where two adjacent radicals R may form a mono- or polycyclic, aliphatic ring system or aromatic ring system, which may be substituted by one or more radicals R'. When R is selected from aromatic and heteroaromatic ring systems, it is preferably selected from aromatic and heteroaromatic ring systems having 5 to 40, preferably 5 to 30, more preferably 5 to 18 aromatic ring atoms.

Preferably, the group Ar is on each occurrence, identically or differently, an aromatic or heteroaromatic ring system having 5 to 18, preferably 6 to 18 aromatic ring atoms, which may in each case also be substituted by one or more radicals R'.

Preferably, R' stands on each occurrence, identically or differently, for H, D, F, Cl, Br, I, CN, a straight-chain alkyl, alkoxy or thioalkyl group having 1 to 10 C atoms or branched or cyclic alkyl, alkoxy or thioalkyl group having 3 to 10 C atoms, where one or more H atoms may be replaced by D or F, or an aromatic or heteroaromatic ring system having 5 to 18, preferably 6 to 18 C atoms.

The following compounds are examples of compounds of formula (1):

| | |
|---|---|
| 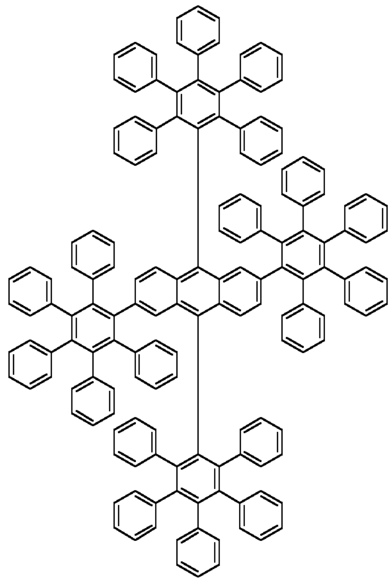 | 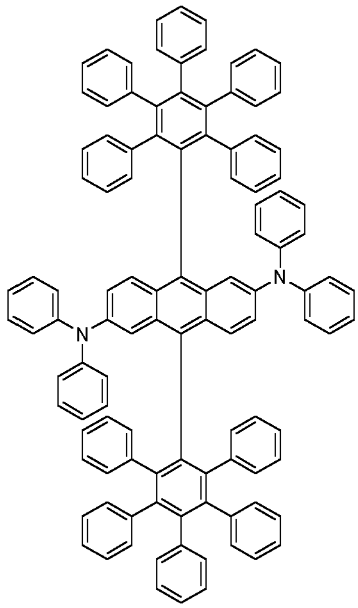 |
| 1 | 2 |
| 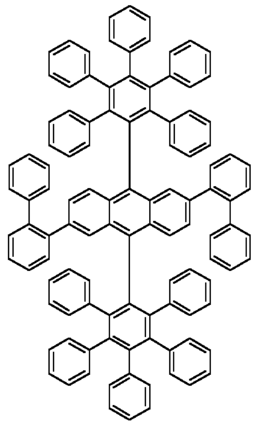 | 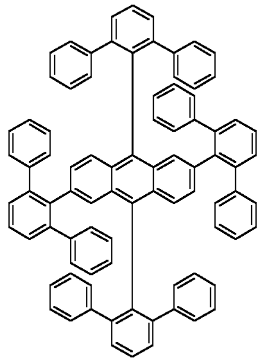 |
| 3 | 4 |
| 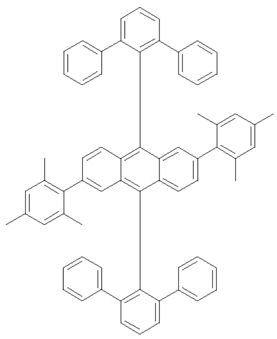 | 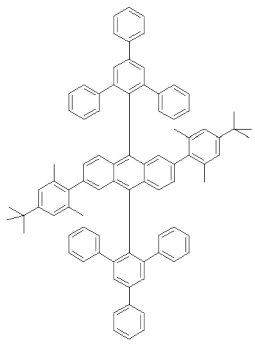 |
| 5 | 6 |

(continued)

| | |
|---|---|
| 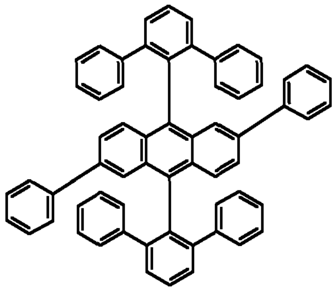 | 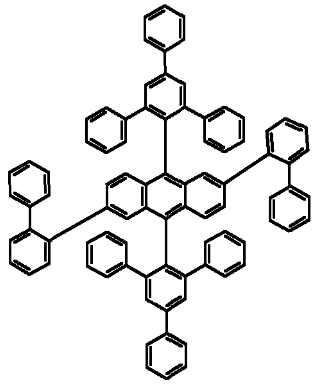 |
| 7 | 8 |
| 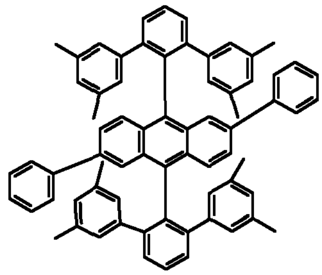 | 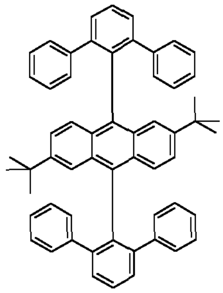 |
| 9 | 10 |
| 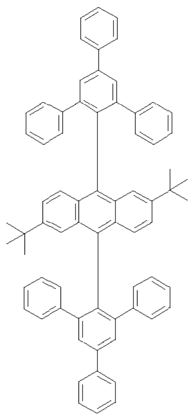 | 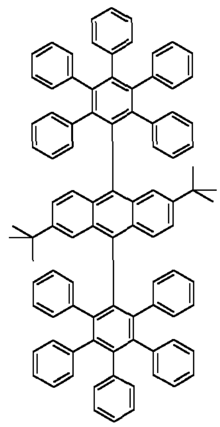 |
| 11 | 12 |
| 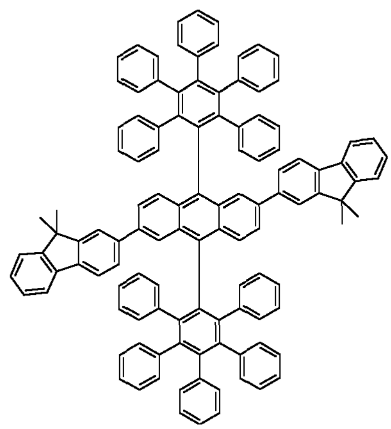 | 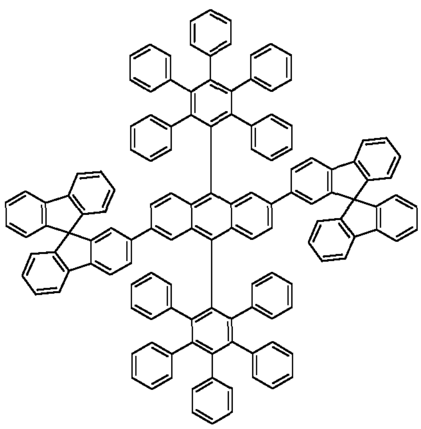 |

(continued)

| 13 | 14 |
|---|---|
| 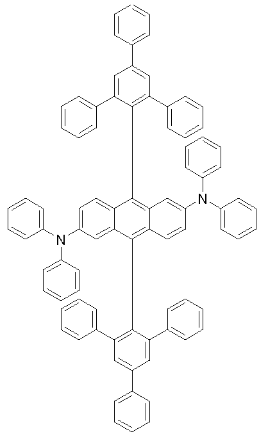 | 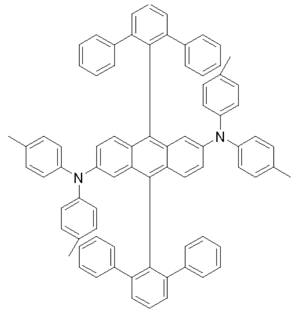 |
| 15 | 16 |
| 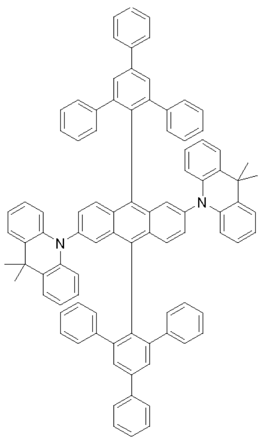 | 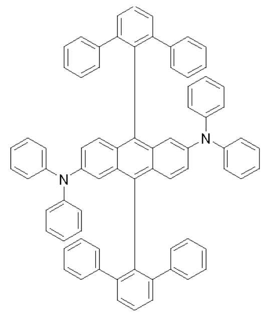 |
| 17 | 18 |
| 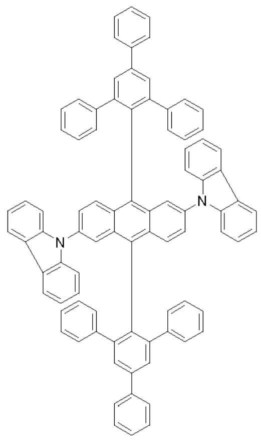 | 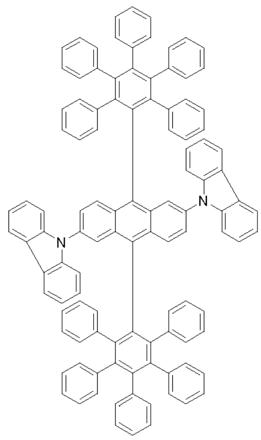 |
| 19 | 20 |

(continued)

| | |
|---|---|
| 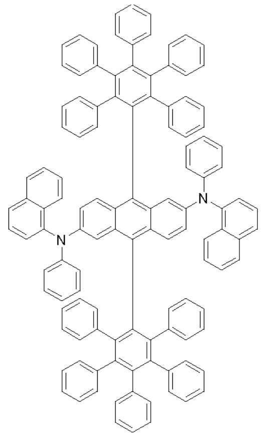 | 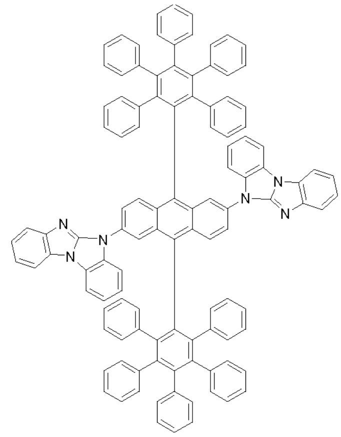 |
| 21 | 22 |
| 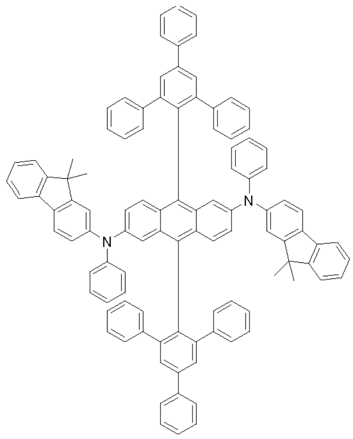 | 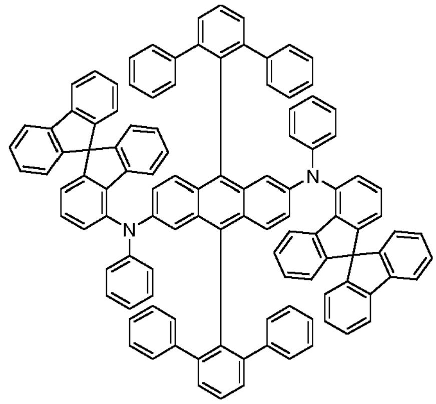 |
| 23 | 24 |

The compounds according to the invention can be prepared by synthesis steps known to the person skilled in the art, such as, for example, bromination, Suzuki coupling, Kumada coupling, Sonogashira coupling, Ullmann coupling, Hartwig-Buchwald coupling, Diels-Alder reaction etc. An example of a suitable synthesis process is depicted in general terms in schemes 1a, 1b, 2a and 2b below.

## Scheme 1a

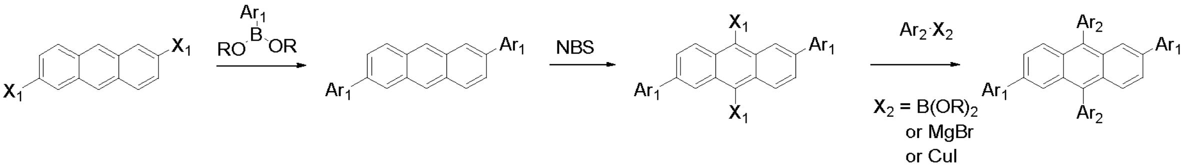


where, in Scheme 1a:

$X_1$ represents a leaving group, preferably selected from a halogen (like Cl, Br, I), a boronic acid, a boronic ester or a triflate selected from a boronic acid, a boronic ester or a triflate;

$X_2$ is $B(OR)_2$, MgBr or Cui;

$Ar^1$ and $Ar^2$ are aromatic or heteroaromatic ring systems.

## Scheme 1b

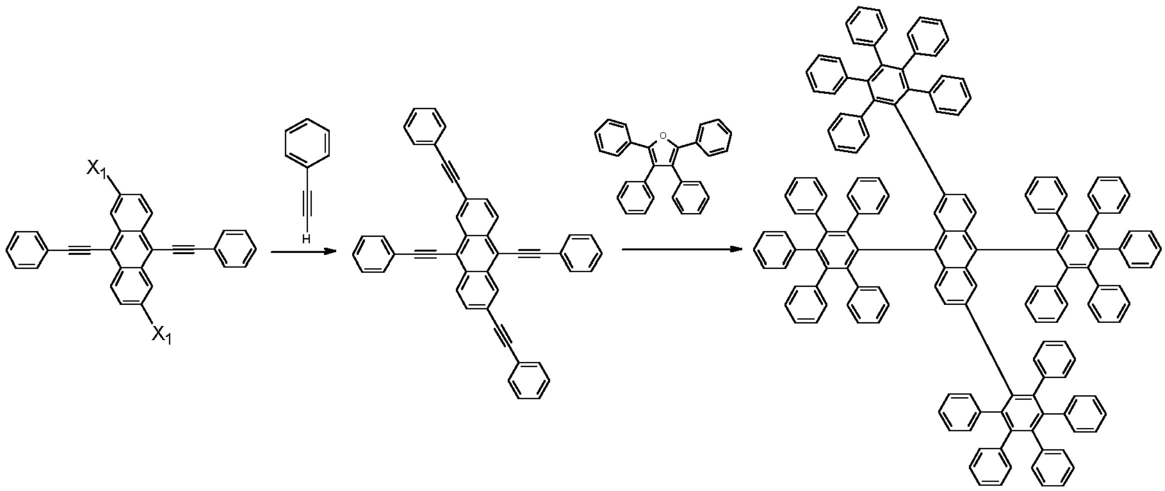

where, in Scheme 1b, $X_1$ is a leaving group, which has the same meaning as above.

## Scheme 2a

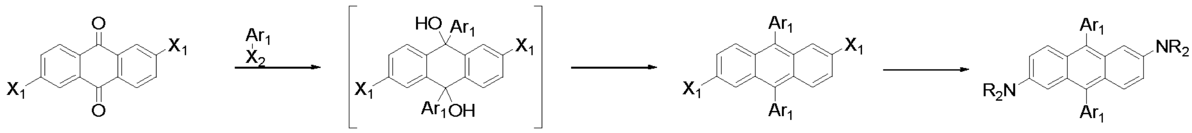


where $X_1$ and $X_2$ have the same meaning as above.

## Scheme 2b

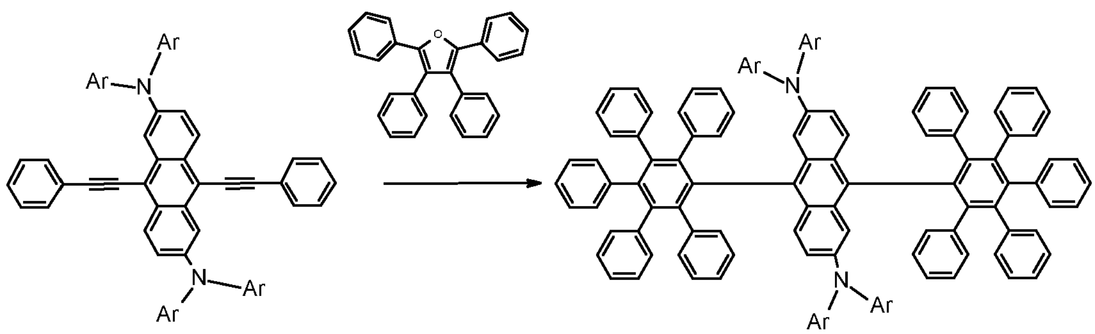

where, in scheme 2b, the group Ar is an aromatic or heteroaromatic ring system.

For the processing of the compounds according to the invention from the liquid phase, for example by spin coating or by printing processes, formulations of the compounds according to the invention are necessary. These formulations can be, for example, solutions, dispersions or emulsions. It may be preferred to use mixtures of two or more solvents for this purpose. Suitable and preferred solvents are, for example, toluene, anisole, o-, m- or p-xylene, methyl benzoate, mesitylene, tetralin, veratrol, THF, methyl-THF, THP, chlorobenzene, dioxane, phenoxytoluene, in particular 3-phenoxytoluene, (-)-fenchone, 1,2,3,5-tetramethylbenzene, 1,2,4,5-tetramethylbenzene, 1-methylnaphthalene, 2-methylbenzothiazole, 2-phenoxyethanol, 2-pyrrolidinone, 3-methylanisole, 4-methylanisole, 3,4-dimethylanisole, 3,5-dimethylanisole, acetophenone, α-terpineol, benzothiazole, butyl benzoate, cumene, cyclohexanol, cyclohexanone, cyclohexylbenzene, decalin, dodecylbenzene, ethyl benzoate, indane, methyl benzoate, NMP, p-cymene, phenetole, 1,4-diisopropylbenzene, dibenzyl ether, diethylene glycol butyl methyl ether, triethylene glycol butyl methyl ether, diethylene

glycol dibutyl ether, triethylene glycol dimethyl ether, diethylene glycol monobutyl ether, tripropylene glycol dimethyl ether, tetraethylene glycol dimethyl ether, 2-isopropylnaphthalene, pentylbenzene, hexylbenzene, heptylbenzene, octylbenzene, 1,1-bis(3,4-dimethylphenyl)ethane or mixtures of these solvents.

The present invention therefore furthermore relates to a formulation comprising a compound according to the invention and at least one further compound. The further compound may be, for example, a solvent, in particular one of the above-mentioned solvents or a mixture of these solvents. However, the further compound may also be at least one further organic or inorganic compound which is likewise employed in the electronic device, for example an emitting compound, in particular a phosphorescent dopant, and/or a further matrix material. Suitable emitting compounds and further matrix materials are indicated below in connection with the organic electroluminescent device. This further compound may also be polymeric.

The compounds and mixtures according to the invention are suitable for use in an electronic device. An electronic device here is taken to mean a device which comprises at least one layer which comprises at least one organic compound. However, the component here may also comprise inorganic materials or also layers built up entirely from inorganic materials.

The present invention therefore furthermore relates to the use of the compounds or mixtures according to the invention in an electronic device, in particular in an organic electroluminescent device.

The present invention again furthermore relates to an electronic device comprising at least one of the compounds or mixtures according to the invention mentioned above.

The electronic device is preferably selected from the group consisting of organic electroluminescent devices (OLEDs, PLEDs), organic integrated circuits (O-ICs), organic field-effect transistors (O-FETs), organic thin-film transistors (O-TFTs), organic light-emitting transistors (O-LETs), organic solar cells (O-SCs), organic dye-sensitised solar cells, organic optical detectors, organic photoreceptors, organic field-quench devices (O-FQDs), light-emitting electrochemical cells (LECs), organic laser diodes (O-lasers) and "organic plasmon emitting devices" (D. M. Koller et al., Nature Photonics 2008, 1-4), preferably organic electroluminescent devices (OLEDs, PLEDs), in particular phosphorescent OLEDs.

The organic electroluminescent device comprises a cathode, an anode and at least one emitting layer. Apart from these layers, it may also comprise further layers, for example in each case one or more hole-injection layers, hole-transport layers, hole-blocking layers, electron-transport layers, electron-injection layers, exciton-blocking layers, electron-blocking layers and/or charge-generation layers. It is likewise possible for interlayers, which have, for example, an exciton-blocking function, to be introduced between two emitting layers. However, it should be pointed out that each of these layers does not necessarily have to be present. The organic electroluminescent device here may comprise one emitting layer or a plurality of emitting layers. If a plurality of emission layers are present, these preferably have in total a plurality of emission maxima between 380 nm and 750 nm, resulting overall in white emission, i.e. various emitting compounds which are able to fluoresce or phosphoresce are used in the emitting layers. Particular preference is given to systems having three emitting layers, where the three layers exhibit blue, green and orange or red emission (for the basic structure see, for example, WO 2005/011013). These can be fluorescent or phosphorescent emission layers or hybrid systems, in which fluorescent and phosphorescent emission layers are combined with one another.

Generally preferred classes of material for use as corresponding functional materials in the organic electroluminescent devices according to the invention are indicated below.

Suitable charge-transport materials, as can be used in the hole-injection or hole-transport layer or electron-blocking layer or in the electron-transport layer of the electronic device according to the invention, are, for example, the compounds disclosed in Y. Shirota et al., Chem. Rev. 2007, 107(4), 953-1010, or other materials as are employed in these layers in accordance with the prior art.

Materials which can be used for the electron-transport layer are all materials as are used in accordance with the prior art as electron-transport materials in the electron-transport layer. Particularly suitable are aluminium complexes, for example $Alq_3$, zirconium complexes, for example $Zrq_4$, lithium complexes, for example LiQ, benzimidazole derivatives, triazine derivatives, pyrimidine derivatives, pyridine derivatives, pyrazine derivatives, quinoxaline derivatives, quinoline derivatives, oxadiazole derivatives, aromatic ketones, lactams, boranes, diazaphosphole derivatives and phosphine oxide derivatives. Furthermore, suitable materials are derivatives of the above-mentioned compounds, as disclosed in JP 2000/053957, WO 2003/060956, WO 2004/028217, WO 2004/080975 and WO 2010/072300.

Preferred hole-transport materials which can be used in a hole-transport, hole-injection or electron-blocking layer in the electroluminescent device according to the invention are indenofluorenamine derivatives (for example in accordance with WO 06/122630 or WO 06/100896), the amine derivatives disclosed in EP 1661888, hexaazatriphenylene derivatives (for example in accordance with WO 01/049806), amine derivatives containing condensed aromatic rings (for example in accordance with US 5,061,569), the amine derivatives disclosed in WO 95/09147, monobenzoindenofluorenamines (for example in accordance with WO 08/006449), dibenzoindenofluorenamines (for example in accordance with WO 07/140847), spirobifluorenamines (for example in accordance with WO 2012/034627 or WO 2013/120577), fluorenamines (for example in accordance with the as applications EP 2875092, EP 2875699 and EP 2875004), spirodibenzopyranamines (for example in accordance with WO 2013/083216) and dihydroacridine derivatives (for example in

accordance with WO 2012/150001). The compounds according to the invention can also be used as hole-transport materials.

Preferably, the organic electroluminescent device comprises at least one compound of the formula (1) or in accordance with the preferred embodiments, as a fluorescent emitter, as an emitter showing TADF (Thermally Activated Delayed Fluorescence), or as a matrix material for fluorescent emitters. More preferably, the organic electroluminescent device comprises a compound of the formula (1) or in accordance with the preferred embodiments as a fluorescent emitter, more particularly blue-emitting fluorescent compound. Particularly preferably, the organic electroluminescent device comprises a compound of the formula (1) or in accordance with the preferred embodiments as a fluorescent emitter in an emitting layer, preferably in combination with a sensitizer selected from compounds that exhibit delayed fluorescence or a phosphorescent compound.

A sensitizer in the sense of the present invention is taken to mean a compound (donor), from which an energy transfer to another compound (acceptor) takes place.

In accordance with a preferred embodiment, the organic electroluminescent device comprises an emitting layer, comprising a fluorescent emitter selected from compounds of formula (1) in combination with a sensitizer selected from compounds that exhibit delayed fluorescence.

Compounds exhibiting delayed fluorescence are preferably compounds which exhibit thermally activated delayed fluorescence. These compounds are abbreviated in the description which follows to "TADF compounds".

TADF compounds are compounds in which the energy gap between the lowest triplet state $T_1$ and the first excited singlet state $S_1$ is sufficiently small that the $S_1$ state is thermally accessible from the $T_1$ state. Preferably, TADF compounds have a gap between the lowest triplet state $T_1$ and the first excited singlet state $S_1$ of $\leq$ 0.30 eV. More preferably, the gap between $S_1$ and $T_1$ is $\leq$ 0.20 eV, even more preferably $\leq$ 0.15 eV, especially more preferably $\leq$ 0.10 eV and even more especially preferably $\leq$ 0.08 eV. The energy of the lowest excited singlet state ($S_1$) and the lowest triplet state ($T_1$) can be determined by quantum-chemical calculation, as detailed below in the present description.

Preferably, the TADF compound is an organic compound. Organic compounds in the context of the present invention are carbonaceous compounds that do not contain any metals. More particularly, organic compounds are formed from the elements C, H, D, B, Si, N, P, O, S, F, Cl, Br and I.

The TADF compound is more preferably an aromatic compound having both donor and acceptor substituents, with only slight spatial overlap between the LUMO and the HOMO of the compound. What is understood by donor and acceptor substituents is known in principle to those skilled in the art. Suitable donor substituents are especially diaryl- or -heteroarylamino groups and carbazole groups or carbazole derivatives, each preferably bonded to the aromatic compound via N. These groups may also have further substitution. Suitable acceptor substituents are especially cyano groups, but also, for example, electron-deficient heteroaryl groups which may also have further substitution, for example substituted or unsubstituted triazine groups.

The preferred dopant concentrations of the TADF compound in the emitting layer are described hereinafter. Because of the difference in production of the organic electroluminescent device, the dopant concentration in the case of production of the emitting layer by vapor deposition is reported in % by volume, and in the case of production of the emitting layer from solution in % by weight. The dopant concentrations in % by volume and % by weight are generally very similar.

In a preferred embodiment of the invention, in the case of production of the emitting layer by vapor deposition, the TADF compound is present in a dopant concentration of 1% to 70% by volume in the emitting layer, more preferably of 5% to 50% by volume, even more preferably of 5% to 30% by volume.

In a preferred embodiment of the invention, in the case of production of the emitting layer from solution, the TADF compound is present in a dopant concentration of 1% to 70% by weight in the emitting layer, more preferably of 5% to 50% by weight, even more preferably of 5% to 30% by weight.

The general art knowledge of the person skilled in the art includes knowledge of which materials are generally suitable as TADF compounds. The following references disclose, by way of example, materials that are potentially suitable as TADF compounds:

- Tanaka et al., Chemistry of Materials 25(18), 3766 (2013).
- Lee et al., Journal of Materials Chemistry C 1(30), 4599 (2013).
- Zhang et al., Nature Photonics advance online publication, 1 (2014), doi: 10.1038/nphoton.2014.12.
- Serevicius et al., Physical Chemistry Chemical Physics 15(38), 15850 (2013).
- Li et al., Advanced Materials 25(24), 3319 (2013).
- Youn Lee et al., Applied Physics Letters 101(9), 093306 (2012).
- Nishimoto et al., Materials Horizons 1, 264 (2014), doi: 10.1039/C3MH00079F.
- Valchanov et al., Organic Electronics, 14(11), 2727 (2013).
- Nasu et al., ChemComm, 49, 10385 (2013).

In addition, the following patent applications disclose potential TADF compounds: WO 2013/154064, WO 2013/133359, WO 2013/161437, WO 2013/081088, WO 2013/081088, WO 2013/011954, JP 2013/116975 und US 2012/0241732.

In addition, the person skilled in the art is able to infer design principles for TADF compounds from these publications. For example, Valchanov et al. show how the color of TADF compounds can be adjusted.

Examples of suitable molecules which exhibit TADF are the structures shown in the following table:

| | | |
|---|---|---|
| 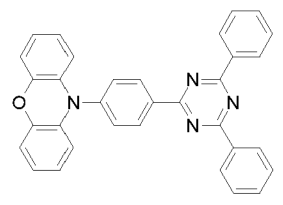 | 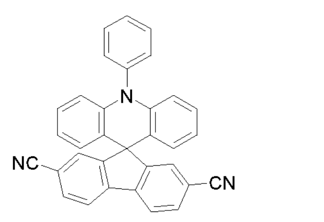 | 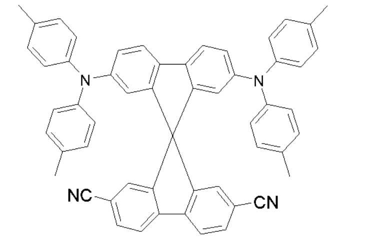 |
| 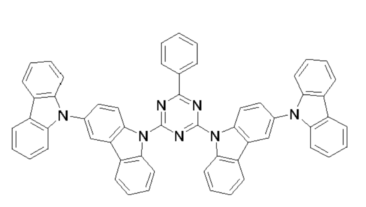 | 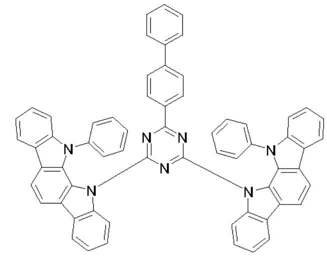 | 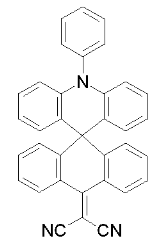 |
| 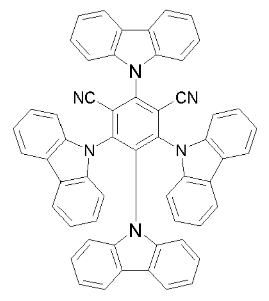 | 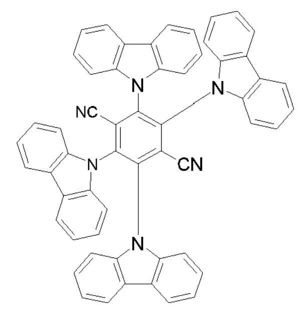 | 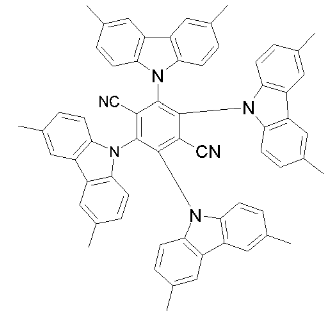 |
| 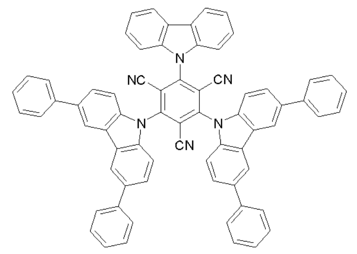 | 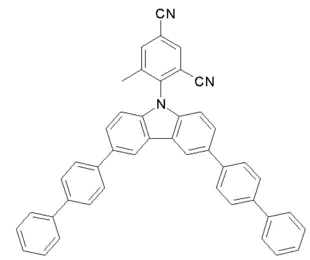 | 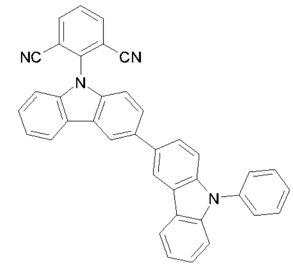 |
| 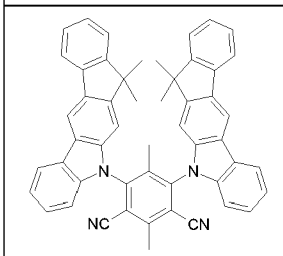 | 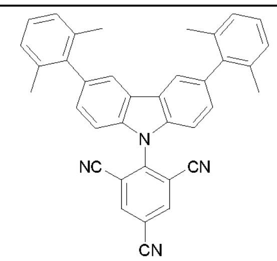 | 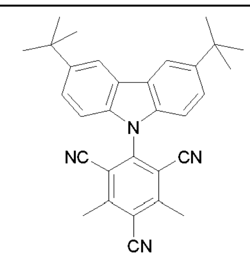 |
| 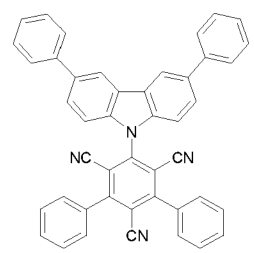 | 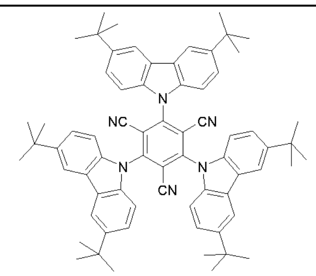 | 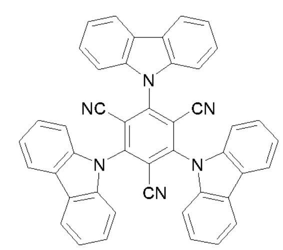 |

| | | |
|---|---|---|
| 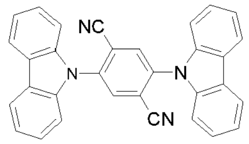 | 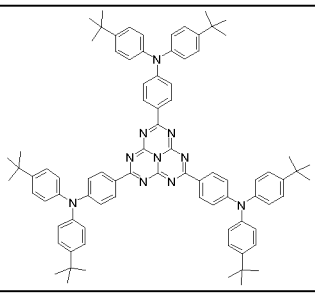 | 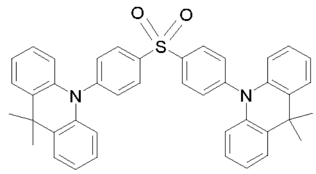 |
| 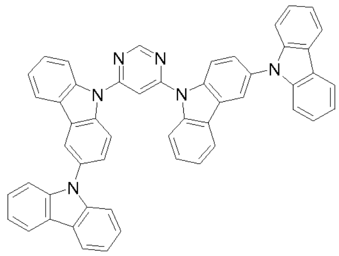 | 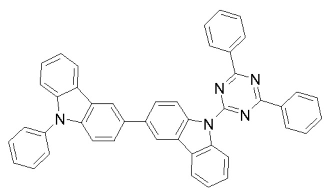 | 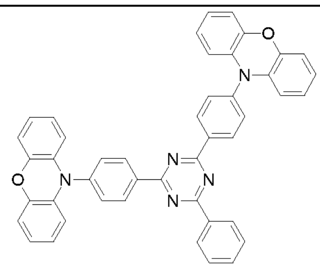 |
| 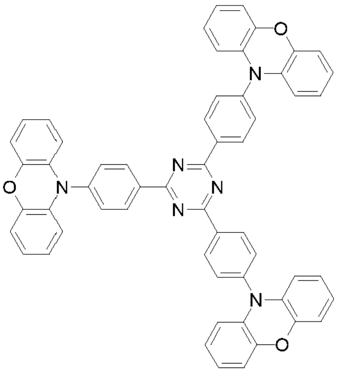 | 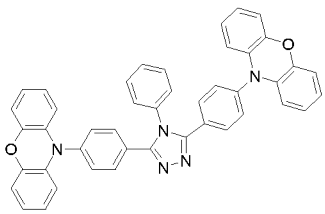 | 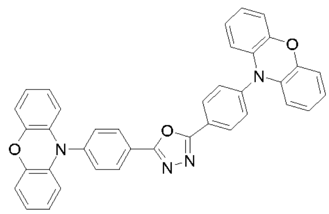 |
| 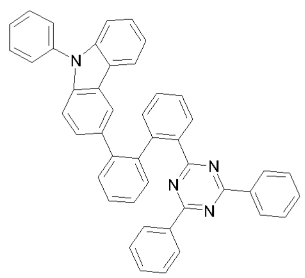 | 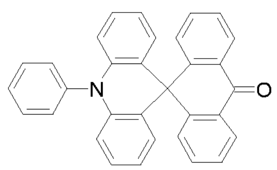 | |

In accordance with another preferred embodiment, the organic electroluminescent device comprises an emitting layer, comprising a fluorescent emitter selected from compounds of formula (1) in combination with a sensitizer selected from phosphorescent compounds.

A phosphorescent compound suitable as a sensitizer according to the invention can be any phosphorescent compound as long as the inter-system crossing rates are fast enough. One skilled in the art will have no difficulty in selecting from a variety of suitable compounds known to him the appropriate compounds for the present purpose. More particularly, a phosphorescent compound in the context of the present invention is a compound which is capable of emitting light at room temperature under optical or electrochemical excitation in an environment such as in an organic electroluminescent device, the emission being produced from a spin-forbidden transition, for example, a transition from an excited triplet state or a mixed singlet/triplet state.

Suitable phosphorescent compounds (= triplet emitters) are in particular compounds which emit light with suitable excitation, preferably in the visible range, and also at least one atom of atomic number greater than 20, preferably greater than 38 and less than 84, particularly preferably greater than 56 and smaller than 80, in particular a metal with this atomic number.

Preferably, the sensitizer is a phosphorescent compound selected from the group of the organometallic complexes, particularly from the group of the transition metal complexes.

Very preferably, the sensitizer is a phosphorescent compound, selected from organometallic complexes containing copper, molybdenum, tungsten, rhenium, ruthenium, osmium, rhodium, iridium, palladium, platinum, silver, gold or europium, particularly organometallic complexes containing copper, iridium or platinum, and very particularly organometallic complexes containing Iridium and platinum. For the purposes of the present invention, all luminescent compounds which contain the abovementioned metals are regarded as phosphorescent compounds.

Particularly preferred are the phosphorescent organometallic complexes, which are described, for example, in WO2015/091716. Also particularly preferred are the phosphorescent organometallic complexes, which are described in WO2000/70655, WO2001/41512, WO2002/02714, WO2002/15645, EP1191612, WO2005/033244, WO2005/019373, US2005/0258742, WO2006/056418, WO2007/115970, WO2007/115981, WO2008/000727, WO2009/050281, WO2009/050290, WO2011/051404, WO2011/073149, WO2012/121936, US2012/0305894, WO2012/170571, WO2012/170461, WO2012/170463, WO2006/121811, WO2007/095118, WO2008/156879, WO2008/156879, WO2010/068876, WO2011/106344, WO2012/172482, EP3126371, WO2015/014835, WO2015/014944, WO2016/020516, US20160072081, WO2010/086089, WO2011/044988, WO2014/008982, WO2014/023377, WO2014/094961, WO2010/069442, WO2012/163471, WO2013/020631, US20150243912, WO2008/000726, WO2010/015307, WO2010/054731, WO2010/054728, WO2010/099852, WO2011/032626, WO2011/157339, WO2012/007086, WO2015/036074, WO2015/104045, WO2015/117718, WO2016/015815, which are preferably iridium and platinum complexes.

Particularly preferred are also the phosphorescent organometallic complexes having polypodal ligands as described, for example, in WO2004/081017, WO2005/042550, US2005/0170206, WO2009/146770, WO2010/102709, WO2011/066898, WO2016124304, WO2017/032439, WO2018/019688, EP3184534 and WO2018/011186.

Particularly preferred are also the phosphorescent binuclear organometallic complexes as described, for example, in WO2011/045337, US20150171350, WO2016/079169, WO2018/019687, WO2018/041769, WO2018/054798, WO2018/069196, WO2018/069197, WO2018/069273.

Particularly preferred are also the copper complexes as described, for example, in WO2010/031485, US2013150581, WO2013/017675, WO2013/007707, WO2013/001086, WO2012/156378, WO2013/072508, EP2543672.

In general, all phosphorescent complexes, which are used according to the prior art for phosphorescent OLEDs and which are known to the person skilled in the art in the field of organic electroluminescence, are suitable. The person skilled in the art can use further phosphorescent complexes without any inventive step.

In a preferred embodiment of the invention, the emitting layer is produced by vapor deposition and the phosphorescent compound is present in a doping concentration of 5 to 99.9% by volume in the emitting layer, preferably from 5 to 60% by volume, very preferably from 10 to 50% by volume, most preferably from 20 to 40% by volume.

In another preferred embodiment of the invention, the emitting layer is produced via a solution process and the phosphorescent compound is present in a doping concentration of 5 to 99.9% by weight in the emitting layer, preferably from 5 to 60% by weight, particularly preferably from 10 to 50% by weight, most preferably 20 to 40% by weight.

Explicit examples of phosphorescent sensitizers are $Ir(ppy)_3$ and its derivatives as well as the structures listed below:

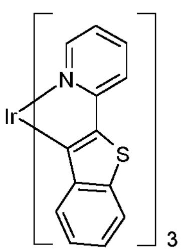
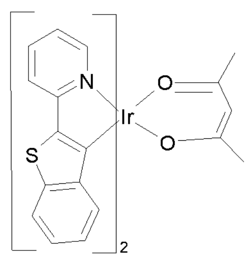
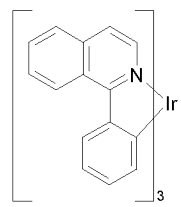

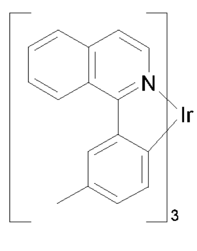
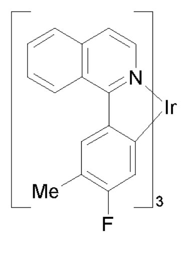
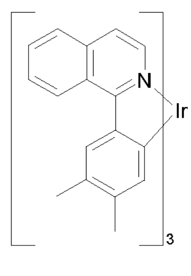

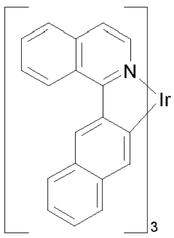
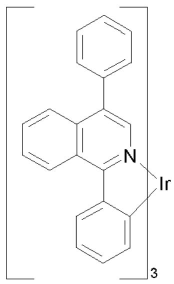
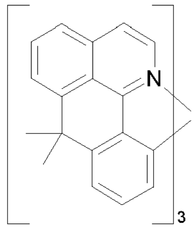
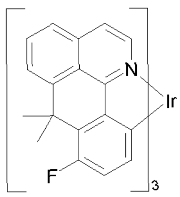
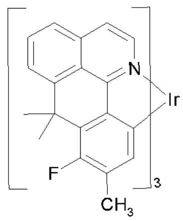
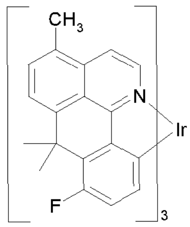
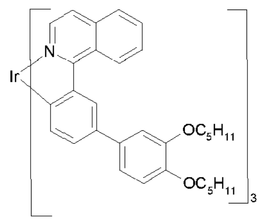
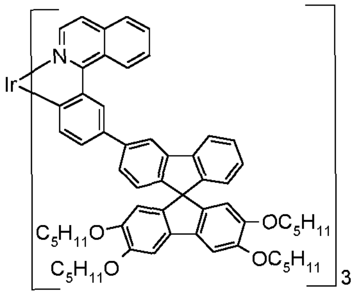
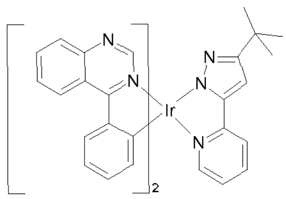
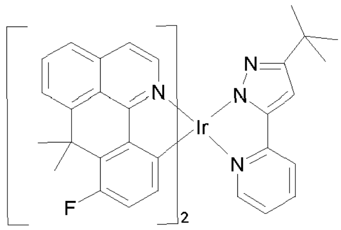
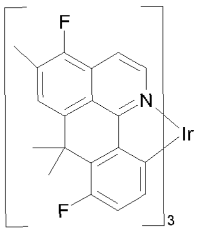
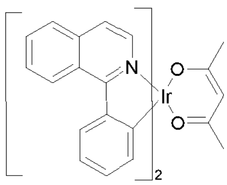
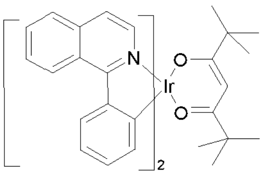
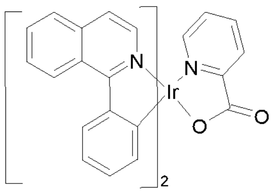
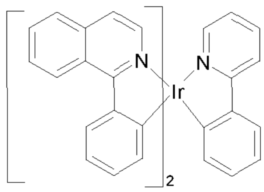
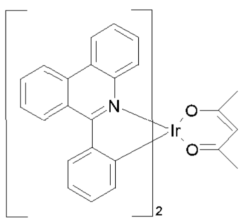
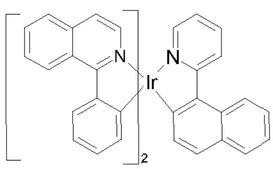
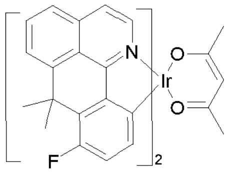

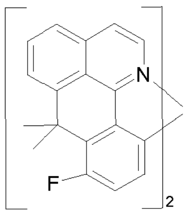
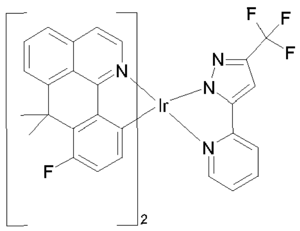
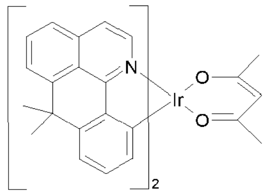
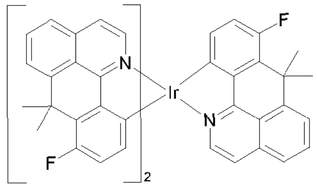
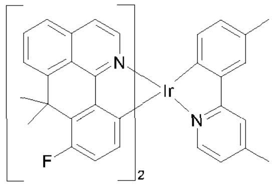
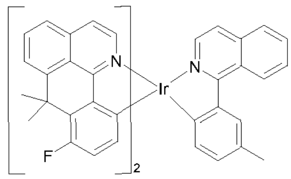
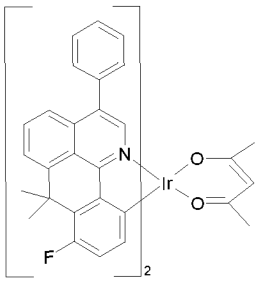
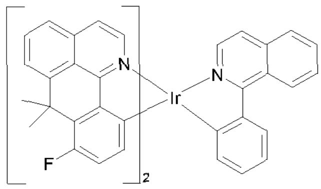
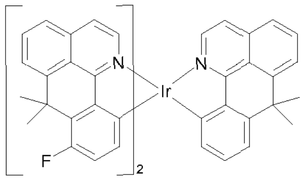
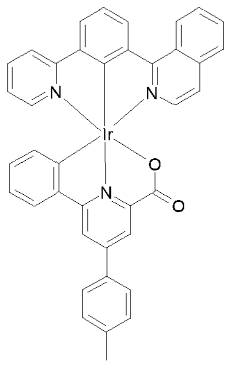
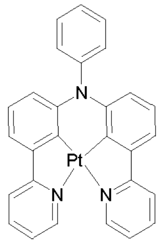
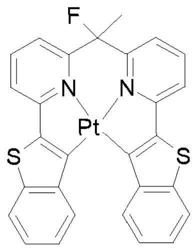
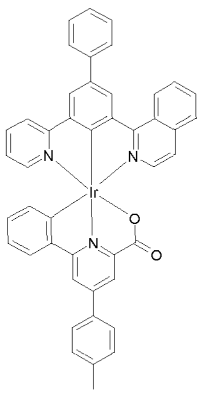
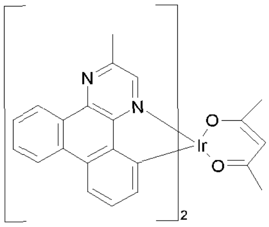
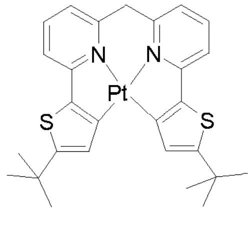

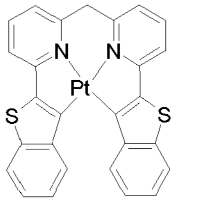
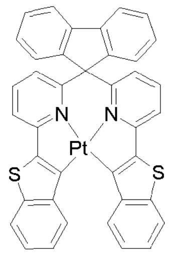
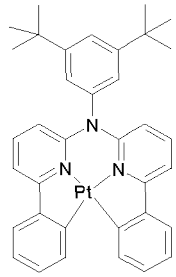

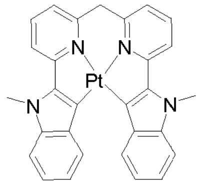
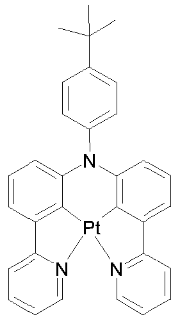
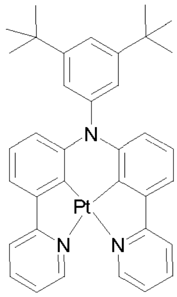

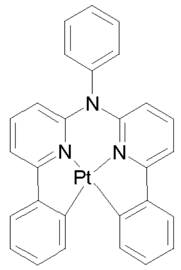
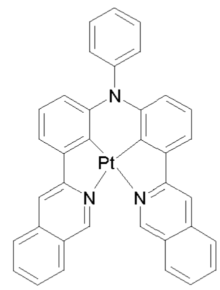

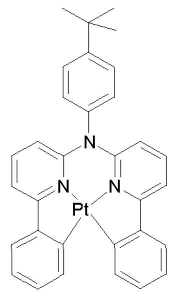
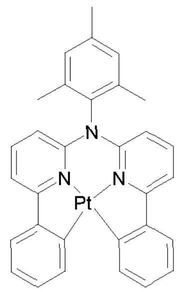
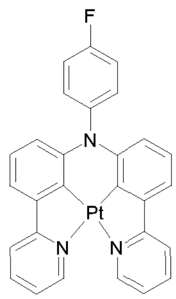

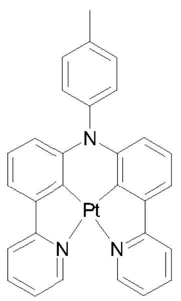
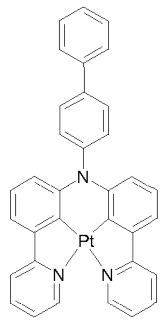
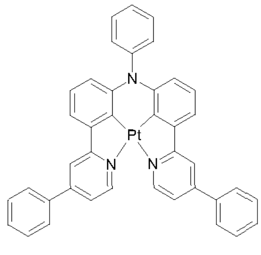

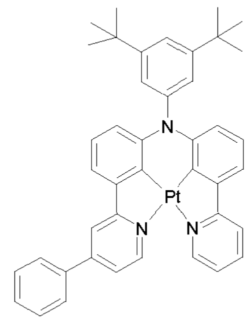
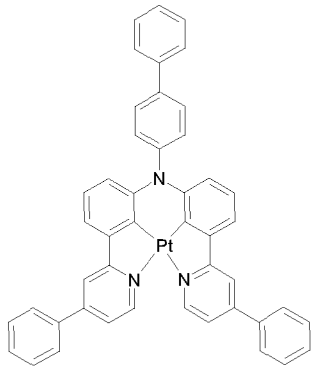
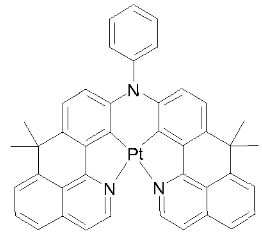

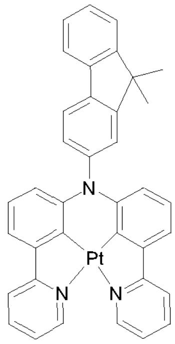
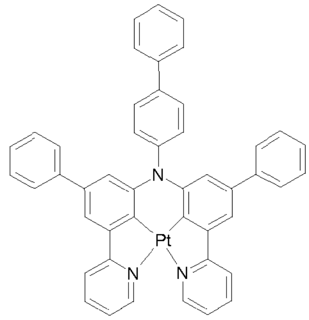
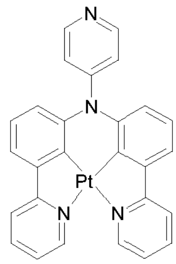

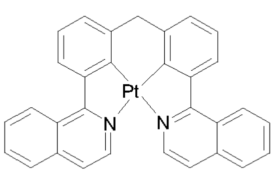
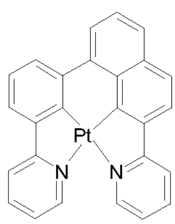
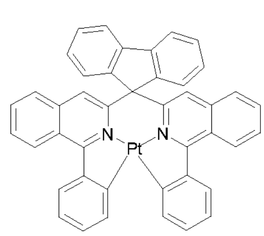

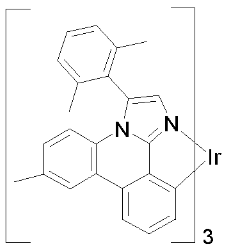
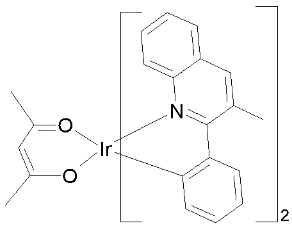
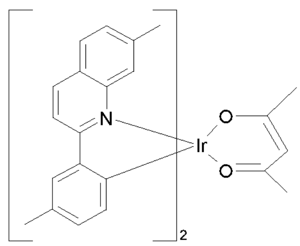

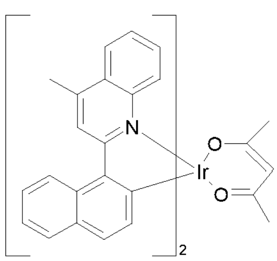
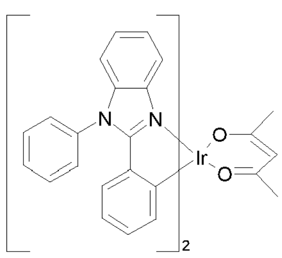
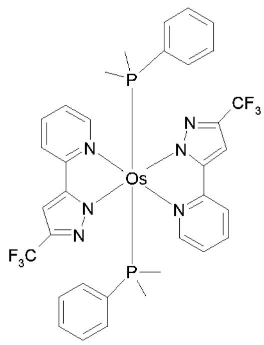

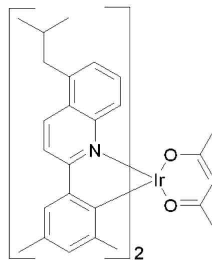
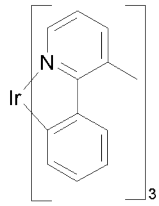
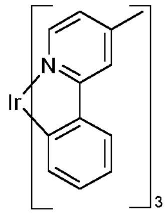
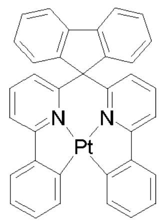
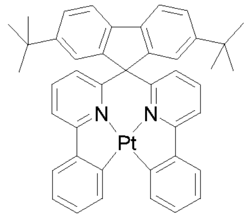
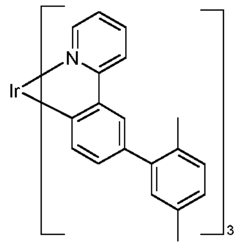
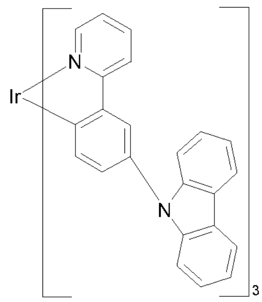
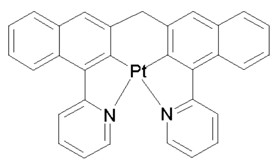
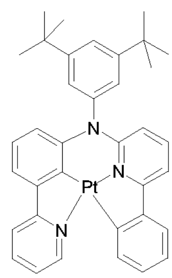
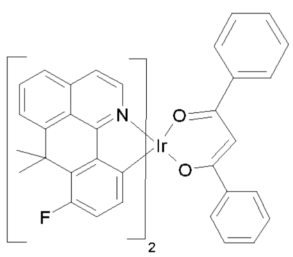
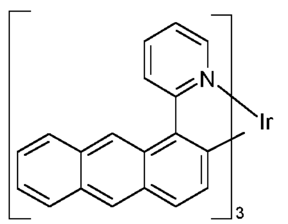
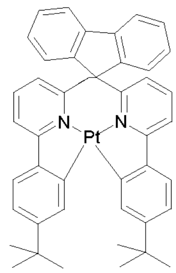
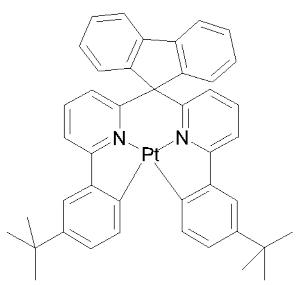
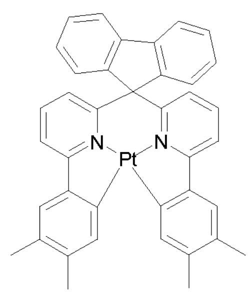
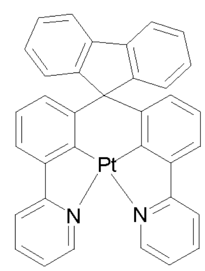

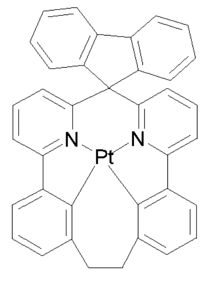
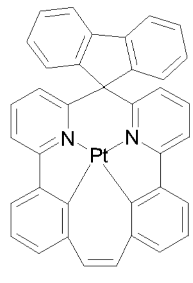
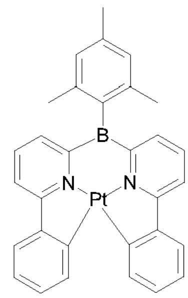

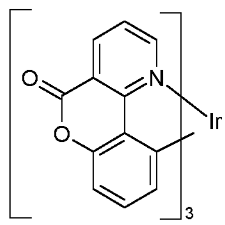
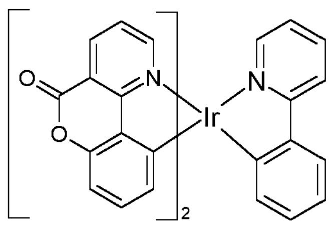
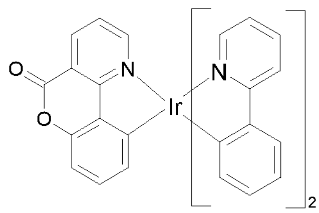

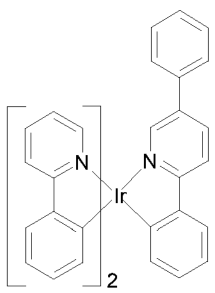
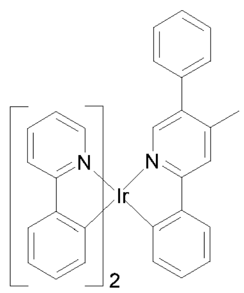
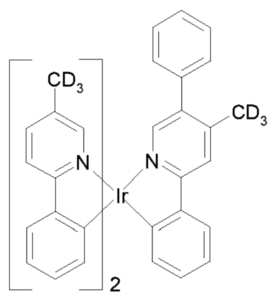

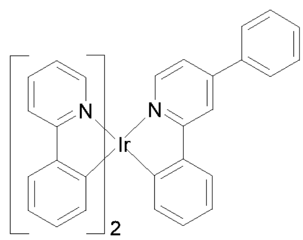
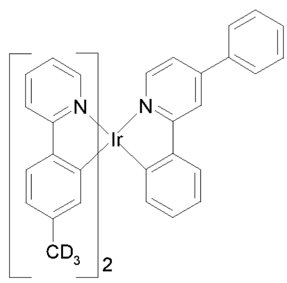
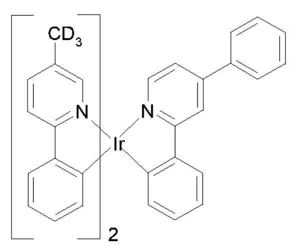

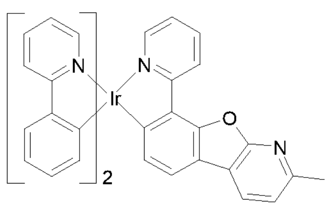
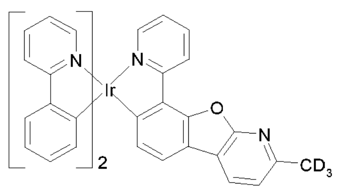
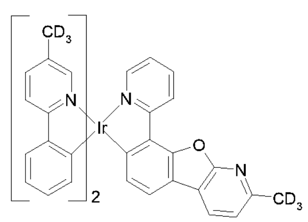

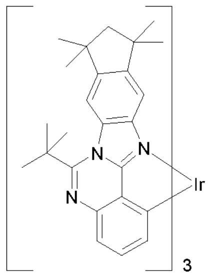

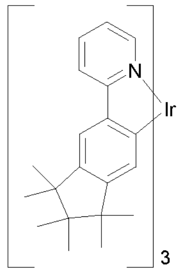

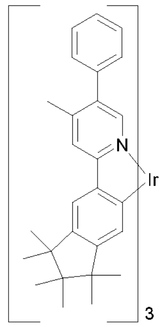

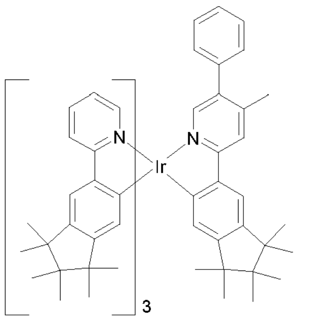

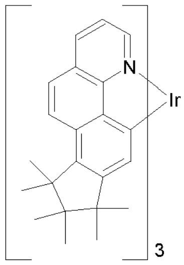

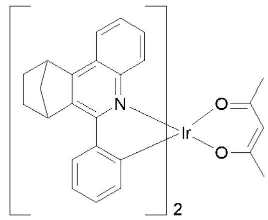

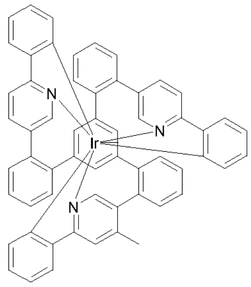

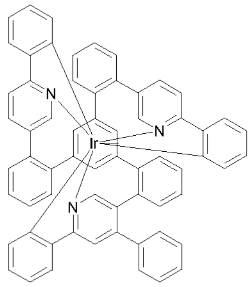

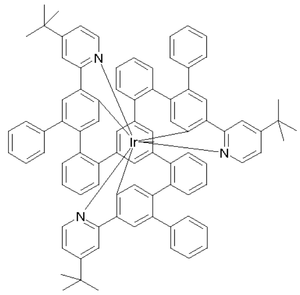

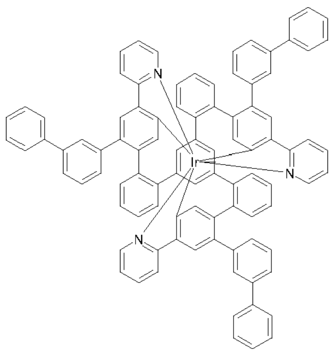

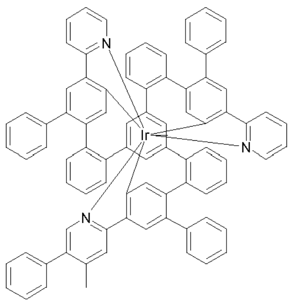

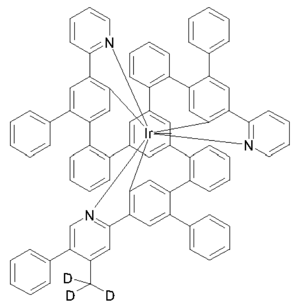

Further explicit examples of phosphorescent sensitizers are iridium and platinum complexes containing carbene ligands and the structures listed below, wherein homoleptic and heteroleptic complexes and meridonal and facial isomers may be suitable:

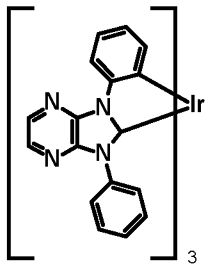 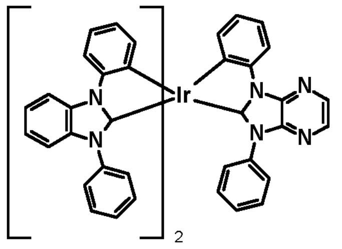 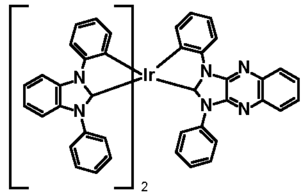

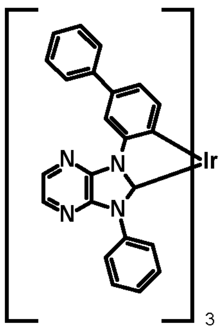 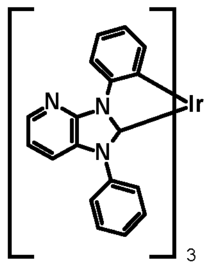 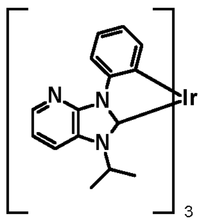

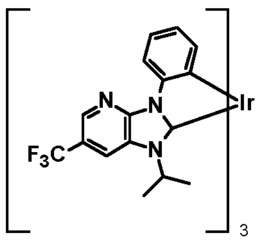 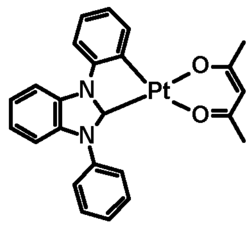 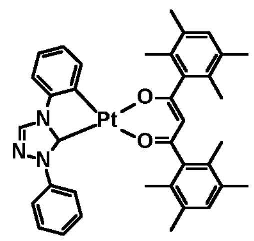

Further explicit examples of phosphorescent sensitizers are also copper complexes and the structures listed below:

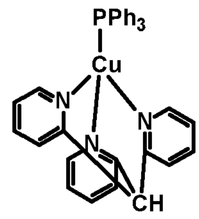 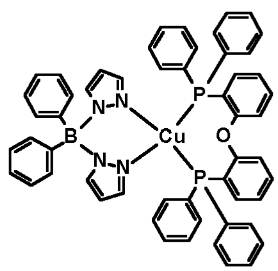 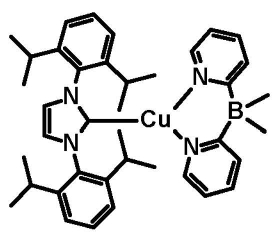

Because of the difference in production of the organic electroluminescent device, the dopant concentration of the compound of formula (1) in the case of production of the emitting layer by vapor deposition is reported in % by volume, and in the case of production of the emitting layer from solution in % by weight.

In a preferred embodiment of the invention, in the case of production of the emitting layer by vapor deposition, the compound of formula (1) is present in a dopant concentration of 0.1% to 25% by volume in the emitting layer, preferably of 1% to 20% by volume, more preferably of 2% to 12% by volume, even more preferably 3% to 10% by volume.

In a preferred embodiment of the invention, in the case of production of the emitting layer from solution, the compound of formula (1) is present in a dopant concentration of 0.1% to 25% by weight in the emitting layer, preferably of 1% to 20% by weight, more preferably of 2% to 12% by weight, even more preferably 3% to 10% by weight.

It is possible here that, especially in the case of a low dopant concentration of the compound of formula (1), the OLED exhibits mixed emission composed of the fluorescent compound and residual emission of the sensitizer compound. This can also be utilized in a controlled manner to generate mixed colors.

Still in accordance with another preferred embodiment, the organic electroluminescent device comprises an emitting layer, comprising a fluorescent emitter selected from compounds of formula (1) in combination with a sensitizer

selected from compounds that exhibit delayed fluorescence or a phosphorescent compound and at least one organic functional material selected from the group consisting of HTM, HIM, HBM, p-dopant, ETM, EIM, EBM, n-dopant, fluorescent emitter, phosphorescent emitter, delayed fluorescent material, matrix material, host material, wide band gap material, quantum material (preferably quantum dot). Preferably, the at least one organic functional material is selected from matrix materials. This further compound is referred to hereinafter as matrix compound or matrix material. This may be a further sensitizer compound in the context of the definition detailed above. In general, the matrix compound, however, is not a sensitizer compound.

In a preferred embodiment of the invention, the matrix compound makes no significant contribution, if any, to the emission of the mixture.

When the emitting layer comprises a compound of formula (1) as an emitter, a TADF compound as a sensitizer and a further matrix compound, it is preferable to avoid exciplex formation in the emitting layer. Therefore, it is preferable that the following applies to LUMO(TADF), i.e. the LUMO of the TADF compound, and the HOMO(matrix), i.e. the HOMO of the matrix compound:

LUMO(TADF) - HOMO(matrix) $\geq$ $S_1$(TADF) - 0.4 eV,
more preferably:

LUMO(TADF) - HOMO(matrix) $\geq$ $S_1$(TADF) - 0.3 eV,
and even more preferably:
LUMO(TADF) - HOMO(matrix) $\geq$ $S_1$(TADF) - 0.2 eV.
$S_1$(TADF) here is the first excited singlet state $S_1$ of the TADF compound.

The energy of the lowest excited singlet state ($S_1$) and the lowest triplet state ($T_1$) as well as the HOMO and LUMO values are determined by quantum-chemical calculations. The Gaussian09 program package (revision D or later) is used. Neutral ground state geometries of all purely organic molecules are optimized at the AM1 level of theory. Subsequently, B3PW91/6-31G(d) single point calculations including a calculation of the lowest singlet and triplet excited states with TD-B3PW91/6-31G(d). HOMO and LUMO values as well as S1 and T1 excitation energies are taken from this single-point calculation at the B3PW91/6-31G(d) level of theory.

Similarly, for metalorganic compounds, neutral ground state geometries are optimized at the HF/LANL2MB level of theory. B3PW91/6-31G(d)+LANL2DZ (LANL2DZ for all metal atoms, 6-31G(d) for all low-weight elements) is subsequently employed to calculate HOMO and LUMO values as well as TD-DFT excitation energies.

HOMO (HEh) and LUMO (LEh) values from the calculation are given in Hartree units. The HOMO and LUMO energy levels calibrated with reference to cyclic voltammetry measurements are determined therefrom in electron volts as follows:

$$\mathrm{HOMO(eV)} = ((\mathrm{HEh}*27.212)-0.9899)/1.1206$$

$$\mathrm{LUMO(eV)} = ((\mathrm{LEh}*27.212)-2.0041)/1.385$$

These values are to be regarded in the sense of the present invention as HOMO and LUMO energy levels of the materials.

The lowest triplet state $T_1$ is defined as the energy of the lowest TD-DFT triplet excitation energy.

The lowest excited singlet state $S_1$ is defined as the energy of the lowest TD-DFT singlet excitation energy.

When the emitting layer comprises a compound of formula (1) as an emitter, a sensitizer selected from TADF compounds and phosphorescent compounds, and a further matrix compound, it is also preferable that the lowest triplet energy of the matrix compound is not more than 0.1 eV lower than the triplet energy of the sensitizer compound. Especially preferably, $T_1$(matrix) $\geq$ $T_1$(sensitizer).

More preferably: $T_1$(matrix) - $T_1$(sensitizer) $\geq$ 0.1 eV;
Most preferably: $T_1$(matrix) - $T_1$(sensitizer) $\geq$ 0.2 eV.

$T_1$(matrix) here is the lowest triplet energy of the matrix compound and $T_1$(sensitizer) is the lowest triplet energy of the sensitizer compound. The triplet energy of the matrix compound $T_1$(matrix) is determined here by quantum-chemical calculation as described above.

Examples of suitable matrix compounds which can be used in the emitting layer of the invention are ketones, phosphine oxides, sulfoxides and sulfones, for example according to WO 2004/013080, WO 2004/093207, WO

2006/005627 or WO 2010/006680, triarylamines, carbazole derivatives, e.g. CBP (N,N-biscarbazolylbiphenyl), m-CBP or the carbazole derivatives disclosed in WO 2005/039246, US 2005/0069729, JP 2004/288381, EP 1205527, WO 2008/086851 or US 2009/0134784, dibenzofuran derivatives, indolocarbazole derivatives, for example according to WO 2007/063754 or WO 2008/056746, indenocarbazole derivatives, for example according to WO 2010/136109 or WO 2011/000455, azacarbazoles, for example according to EP 1617710, EP 1617711, EP 1731584, JP 2005/347160, bipolar matrix materials, for example according to WO 2007/137725, silanes, for example according to WO 2005/111172, azaboroles or boronic esters, for example according to WO 2006/117052, diazasilole derivatives, for example according to WO 2010/054729, diazaphosphole derivatives, for example according to WO 2010/054730, triazine derivatives, for example according to WO 2010/015306, WO 2007/063754 or WO 2008/056746, pyrimidine derivatives, quinoxaline derivatives, Zn complexes, Al complexes or Be complexes, for example according to EP 652273 or WO 2009/062578, or bridged carbazole derivatives, for example according to US 2009/0136779, WO 2010/050778, WO 2011/042107 or WO 2011/088877. Suitable matrix materials are also those described in WO 2015/135624. These are incorporated into the present invention by reference. It is also possible to use mixtures of two or more of these matrix materials.

Preferably, the matrix compound has a glass transition temperature $T_G$ of greater than 70°C, more preferably greater than 90°C, most preferably greater than 110°C.

The matrix compounds for the sensitizer are preferably charge-transporting, i.e. electron-transporting or hole-transporting, or bipolar compounds. Matrix compounds used may additionally also be compounds which are neither hole- nor electron-transporting in the context of the present application. An electron-transporting compound in the context of the present invention is a compound having a LUMO $\leq$ -2.50 eV. Preferably, the LUMO is $\leq$ -2.60 eV, more preferably $\leq$ -2.65 eV, most preferably $\leq$ -2.70 eV. The value of the LUMO of the compound is determined by quantum-chemical calculation, as described in general terms above. A hole-transporting compound in the context of the present invention is a compound having a HOMO $\geq$ -5.5 eV. The HOMO is preferably $\geq$ -5.4 eV, more preferably $\geq$ -5.3 eV. The HOMO is the highest occupied molecular orbital. The value of the HOMO of the compound is determined by quantum-chemical calculation, as described in above. A bipolar compound in the context of the present invention is a compound which is both hole- and electron-transporting. Suitable electron-conducting matrix compounds are selected from the substance classes of the triazines, the pyrimidines, the lactams, the metal complexes, especially the Be, Zn and Al complexes, the aromatic ketones, the aromatic phosphine oxides, the azaphospholes, the azaboroles substituted by at least one electron-conducting substituent, and the quinoxalines. In a preferred embodiment of the invention, the electron-conducting compound is a purely organic compound, i.e. a compound containing no metals.

In the further layers of the inventive organic electroluminescent device, especially in the hole injection and transport layers and in the electron injection and transport layers, it is possible to use any materials as typically used according to the prior art. The hole transport layers may also be p-doped or the electron transport layers may also be n-doped. A p-doped layer is understood to mean a layer in which free holes are generated and which has increased conductivity as a result. A comprehensive discussion of doped transport layers in OLEDs can be found in Chem. Rev. 2007, 107, 1233. More preferably, the p-dopant is capable of oxidizing the hole transport material in the hole transport layer, i.e. has a sufficiently high redox potential, especially a higher redox potential than the hole transport material. Suitable dopants are in principle any compounds which are electron acceptor compounds and which can increase the conductivity of the organic layer by oxidizing the host. The person skilled in the art, in the context of his common knowledge in the art, is able to identify suitable compounds without any great effort. Especially suitable dopants are the compounds disclosed in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709 and US 2010/0096600.

The person skilled in the art will therefore be able, without exercising inventive skill, to use all the materials known for organic electroluminescent devices in combination with the emitting layer of the invention.

Preferred cathodes are metals having a low work function, metal alloys or multilayer structures composed of various metals, for example alkaline earth metals, alkali metals, main group metals or lanthanoids (e.g. Ca, Ba, Mg, Al, In, Mg, Yb, Sm, etc.). Additionally, suitable are alloys composed of an alkali metal or alkaline earth metal and silver, for example an alloy composed of magnesium and silver. In the case of multilayer structures, in addition to the metals mentioned, it is also possible to use further metals having a relatively high work function, for example Ag, in which case combinations of the metals such as Ca/Ag or Ba/Ag, for example, are generally used. It may also be preferable to introduce a thin interlayer of a material having a high dielectric constant between a metallic cathode and the organic semiconductor. Examples of useful materials for this purpose are alkali metal or alkaline earth metal fluorides, but also the corresponding oxides or carbonates (e.g. LiF, $Li_2O$, $BaF_2$, MgO, NaF, CsF, $Cs_2CO_3$, etc.). The layer thickness of this layer is preferably between 0.5 and 5 nm.

Preferred anodes are materials having a high work function. Preferably, the anode has a work function of greater than 4.5 eV versus vacuum. Firstly, metals having a high redox potential are suitable for this purpose, for example Ag, Pt or Au. On the other hand, metal/metal oxide electrons (e.g. Al/Ni/$NiO_x$, Al/$PtO_x$) may also be preferred. In this case, at least one of the electrodes has to be transparent or semitransparent in order to enable the emission of light. A preferred structure uses a transparent anode. Preferred anode materials here are conductive mixed metal oxides. Particular

preference is given to indium tin oxide (ITO) or indium zinc oxide (IZO). Preference is further given to conductive doped organic materials, especially conductive doped polymers.

The device is correspondingly (according to the application) structured, contact-connected and finally hermetically sealed, since the lifetime of such devices is severely shortened in the presence of water and/or air.

Additionally, preferred is an organic electroluminescent device, characterized in that one or more layers are coated by a sublimation process. In this case, the materials are applied by vapor deposition in vacuum sublimation systems at an initial pressure of less than $10^{-5}$ mbar, preferably less than $10^{-6}$ mbar. It is also possible that the initial pressure is even lower, for example less than $10^{-7}$ mbar.

Preference is likewise given to an organic electroluminescent device, characterized in that one or more layers are coated by the OVPD (organic vapor phase deposition) method or with the aid of a carrier gas sublimation. In this case, the materials are applied at a pressure between $10^{-5}$ mbar and 1 bar. A special case of this method is the OVJP (organic vapor jet printing) method, in which the materials are applied directly by a nozzle and thus structured (for example, M. S. Arnold et al., Appl. Phys. Lett. 2008, 92, 053301).

Preference is additionally given to an organic electroluminescent device, characterized in that one or more layers are produced from solution, for example by spin-coating, or by any printing method, for example screen printing, flexographic printing, offset printing, LITI (light-induced thermal imaging, thermal transfer printing), inkjet printing or nozzle printing. For this purpose, soluble compounds are needed, which are obtained, for example, through suitable substitution. Since the fluorescent compound having high steric shielding typically has good solubility in a multitude of standard organic solvents by virtue of the shielding groups, the production of the emitting layer from solution is preferred.

These methods are known in general terms to those skilled in the art and can be applied by those skilled in the art without exercising inventive skill to organic electroluminescent devices comprising the compounds of the invention.

Also possible are hybrid processes, in which, for example, one or more layers are applied from solution and one or more further layers are applied by vapour deposition. Thus, it is possible, for example, to apply the emitting layer from solution and to apply the electron-transport layer by vapour deposition.

These processes are generally known to the person skilled in the art and can be applied by him without inventive step to organic electroluminescent devices comprising the compounds according to the invention.

In accordance with the invention, the electronic devices comprising one or more compounds according to the invention can be employed in displays, as light sources in lighting applications and as light sources in medical and/or cosmetic applications (for example light therapy).

The invention will now be explained in greater detail by the following examples, without wishing to restrict it thereby.

**A) Synthesis examples**

**Example A-1: 2,6,9,10-tetrakis(2,3,4,5,6-pentakis-phenylphenyl)-anthracene (1)**

**a) 2,6-Dibromo-9,10-bis(2-phenylethinyl)anthracene (2)**

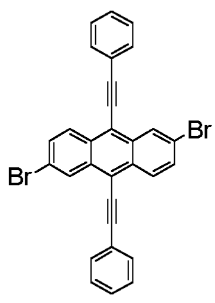

A solution of phenylacetylene (4.2 g, 4.5 mL, 41.0 mmol, 3.0 equiv.) in THF (200 mL) is placed in a three-necked flask equipped with a magnetic stirring bar under argon atmosphere. The reaction mixture is degassed with argon for 20 min before it is cooled down to -78 °C. The reaction mixture is then stirred for 10 min, before n-BuLi (2.5 M in hexane, 16.4 mL, 40.0 mmol, 2.9 equiv.) is slowly added to it. The reaction mixture is further stirred for 2 hours before 2,6-dibromo-9,10-anthracenedione (5.0 g, 13.7 mmol, 1.0 equiv.) is added to the mixture as a solid. The reaction mixture is then heated overnight at room temperature. Then, tin(II)chloride dihydrate (12.6 g, 50.0 mmol, 3.7 equiv.) is dissolved in hydrochloric acid (12 %, 20 mL) and added to the mixture. The reaction mixture is heated for 1 h under

reflux. The product **(2)** precipitates as a red solid, which is then filtered, washed with heptane and dried in vacuo (4.7 g, 8.8 mmol, 62 %).

**b) 2,6,9,10-Tetrakis(2-phenylethinyl)anthracene (3)**

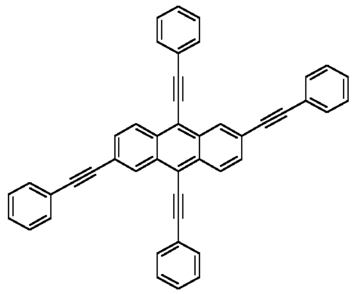

5.00 g of 2,6-Dibromo-9,10-bis(2-phenylethinyl)anthracene **(2)** (9.3 mmol, 1.0 equiv.) in TEA (1000 mL) are introduced in a four-necked flask equipped with a magnetic stirring bar under argon. The reaction mixture is degassed under argon for 20 min before CuI (0.05 g, 0.3 mmol, 0.03 equiv.), $Pd(PPh_3)_2Cl_2$ (0.13 g, 0.2 mmol, 0.02 equiv.) and phenylacetylene (5.71 g, 6.1 mL, 55.9 mmol, 6.0 equiv.) are added. The reaction mixture is then stirred for 3 h under reflux. The reaction is cooled down to room temperature. The solvent is removed. The black residue is recovered with toluene and filtered over aluminum oxide. The solvent is removed. A red solid remains, which is recrystallized from toluene (120 mL). The desired product **(3)** is isolated as a red solid (2.2 g, 5.9 mmol, 41 %).

**c) 2,6,9,10-Tetrakis(2,3,4,5,6-pentakis-phenylphenyl)-anthracene (1)**

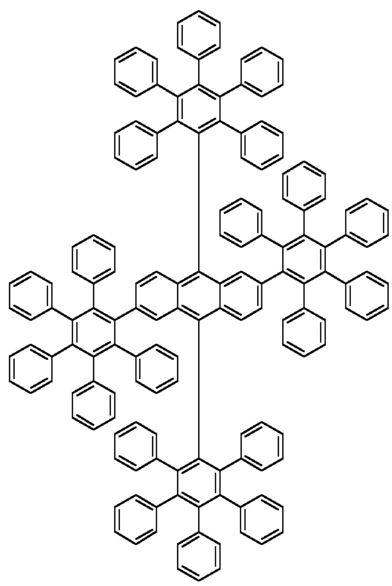

2,6,9,10-Tetrakis(2-phenylethinyl)anthracene **(3)** (24.5 g, 42.2 mmol, 1.0 equiv.) and tetraphenylcyclopenta-2,4-dienone (74.0 g, 192.5 mmol, 4.6 equiv.) are placed in a two-necked flask equipped with a magnetic stirring bar under argon. Diphenyl ether (1000 mL) is added to the reaction mixture, which is heated for 14 h under reflux. After the reaction mixture has cooled down, MeOH (5000 mL) is added. A black solid precipitates, which is filtered off. The solid is then purified by hot extraction using toluene over aluminium oxide. The solid is then recrystallized from toluene three times. The resulting solid (6.0 g, 3.0 mmol, 7 %, HPLC: 99.6 %) is recrystallized from THF. The product **(1)** is then isolated as a light yellow solid (2.0 g, 1.0 mmol, 2 %, HPLC: 99.9 %) and dried at 400 °C in a high vacuum.

**Example A-2: 9,10-Bis(2,3,4,5,6-Pentakis-phenylphenyl)-N2,N2,N6,N6-tetraphenyl-anthracene-2,6-diamine (4)**

**a) N2,N2,N6,N6-Tetraphenyl-9,10-bis(2-phenylethynyl)anthracene-2,6-diamine (5)**

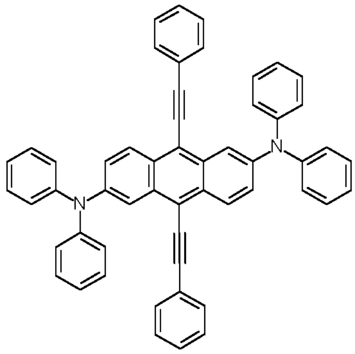

2,6-Dibromo-9,10-bis(2-phenylethinyl)anthracene **(2)** (1.35 g, 2.52 mmol, 1.0 equiv.) and of N-phenylaniline (purchased product, CAS:122-39-4) (0.94 g, 5.54 mmol, 2.2 equiv.) in dried toluene (50 mL) are placed in a four-necked flask equipped with a magnetic stirring bar under argon. The reaction mixture is degassed with argon for 20 minutes. Subsequently, sodium tert-pentoxide (0.83 g, 7.55 mmol, 3.0 equiv.), tri-tert-butylphosphine tetrafluoroborate (110 mg, 378 μmol, 0.15 equiv.) and palladium acetate (17 mg, 76 μmol, 0.03 equiv.) are added to the mixture. The reaction mixture is then gradually heated to 100 °C and stirred for 17 h at this temperature. After the reaction mixture has cooled down, an aqueous acetylcysteine solution is added and stirred for 0.5 hour, followed by the extraction with toluene. The resulting red solid is recrystallized from toluene and heptane, followed by column chromatography with DCM / heptane 1:2. The product **(5)** is then isolated as a red solid (1.4 g, 2.5 mmol, 78%).

**b) 9,10-bis(2,3,4,5,6-Pentakis-phenylphenyl)-N2, N2, N6,N6-tetraphenyl-anthracene-2,6-diamine (4)**

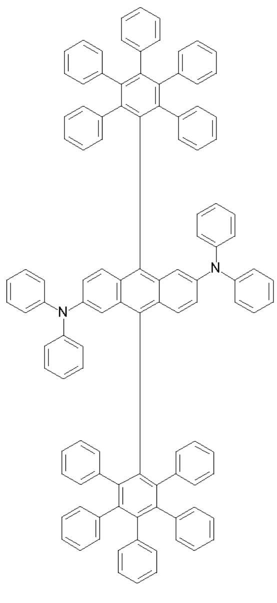

N2, N2, N6, N6-Tetraphenyl-9,10-bis(2-phenylethynyl)anthracene-2,6-diamine **(5)** (16.0 g, 22 mmol, 1.0 equiv.) and tetraphenyl-cyclopentadienone (purchased, CAS: 479-33-4) (19.37 g, 49 mmol, 2.2 equiv.) are suspended in diphenyl ether (350 mL). The suspension is degassed with argon for 20 minutes and then slowly heated to 240 °C for 100 h. The suspension is then degassed with argon. After the reaction has cooled down, methanol is added and the precipitated yellow solid is filtered off. This solid is recrystallized from toluene and anisole. The desired product **(4)** (13.90 g, 9.75

mmol, 43%) is then sublimed.

## B) Device examples

### Example B-1: Photophysical measurements

### Peak emission wavelength $\lambda_{max}$

To measure the peak emission wavelength the material is dissolved in toluene at a concentration of 1 mg/100 ml. The solution is excited in a Hitachi F-4500 fluorescence spectrometer at room temperature.

The peak emission wavelength $\lambda_{max}$ is the wavelength at which the spectrum shows the first maximum starting at low wavelengths. Typically, the first maximum is also the global maximum. If the first maximum is not the global maximum, then all maxima having an intensity of at least 0.5 or higher are considered.

### Photoluminescence quantum yield PLQY

The PLQY is determined using a Hamamatsu C9920-02 measurement system. The principle is based on the excitation of the sample with light of a defined wavelength and the measurement of the radiation absorbed and emitted. During the measurement, the sample is within an Ulbricht sphere ("integrating sphere"). The spectrum of the excitation light is approximately Gaussian with a half-height width of < 10 nm. The PLOY is determined by the evaluation method customary for said measurement system. The measurement is carried out at room temperature.

To determine the PLQY of a compound, the material is dissolved in toluene. A concentration of 1 mg/100 mL is used. The solution is filled into a suitable quartz cuvette. It is insured that the solution does not come into contact with air by handling the solution and cuvette in a glove box. Measurements at excitation wavelengths of 370, 380, 390, 400, 410 and 420nm are done. The PLQY is the mean value of the values obtained at the different wavelengths.

**Table 1: Peak emission wavelength ($\lambda_{max}$) and photoluminescence quantum yield (PLQY)**

| Compound | $\lambda_{max}$ [nm] | PLQY |
|---|---|---|
| V-01 | 416 | 0.95 |
| A-1 | 435 | 0.98 |

The inventive compound A-1 shows a peak emission wavelength in the blue spectral region which is more suitable for display applications compared to the state of the art compound V-01, which is emitting in the UV region (see Figure 1). Furthermore, A-1 shows a higher photoluminescence quantum yield of 0.98 compared to V-01 (0.95). Thus, it is better in terms of use in OLED display applications showing blue emission.

### Example B-2: Organic electroluminescent devices

Glass substrates coated with structured ITO (50 nm, indium tin oxide) are wet-cleaned (dishwasher, Merck Extran cleaner) and then treated with UV/ozone for 15 minutes. A 20 nm thick PEDOT:PSS (HC-Starck Baytron PVPA14084) layer is then spin-coated onto the substrates. The substrates are then annealed for 10 minutes on a hot plate at 180°C in air. Different layers as given in the examples are then coated on these substrates to form the OLEDs. The materials in these layers are thermally evaporated in a vacuum chamber.

After the production, the OLEDs are encapsulated under a nitrogen atmosphere in a glovebox for protection against oxygen and water vapor. The exact layer structure of the OLEDs can be found in the examples. The materials required for the production of the OLEDs are shown in Table 3. The emission layer(s) always consist(s) of at least one host material and an emitting material. An indication such as DPEPO(59%):TADF-01(40%):A-1(1%) means that the material DPEPO is present in a volume fraction of 59%, TADF-01 is present in a volume fraction of 40% and A-1 is present in a volume fraction of 1% in the layer. Similarly, the electron transport layer and hole injection layer consist of a mixture of two materials.

The OLEDs are characterised by standard methods. For this purpose, the electroluminescence spectra and luminance (measured in cd/m$^2$) are recorded using a calibrated CAS140-CT spectrometer together with a TOP200 telescopic optical probe from Instrument Systems in dependence of current density and voltage (measured with a Keithley 2635B source measure unit). The external quantum efficiency (EQE, measured in percent) is calculated assuming a lambertian emission characteristics from the current/voltage/luminance characteristic. The indication U1000 indicates the voltage required for a luminance of 1000 cd/m$^2$. EQE1000 refers to the external quantum efficiency at an operating

luminance of 1000 cd/m$^2$.

### Device examples

The following layers are thermally evaporated on top of the PEDOT:PSS layer to form the OLEDs: HTM(95%):p-D(5%) 5nm / HTM 30nm / H-02 10nm / Emission layer / H-01 10nm / ETM(50%):LiQ(50%) 20nm / Aluminum 100nm.

The results for different emission layers are given in Table 2. The OLEDs show deep blue emission with CIE x/y coordinates of 0.15/0.15 (Exp 1) and 0.15/0.14 (Exp 2)

**Table 2: Device Results**

| Ex. | Emission Layer | EQE1000 [%] | U1000 [V] |
|---|---|---|---|
| 1 | DPEPO(59%):TADF-01(40%):A-1(1%) 45nm | 7.2 | 7.6 |
| 2 | DPEPO(58%):TADF-01(40%):A-1(2%) 45nm | 7.5 | 7.2 |

**Table 3: Structures of the OLED materials**

| | |
|---|---|
| 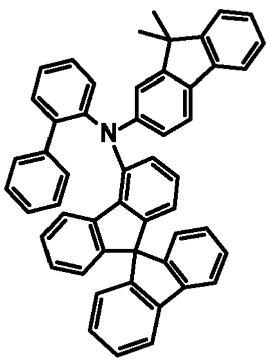 | 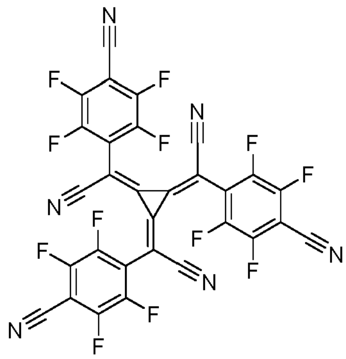 |
| HTM | p-D [US2010102709A1; WO2015007729A1] |
| 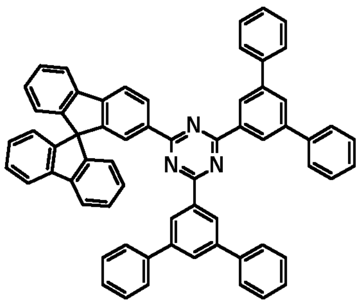 | 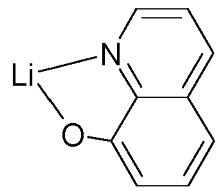 |
| ETM | LiQ |
| 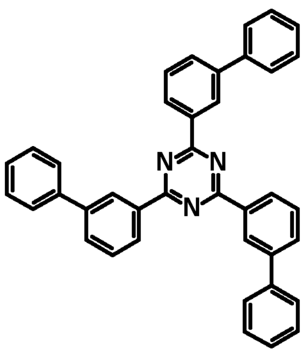 | 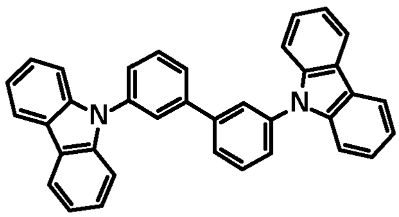 |
| H-01 | H-02 |

(continued)

| | |
|---|---|
| 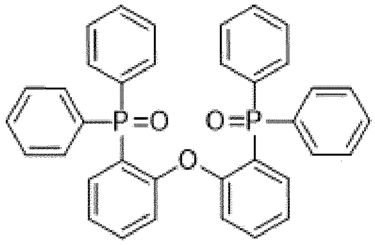 | 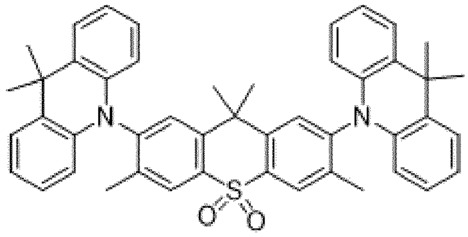 |
| DPEPO | TADF-01 |
| 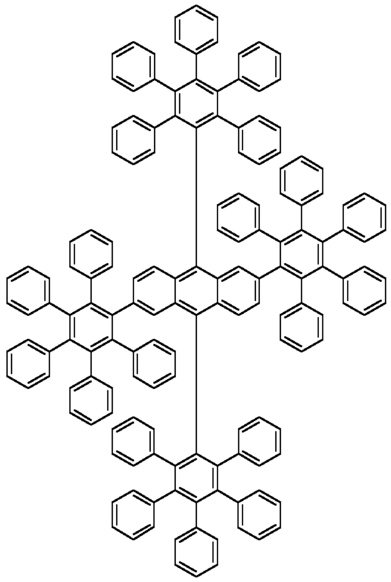 | |
| A-1 | |

## Claims

**1.** Compound of the formula (1),

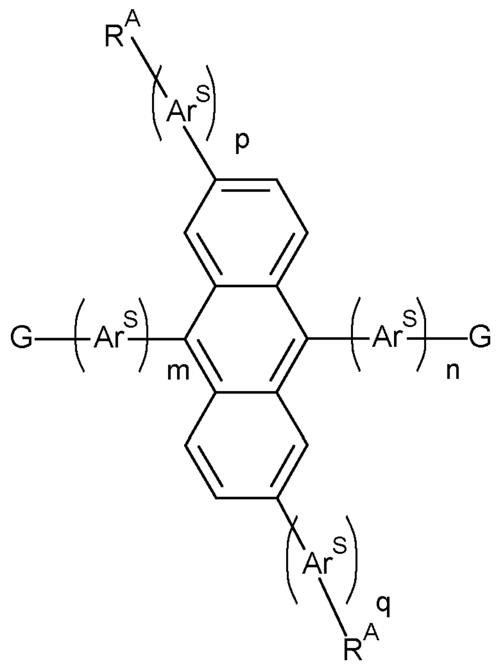

formula (1)

where the following applies to the symbols and indices used:

G is a group of formula (G-1),

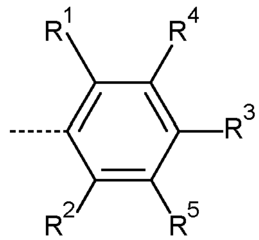

(G-1)

where the dashed bond indicates the bonding to $Ar^S$ or, if $Ar^S$ is absent, to the central benzene of the anthracene as depicted in formula (1);

$R^1$, $R^2$ stand, on each occurrence, identically or differently, for an aromatic or heteroaromatic ring system having 5 to 24 aromatic ring atoms, which may in each case also be substituted by one or more radicals R;

$R^3$, $R^4$, $R^5$ stand, on each occurrence, identically or differently, for H, D, F, Cl, Br, I, CHO, CN, $N(Ar)_2$, C(=O)Ar, $P(=O)(Ar)_2$, S(=O)Ar, $S(=O)_2Ar$, $NO_2$, $Si(R)_3$, $B(OR)_2$, $OSO_2R$, a straight-chain alkyl, alkoxy or thioalkyl group having 1 to 40 C atoms or a branched or cyclic alkyl, alkoxy or thioalkyl group having 3 to 40 C atoms, each of which may be substituted by one or more radicals R, where in each case one or more non-adjacent $CH_2$ groups may be replaced by RC=CR, C=C, $Si(R)_2$, $Ge(R)_2$, $Sn(R)_2$, C=O, C=S, C=Se, P(=O)(R), SO, $SO_2$, O, S or CONR and where one or more H atoms may be replaced by D, F, Cl, Br, I, CN or $NO_2$, an aromatic or heteroaromatic ring system having 5 to 60 aromatic ring atoms, which may in each case be substituted by one or more radicals R, an aryloxy group having 5 to 60 aromatic ring atoms, which may be substituted by one or more radicals R, or an aralkyl or heteroaralkyl group which has 5 to 60 aromatic ring atoms, which may be substituted by one or more R radicals; where two adjacent radicals selected from $R^3$, $R^4$, $R^5$ may form a mono- or polycyclic, aliphatic ring system or aromatic ring system, which may be substituted by one or more radicals R;

$R^A$ stands, on each occurrence, identically or differently, for F, CN, $N(Ar^N)_2$, C(=O)Ar, $P(=O)(Ar)_2$, S(=O)Ar, $S(=O)_2Ar$, $Si(R)_3$, $B(OR)_2$, $OSO_2R$, a straight-chain alkyl, alkoxy or thioalkyl group having 1 to 40 C atoms or a

branched or cyclic alkyl, alkoxy or thioalkyl group having 3 to 40 C atoms, each of which may be substituted by one or more radicals R, where in each case one or more non-adjacent $CH_2$ groups may be replaced by RC=CR, C=C, $Si(R)_2$, $Ge(R)_2$, $Sn(R)_2$, C=O, C=S, C=Se, P(=O)(R), SO, $SO_2$, O, S or CONR and where one or more H atoms may be replaced by D, F, Cl, Br, I, CN or $NO_2$, an aromatic or heteroaromatic ring system having 5 to 60 aromatic ring atoms, which may in each case be substituted by one or more radicals R, an aryloxy group having 5 to 60 aromatic ring atoms, which may be substituted by one or more radicals R, or an aralkyl or heteroaralkyl group which has 5 to 60 aromatic ring atoms, which may be substituted by one or more R radicals;

$Ar^N$ stands, on each occurrence, identically or differently, for an aromatic or heteroaromatic ring system having 5 to 60 aromatic ring atoms, which may in each case also be substituted by one or more radicals R,and wherein two groups $Ar^N$ may be connected with one another by a single bond or by a divalent bridge selected from N(R), O, S, $C(R)_2$, $C(R)_2$-$C(R)_2$, $Si(R)_2$ or B(R);

$Ar^S$ stands, on each occurrence, identically or differently, for a single bond or for an aromatic or heteroaromatic ring system having 5 to 40 aromatic ring atoms, which may in each case also be substituted by one or more radicals R;

R stands on each occurrence, identically or differently, for H, D, F, Cl, Br, I, CHO, CN, $N(Ar)_2$, C(=O)Ar, $P(=O)(Ar)_2$, S(=O)Ar, $S(=O)_2Ar$, $NO_2$, $Si(R')_3$, $B(OR')_2$, $OSO_2R'$, a straight-chain alkyl, alkoxy or thioalkyl group having 1 to 40 C atoms or a branched or cyclic alkyl, alkoxy or thioalkyl group having 3 to 40 C atoms, each of which may be substituted by one or more radicals R', where in each case one or more non-adjacent $CH_2$ groups may be replaced by R'C=CR', C=C, $Si(R')_2$, $Ge(R')_2$, $Sn(R')_2$, C=O, C=S, C=Se, P(=O)(R'), SO, $SO_2$, O, S or CONR' and where one or more H atoms may be replaced by D, F, Cl, Br, I, CN or $NO_2$, an aromatic or heteroaromatic ring system having 5 to 60 aromatic ring atoms, which may in each case be substituted by one or more radicals R', or an aryloxy group having 5 to 60 aromatic ring atoms, which may be substituted by one or more radicals R', where two adjacent radicals R may form a mono- or polycyclic, aliphatic ring system or aromatic ring system, which may be substituted by one or more radicals R';

Ar is on each occurrence, identically or differently, an aromatic or heteroaromatic ring system having 5 to 24 aromatic ring atoms, which may in each case also be substituted by one or more radicals R';

R' stands on each occurrence, identically or differently, for H, D, F, Cl, Br, I, CN, a straight-chain alkyl, alkoxy or thioalkyl group having 1 to 20 C atoms or a branched or cyclic alkyl, alkoxy or thioalkyl group having 3 to 20 C atoms, where in each case one or more non-adjacent $CH_2$ groups may be replaced by SO, $SO_2$, O, S and where one or more H atoms may be replaced by D, F, Cl, Br or I, or an aromatic or heteroaromatic ring system having 5 to 24 C atoms; and

n, m, p, q are, identically or differently, 0,1 or 2.

2. Compound according to one or more of the preceding claims, **characterized in that** $R^3$, $R^4$, $R^5$ stand, on each occurrence, identically or differently, for a straight-chain alkyl or alkoxy group having 1 to 10 C atoms or a branched or cyclic alkyl or alkoxy group having 3 to 40 C atoms, each of which may be substituted by one or more radicals R, where in each case one or more non-adjacent $CH_2$ groups may be replaced by RC=CR, C=C, $Si(R)_2$, $Ge(R)_2$, $Sn(R)_2$, C=O, C=S, C=Se, P(=O)(R), SO, $SO_2$, O, S or CONR and where one or more H atoms may be replaced by D, F, Cl, Br, I, CN or $NO_2$, an aromatic or heteroaromatic ring system having 5 to 40 aromatic ring atoms, which may in each case be substituted by one or more radicals R, an aryloxy group having 5 to 40 aromatic ring atoms, which may be substituted by one or more radicals R, or an aralkyl or heteroaralkyl group which has 5 to 40 aromatic ring atoms, which may be substituted by one or more R radicals.

3. Compound according to one or more of the preceding claims, **characterized in that** $R^3$, $R^4$, $R^5$ stand, on each occurrence, identically or differently, for a straight-chain alkyl or alkoxy group having 1 to 10 C atoms or a branched or cyclic alkyl or alkoxy group having 3 to 40 C atoms, each of which may be substituted by one or more radicals R, where in each case one or more non-adjacent $CH_2$ groups may be replaced by RC=CR, C=C, $Si(R)_2$, $Ge(R)_2$, $Sn(R)_2$, C=O, C=S, C=Se, P(=O)(R), SO, $SO_2$, O, S or CONR and where one or more H atoms may be replaced by D, F, Cl, Br, I, CN or $NO_2$, an aromatic or heteroaromatic ring system having 5 to 40 aromatic ring atoms, which may in each case be substituted by one or more radicals R, an aryloxy group having 5 to 40 aromatic ring atoms, which may be substituted by one or more radicals R, or an aralkyl or heteroaralkyl group which has 5 to 40 aromatic ring atoms, which may be substituted by one or more R radicals.

4. Compound according to one or more of the preceding claims, **characterized in that** $R^3$, $R^4$, $R^5$ stand, on each occurrence, identically or differently, for an aromatic or heteroaromatic ring system having 5 to 24 aromatic ring atoms, which may in each case also be substituted by one or more radicals R

5. Compound according to one or more of the preceding claims, **characterized in that** the group G stands for a group

of formula (G-2), (G-3) or (G-4),

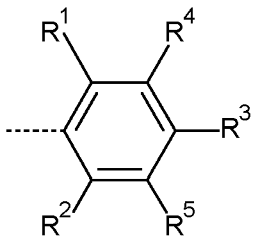

(G-2)

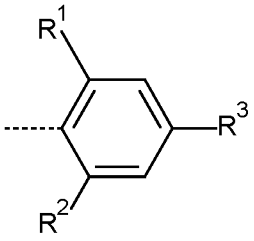

(G-3)

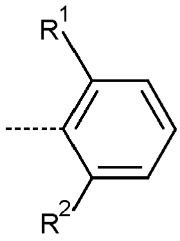

(G-4)

where $R^1$ and $R^2$ have the same definition as in claim 1; and
$R^3$, $R^4$, $R^5$ stand on each occurrence, identically or differently for a straight-chain alkyl, alkoxy or thioalkyl group having 1 to 40 C atoms or a branched or cyclic alkyl, alkoxy or thioalkyl group having 3 to 40 C atoms, each of which may be substituted by one or more radicals R, where in each case one or more non-adjacent $CH^2$ groups may be replaced by RC=CR, C=C, $Si(R)^2$, $Ge(R)^2$, $Sn(R)^2$, C=O, C=S, C=Se, P(=O)(R), SO, $SO_2$, O, S or CONR and where one or more H atoms may be replaced by D, F, Cl, Br, I, CN or $NO_2$, an aromatic or heteroaromatic ring system having 5 to 60 aromatic ring atoms, which may in each case be substituted by one or more radicals R, an aryloxy group having 5 to 60 aromatic ring atoms, which may be substituted by one or more radicals R, or an aralkyl or heteroaralkyl group which has 5 to 60 aromatic ring atoms, which may be substituted by one or more R radicals.

**6.** Compound according to one or more of the preceding claims, **characterized in that** $R^A$ stands, on each occurrence, identically or differently, for F, CN, $N(Ar^N)_2$, C(=O)Ar, $P(=O)(Ar)_2$, S(=O)Ar, $S(=O)_2Ar$, $Si(R)_3$, $B(OR)_2$, $OSO_2R$, a straight-chain alkyl, alkoxy or thioalkyl group having 1 to 40 C atoms or a branched or cyclic alkyl, alkoxy or thioalkyl group having 3 to 40 C atoms, each of which may be substituted by one or more radicals R, where in each case one or more non-adjacent $CH_2$ groups may be replaced by RC=CR, C=C, $Si(R)_2$, $Ge(R)_2$, $Sn(R)_2$, C=O, C=S, C=Se, P(=O)(R), SO, $SO_2$, O, S or CONR and where one or more H atoms may be replaced by D, F, Cl, Br, I, CN or $NO_2$, an aromatic or heteroaromatic ring system having 5 to 60 aromatic ring atoms, which may in each case be substituted by one or more radicals R, an aryloxy group having 5 to 60 aromatic ring atoms, which may be substituted by one or more radicals R, or an aralkyl or heteroaralkyl group which has 5 to 60 aromatic ring atoms, which may be substituted by one or more R radicals.

**7.** Compound according to one or more of the preceding claims, **characterized in that** $R^A$ is selected on each occurrence, identically or differently,

- from $N(Ar^N)_2$; where $Ar^N$ has the same definition as in claim 1;
- from branched or cyclic alkyl groups represented by the general following formula (RS-a)

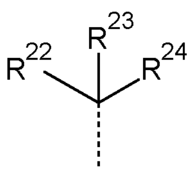

(RS-a)

wherein

$R^{22}$, $R^{23}$, $R^{24}$ are at each occurrence, identically or differently, selected from H, a straight-chain alkyl group having 1 to 10 carbon atoms, or a branched or cyclic alkyl group having 3 to 10 carbon atoms, where the above-mentioned groups may each be substituted by one or more radicals $R^{25}$, and where two of radicals $R^{22}$, $R^{23}$, $R^{24}$ or all radicals $R^{22}$, $R^{23}$, $R^{24}$ may be joined to form a (poly)cyclic alkyl group, which may be substituted by one or more radicals $R^{25}$;
$R^{25}$ is at each occurrence, identically or differently, selected from a straight-chain alkyl group having 1 to

10 carbon atoms, or a branched or cyclic alkyl group having 3 to 10 carbon atoms; with the proviso that at each occurrence at least one of radicals $R^{22}$, $R^{23}$ and $R^{24}$ is other than H, with the proviso that at each occurrence all of radicals $R^{22}$, $R^{23}$ and $R^{24}$ together have at least 4 carbon atoms and with the proviso that at each occurrence, if two of radicals $R^{22}$, $R^{23}$, $R^{24}$ are H, the remaining radical is not a straight-chain;

- or from branched or cyclic alkoxy groups represented by the general following formula (RS-b)

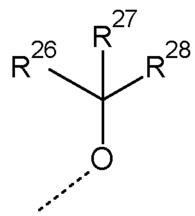

(RS-b)

wherein
$R^{26}$, $R^{27}$, $R^{28}$ are at each occurrence, identically or differently, selected from H, a straight-chain alkyl group having 1 to 10 carbon atoms, or a branched or cyclic alkyl group having 3 to 10 carbon atoms, where the above-mentioned groups may each be substituted by one or more radicals $R^{25}$ as defined above, and where two of radicals $R^{26}$, $R^{27}$, $R^{28}$ or all radicals $R^{26}$, $R^{27}$, $R^{28}$ may be joined to form a (poly)cyclic alkyl group, which may be substituted by one or more radicals $R^{25}$ as defined above; with the proviso that at each occurrence only one of radicals $R^{26}$, $R^{27}$ and $R^{28}$ may be H;
- or from aralkyl groups represented by the general following formula (RS-c)

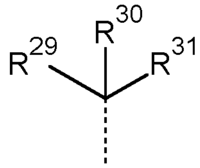

(RS-c)

wherein

$R^{29}$, $R^{30}$, $R^{31}$ are at each occurrence, identically or differently, selected from H, a straight-chain alkyl group having 1 to 10 carbon atoms, or a branched or cyclic alkyl group having 3 to 10 carbon atoms, where the above-mentioned groups may each be substituted by one or more radicals $R^{32}$, or an aromatic ring system having 6 to 30 aromatic ring atoms, which may in each case be substituted by one or more radicals $R^{32}$, and where two or all of radicals $R^{29}$, $R^{30}$, $R^{31}$ may be joined to form a (poly)cyclic alkyl group or an aromatic ring system, each of which may be substituted by one or more radicals $R^{32}$;
$R^{32}$ is at each occurrence, identically or differently, selected from a straight-chain alkyl group having 1 to 10 carbon atoms, or a branched or cyclic alkyl group having 3 to 10 carbon atoms, or an aromatic ring system having 6 to 24 aromatic ring atoms;
with the proviso that at each occurrence at least one of radicals $R^{29}$, $R^{30}$ and $R^{31}$ is other than H and that at each occurrence at least one of radicals $R^{29}$, $R^{30}$ and $R^{31}$ is or contains an aromatic ring system having at least 6 aromatic ring atoms;

- or from aromatic ring systems represented by the general following formula (RS-d)

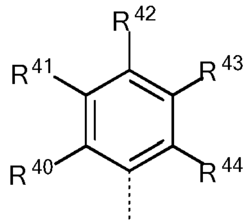

(RS-d)

wherein

$R^{40}$ to $R^{44}$ is at each occurrence, identically or differently, selected from H, a straight-chain alkyl group having 1 to 10 carbon atoms, or a branched or cyclic alkyl group having 3 to 10 carbon atoms, where the above-mentioned groups may each be substituted by one or more radicals $R^{32}$, or an aromatic ring system having 6 to 30 aromatic ring atoms, which may in each case be substituted by one or more radicals $R^{32}$, and where two or more of radicals $R^{40}$ to $R^{44}$ may be joined to form a (poly)cyclic alkyl group or an aromatic ring system, each of which may be substituted by one or more radicals $R^{32}$ as defined above.

**8.** Compound according to one or more of the preceding claims, **characterized in that** $Ar^S$ stands for benzene, biphenyl, terphenyl, naphthalene, fluorene, indenofluorene, spirobifluorene, triazine, benzoquinoline, benzoquinazoline, dibenzofuran, dibenzothiophene, and carbazole, where each of the above-mentioned groups may be substituted by one or more radicals R.

**9.** Compound according to one or more of the preceding claims, **characterized in that** m = n = 0.

**10.** Compound according to one or more of the preceding claims, **characterized in that** p = q = 0.

**11.** Compound according to one or more of the preceding claims, **characterized in that** it is selected from compounds of formula (2), (3) or (4),

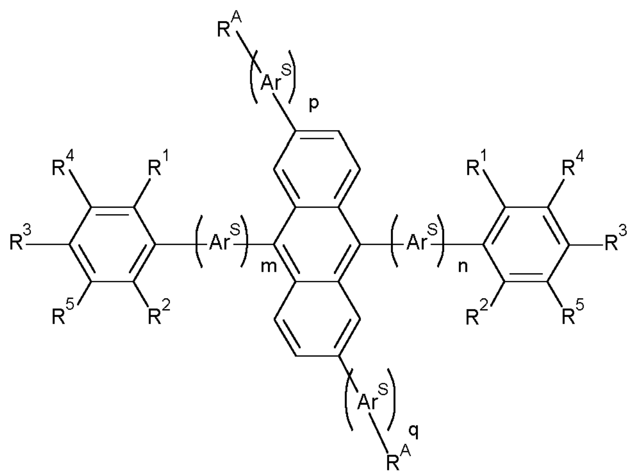

formula (2)

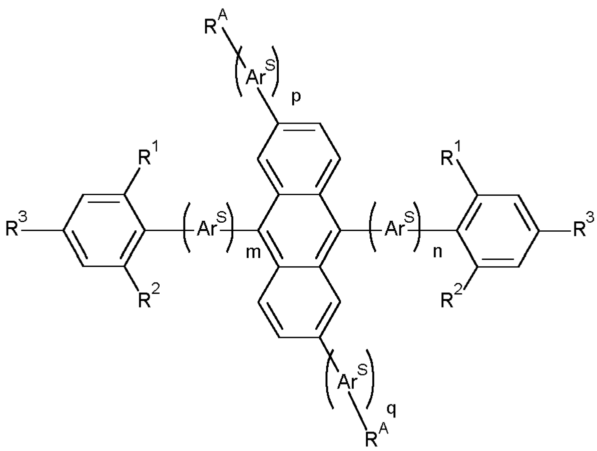

formula (3)

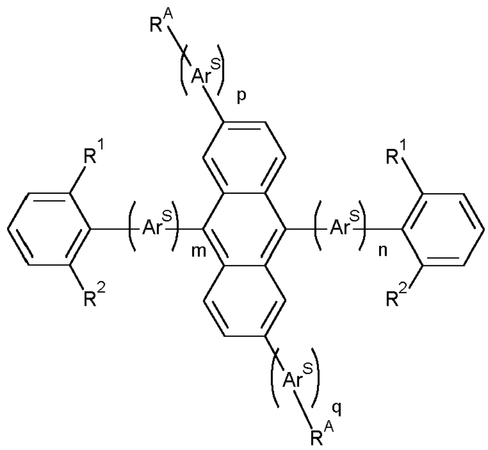

formula (4)

where $R^3$, $R^4$, $R^5$ stand on each occurrence, identically or differently for a straight-chain alkyl, alkoxy or thioalkyl group having 1 to 40 C atoms or a branched or cyclic alkyl, alkoxy or thioalkyl group having 3 to 40 C atoms, each of which may be substituted by one or more radicals R, where in each case one or more non-adjacent $CH^2$ groups may be replaced by RC=CR, C=C, $Si(R)^2$, $Ge(R)^2$, $Sn(R)^2$, C=O, C=S, C=Se, P(=O)(R), SO, $SO_2$, O, S or CONR and where one or more H atoms may be replaced by D, F, Cl, Br, I, CN or $NO_2$, an aromatic or heteroaromatic ring system having 5 to 60 aromatic ring atoms, which may in each case be substituted by one or more radicals R, an aryloxy group having 5 to 60 aromatic ring atoms, which may be substituted by one or more radicals R, or an aralkyl or heteroaralkyl group which has 5 to 60 aromatic ring atoms, which may be substituted by one or more R radicals; and
where the other symbols and indices have the same meaning as in claim 1.

**12.** Compound according to one or more of the preceding claims, **characterized in that** it is selected from compounds of formulae (2-1a) to (2-4a), (4-1a) to (4-3a),

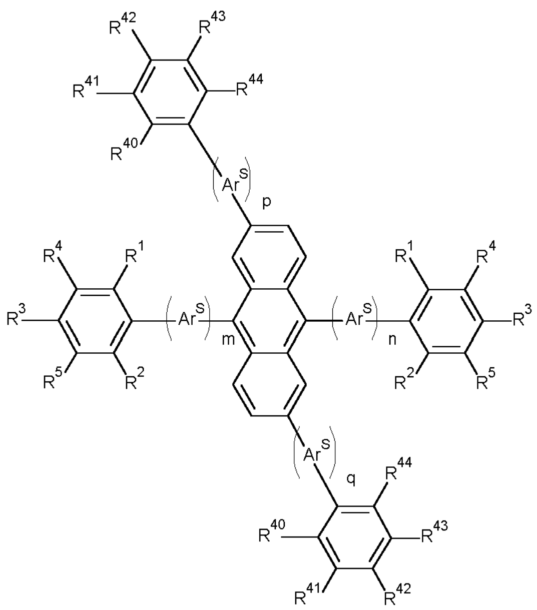

formula (2-1-a)

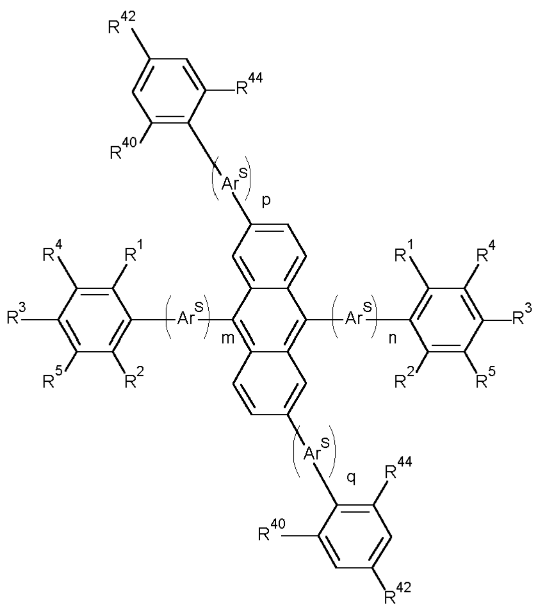

formula (2-2-a)

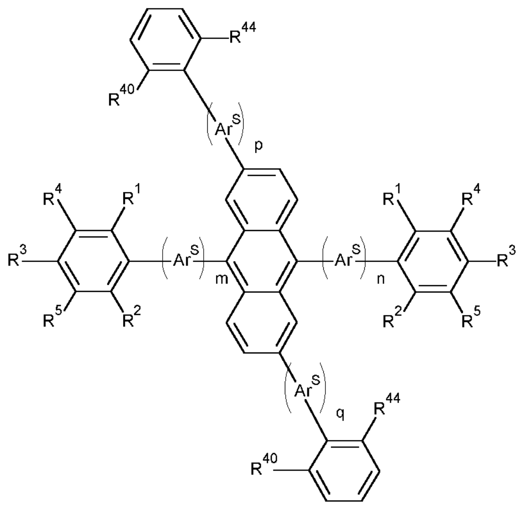

formula (2-3-a)

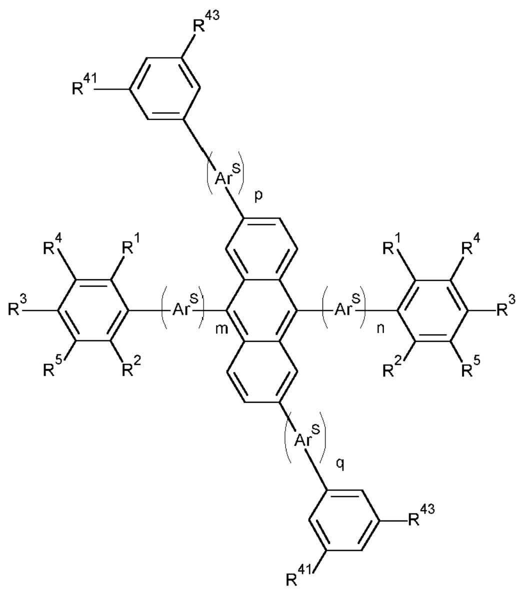

formula (2-4-a)

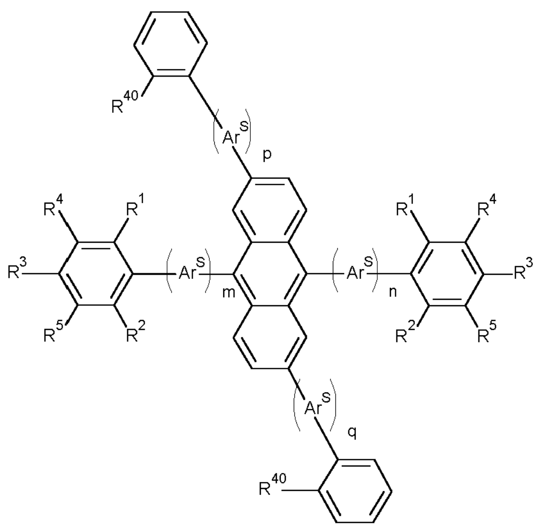

formula (2-5-a)

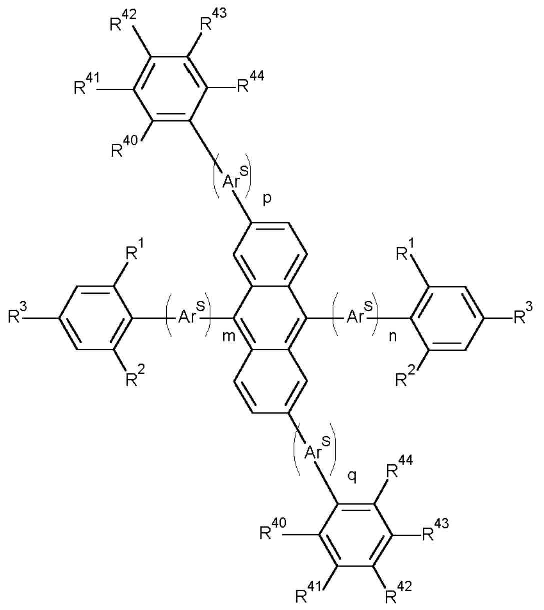

formula (3-1-a)

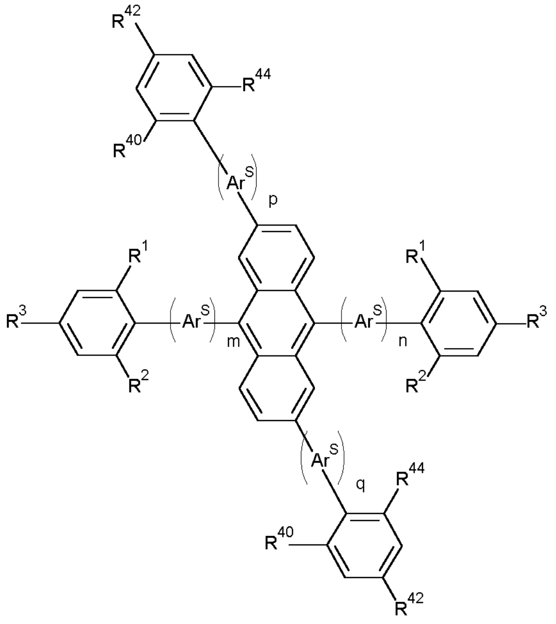

formula (3-2-a)

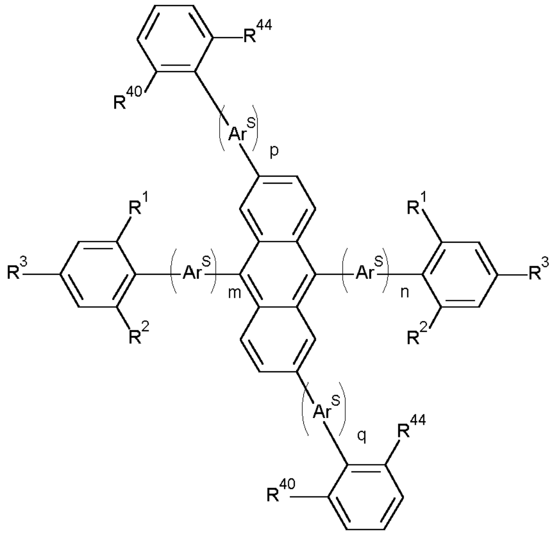

formula (3-3-a)

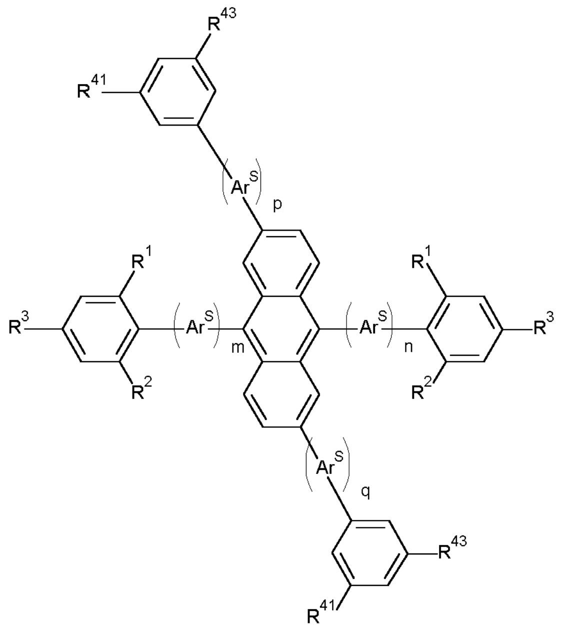

formula (3-4-a)

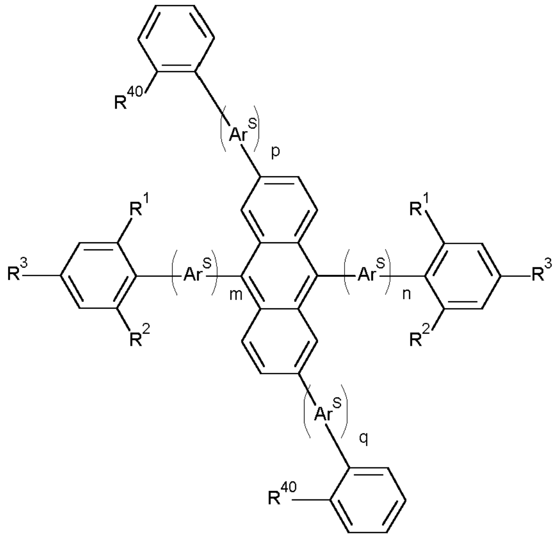

formula (3-5-a)

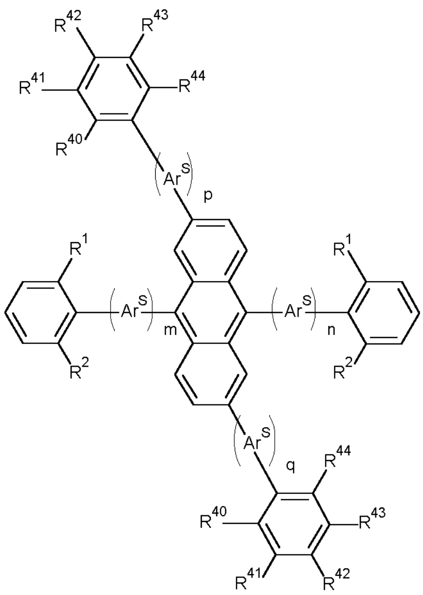

formula (4-1-a)

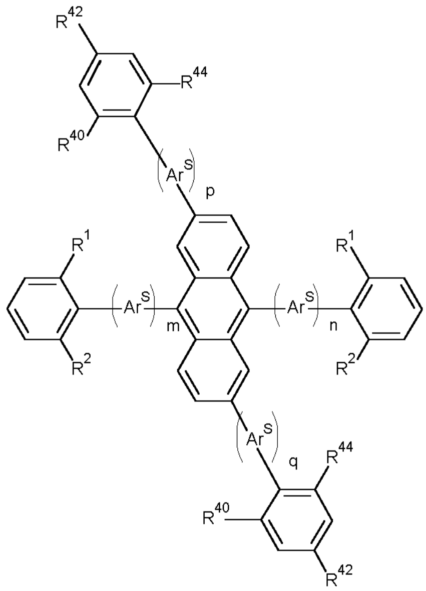

formula (4-2-a)

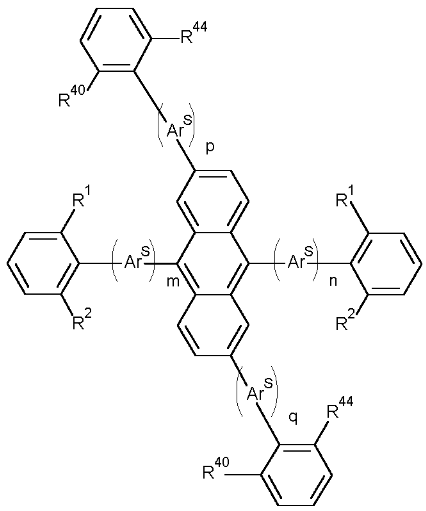

formula (4-3-a)

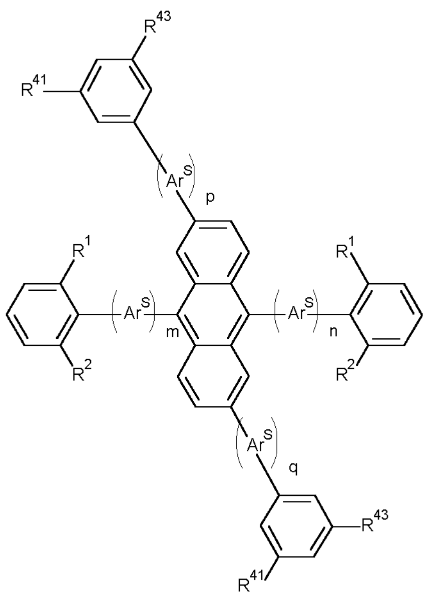

formula (4-4-a)

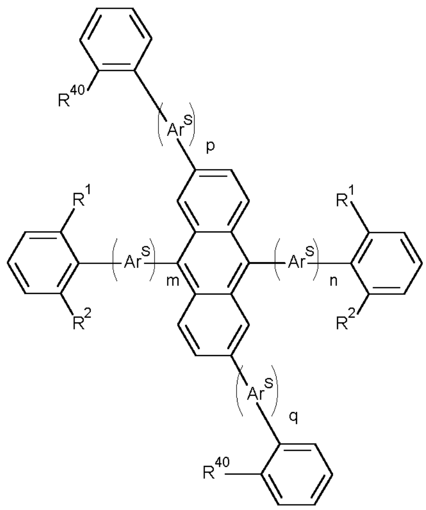

formula (4-5-a)

where

$R^3$, $R^4$, $R^5$ stand on each occurrence, identically or differently for a straight-chain alkyl, alkoxy or thioalkyl group having 1 to 40 C atoms or a branched or cyclic alkyl, alkoxy or thioalkyl group having 3 to 40 C atoms, each of which may be substituted by one or more radicals R, where in each case one or more non-adjacent $CH^2$ groups may be replaced by RC=CR, C=C, $Si(R)^2$, $Ge(R)^2$, $Sn(R)^2$, C=O, C=S, C=Se, P(=O)(R), SO, $SO_2$, O, S or CONR and where one or more H atoms may be replaced by D, F, Cl, Br, I, CN or $NO_2$, an aromatic or heteroaromatic ring system having 5 to 60 aromatic ring atoms, which may in each case be substituted by one or more radicals R, an aryloxy group having 5 to 60 aromatic ring atoms, which may be substituted by one or more radicals R, or an aralkyl or heteroaralkyl group which has 5 to 60 aromatic ring atoms, which may be substituted by one or more R radicals;

$R^{40}$ to $R^{44}$ are on each occurrence, identically or differently, selected from a straight-chain alkyl group having 1 to 10 carbon atoms, or a branched or cyclic alkyl group having 3 to 10 carbon atoms, where the above-mentioned groups may each be substituted by one or more radicals $R^{32}$, or an aromatic ring system having 6 to 30 aromatic ring atoms, which may in each case be substituted by one or more radicals $R^{32}$; where $R^{32}$ is as defined in claim 6;

and where $R^1$, $R^2$, R, m, n, p and q have the same definition than in claim 1.

**13.** Compound according to claim 11, **characterized in that** $R^{40}$ to $R^{44}$ are at each occurrence, identically or differently, selected from an aromatic ring system having 6 to 30 aromatic ring atoms, which may in each case be substituted by one or more radicals $R^{32}$; where $R^{32}$ is as defined in claim 6.

**14.** Compound according to claim 11 or 12, **characterized in that** it is selected from compounds of formulae (2-1-a') to (4-5-a'),

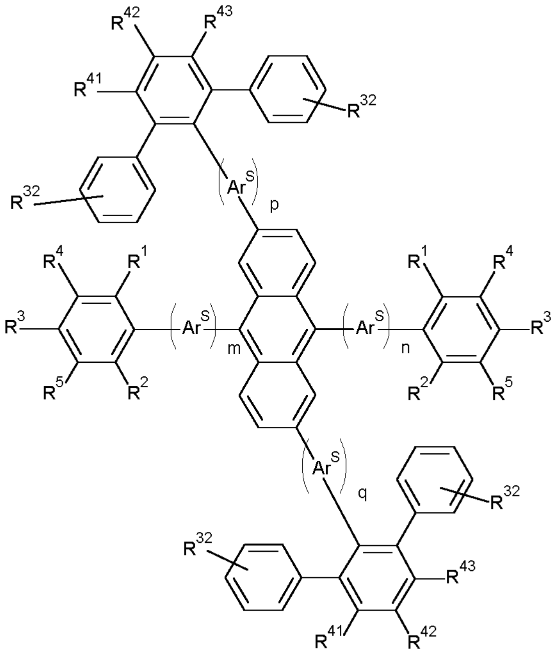

formula (2-1-a´)

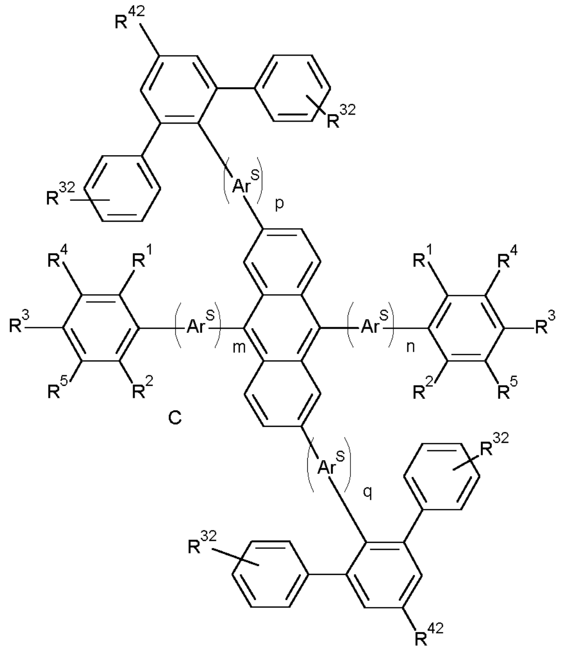

formula (2-2-a´)

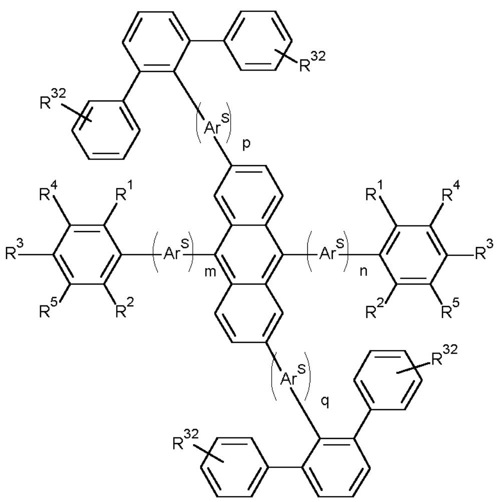

formula (2-3-a´)

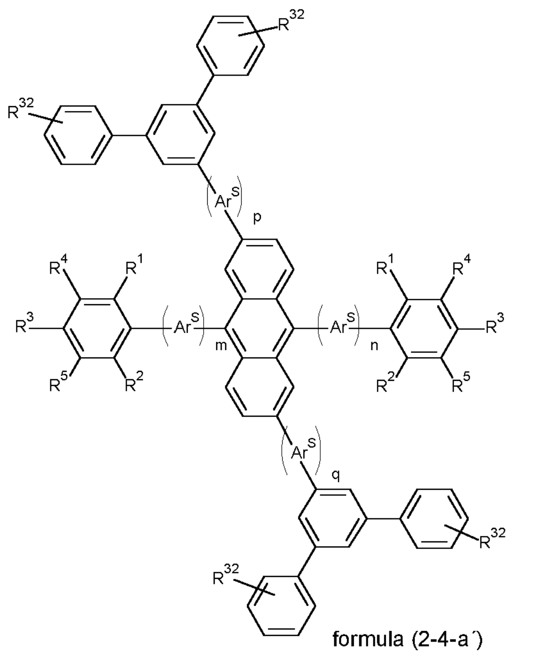

formula (2-4-a´)

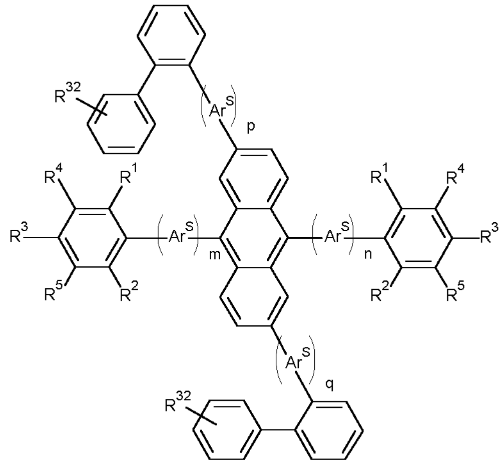

formula (2-5-a´)

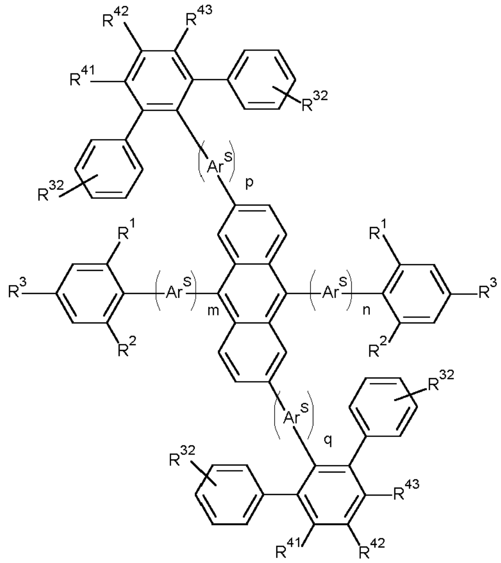

formula (3-1-a´)

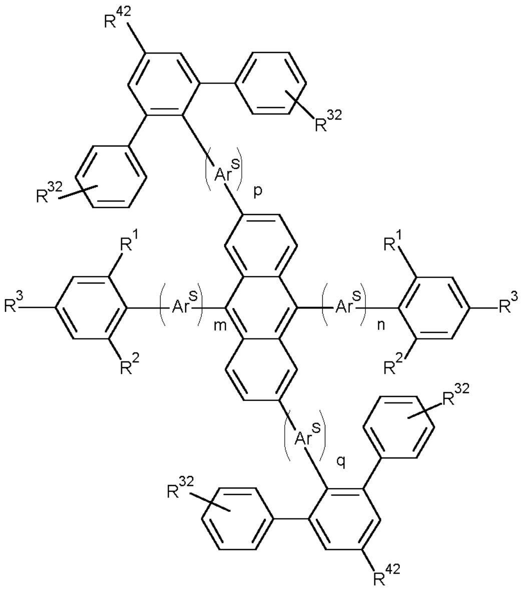

formula (3-2-a´)

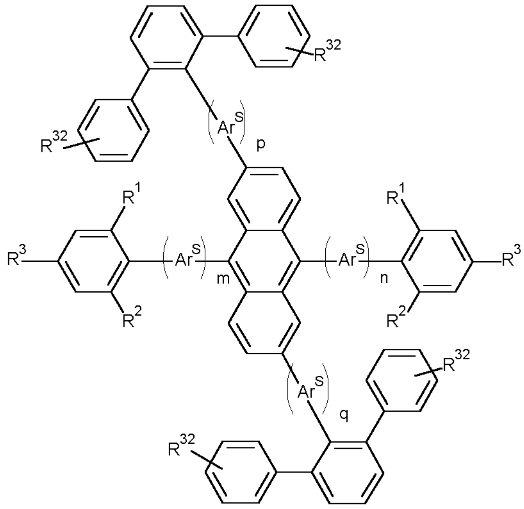

formula (3-3-a´)

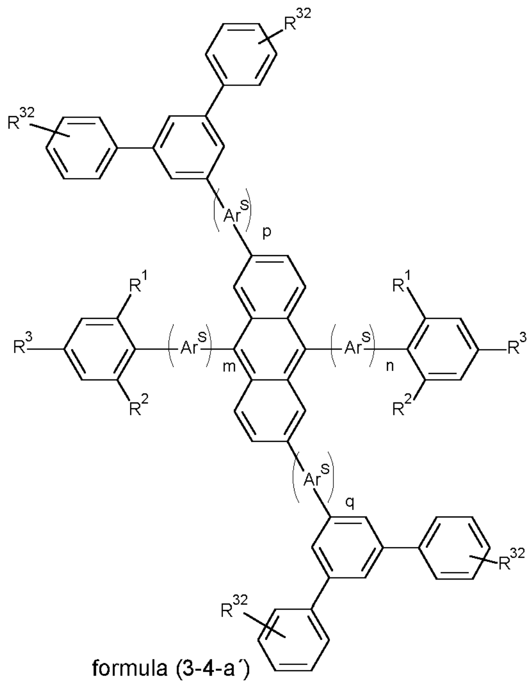

formula (3-4-a´)

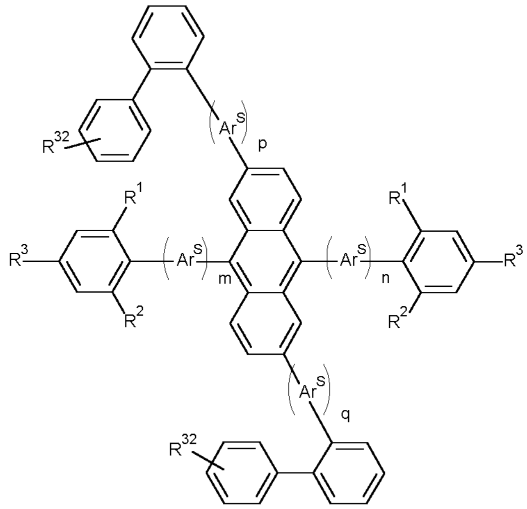

formula (3-5-a´)

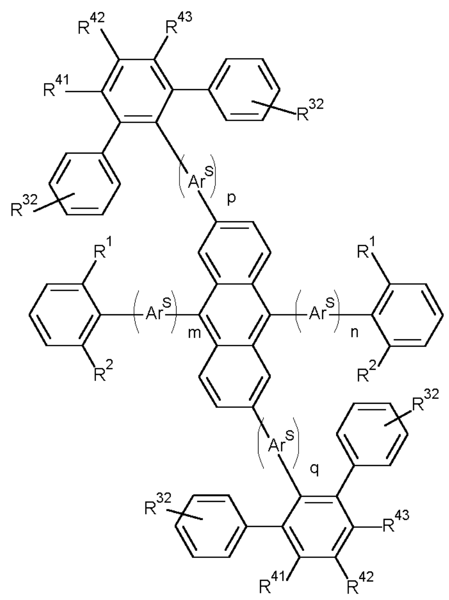

formula (4-1-a´)

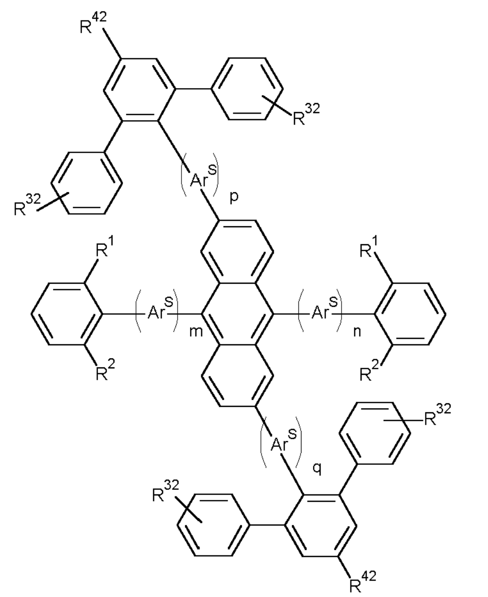

formula (4-2-a´)

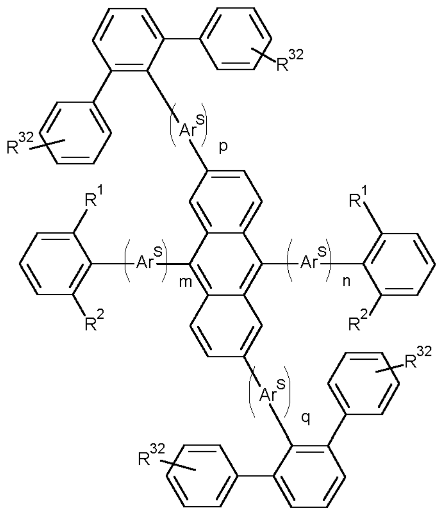

formula (4-3-a´)

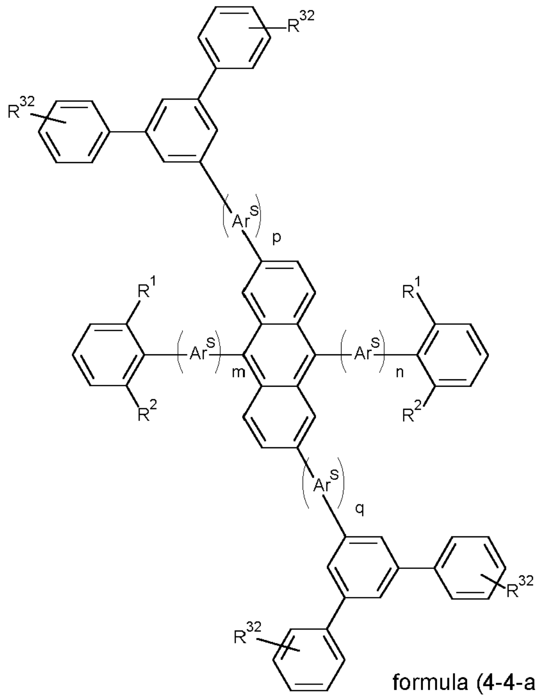

formula (4-4-a´)

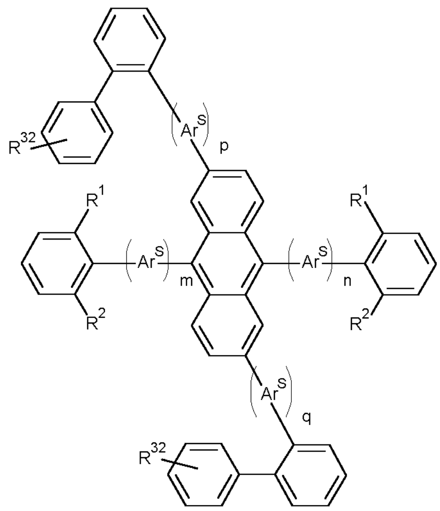

formula (4-5-a´)

where the symbols and indices have the same meaning as in claim 11.

**15.** Compound according to one or more of claims 1 to 10, **characterized in that** it is selected from compounds of formulae (2-b) to (4-b),

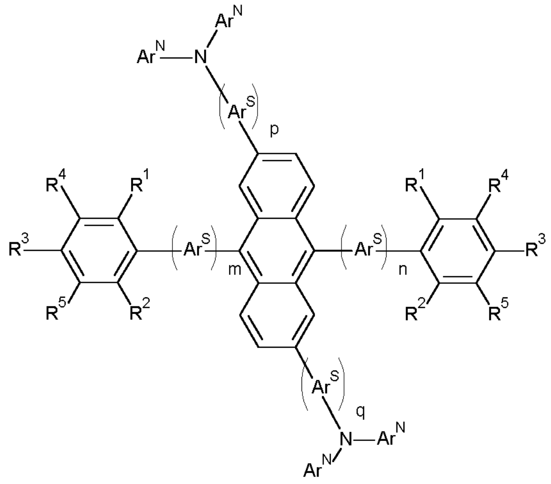

formula (2-b)

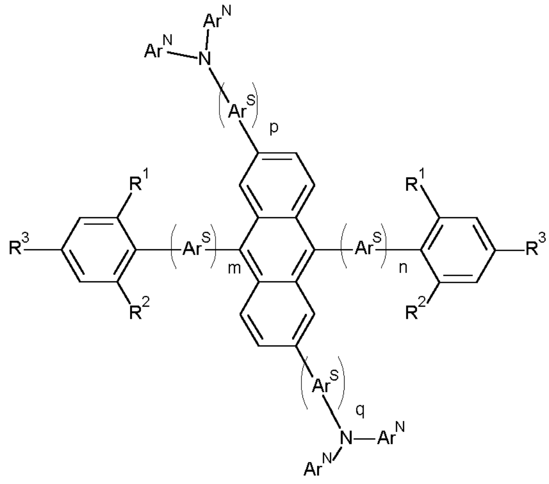

formula (3-b)

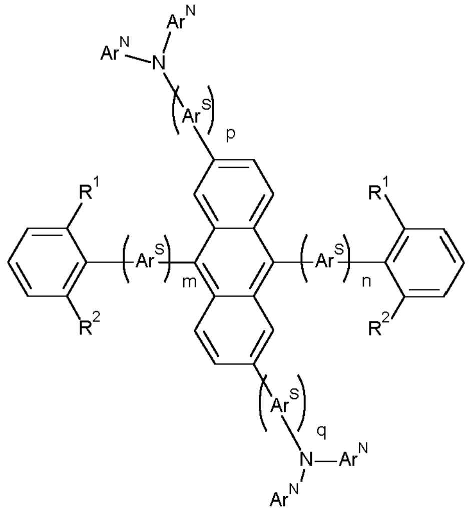

formula (4-b)

where the symbols and indices have the same meaning as in claim1.

**16.** Polymer, oligomer or dendrimer containing one or more compounds according to one or more of the claims 1, where the bond(s) to the polymer, oligomer or dendrimer may be localised at any positions in formula (1) which is substituted by $R^1$ to $R^5$ or $R^A$.

**17.** Formulation comprising at least one compound according to one or more of the claims 1 to 15 or at least one polymer, oligomer or dendrimer according to claim 16 and at least one solvent.

**18.** Organic electroluminescent device comprising an anode, a cathode and at least one organic layer comprising at least one compound according to one or more of claims 1 to 15 or at least one polymer, oligomer or dendrimer according to claim 16, **characterised in that** the compound according to one or more of claims 1 to 15 or the polymer, oligomer or dendrimer according to claim 16 is employed as an emitter in an emitting layer.

**19.** Organic electroluminescent device according to claim 18, **characterized in that** the compound according to one or more of claims 1 to 15 or the polymer, oligomer or dendrimer according to claim 16 is employed as a fluorescent emitter in an emitting layer, wherein the emitting layer comprises at least one further component selected from matrix materials.

**20.** Organic electroluminescent device according to claim 18 or 19, **characterized in that** the compound according to one or more of claims 1 to 15 or the polymer, oligomer or dendrimer according to claim 16 is employed as a fluorescent

emitter in an emitting layer, wherein the emitting layer comprises at least one sensitizer compound selected from compounds that exhibit delayed fluorescence and from phosphorescent compounds.

**21.** Organic electroluminescent device according to claim 20, **characterized in that** the emitting layer further comprises at least one organic functional material selected from matrix materials.

**Patentansprüche**

**1.** Verbindung der Formel (1),

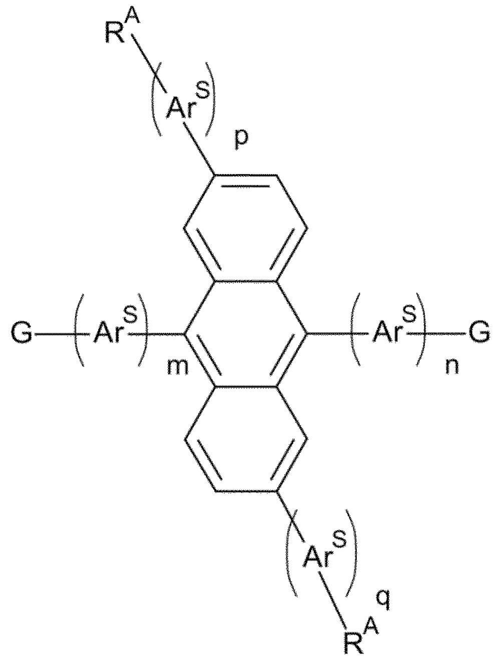

Formel (1)

wobei für die verwendeten Symbole und Indizes Folgendes gilt:

G ist eine Gruppe der Formel (G-1),

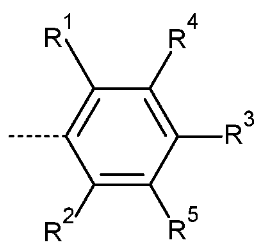

(G-1)

wobei die gestrichelte Bindung die Anbindung an $Ar^S$ oder, falls $Ar^S$ fehlt, an das zentrale Benzol des Anthracens gemäß der Darstellung in Formel (1) anzeigt;
$R^1$, $R^2$ stehen bei jedem Auftreten gleich oder verschieden für ein aromatisches oder heteroaromatisches Ringsystem mit 5 bis 24 aromatischen Ringatomen, das jeweils auch durch einen oder mehrere Reste R substituiert sein kann;
$R^3$, $R^4$, $R^5$ stehen bei jedem Auftreten gleich oder verschieden für H, D, F, Cl, Br, I, CHO, CN, $N(Ar)_2$, C(=O)Ar,

$P(=O)(Ar)_2$, $S(=O)Ar$, $S(=O)_2Ar$, $NO_2$, $Si(R)_3$, $B(OR)_2$, $OSO_2R$, eine geradkettige Alkyl-, Alkoxy- oder Thioalkylgruppe mit 1 bis 40 C-Atomen oder eine verzweigte oder cyclische Alkyl-, Alkoxy- oder Thioalkylgruppe mit 3 bis 40 C-Atomen, wobei jede dieser Gruppen durch einen oder mehrere Reste R substituiert sein kann, wobei jeweils eine oder mehrere nicht benachbarte $CH_2$-Gruppen durch RC=CR, C=C, $Si(R)_2$, $Ge(R)_2$, $Sn(R)_2$, C=O, C=S, C=Se, P(=O)(R), SO, $SO_2$, O, S oder CONR ersetzt sein können und wobei ein oder mehrere H-Atome durch D, F, Cl, Br, I, CN oder $NO_2$ ersetzt sein können, ein aromatisches oder heteroaromatisches Ringsystem mit 5 bis 60 aromatischen Ringatomen, das jeweils durch einen oder mehrere Reste R substituiert sein kann, eine Aryloxygruppe mit 5 bis 60 aromatischen Ringatomen, die durch einen oder mehrere Reste R substituiert sein kann, oder eine Aralkyl- oder Heteroaralkylgruppe mit 5 bis 60 aromatischen Ringatomen, die durch einen oder mehrere Reste R substituiert sein kann; wobei zwei benachbarte Reste, die aus $R^3$, $R^4$, $R^5$ ausgewählt sind, ein mono- oder polycyclisches, aliphatisches Ringsystem oder aromatisches Ringsystem, das durch einen oder mehrere Reste R substituiert sein kann, bilden können;

$R^A$ steht bei jedem Auftreten gleich oder verschieden für F, CN, $N(Ar^N)_2$, $C(=O)Ar$, $P(=O)(Ar)_2$, $S(=O)Ar$, $S(=O)_2Ar$, $Si(R)_3$, $B(OR)_2$, $OSO_2R$, eine geradkettige Alkyl-, Alkoxy- oder Thioalkylgruppe mit 1 bis 40 C-Atomen oder eine verzweigte oder cyclische Alkyl-, Alkoxy- oder Thioalkylgruppe mit 3 bis 40 C-Atomen, wobei jede dieser Gruppen durch einen oder mehrere Reste R substituiert sein kann, wobei jeweils eine oder mehrere nicht benachbarte $CH_2$-Gruppen durch RC=CR, C=C, $Si(R)_2$, $Ge(R)_2$, $Sn(R)_2$, C=O, C=S, C=Se, P(=O)(R), SO, $SO_2$, O, S oder CONR ersetzt sein können und wobei ein oder mehrere H-Atome durch D, F, Cl, Br, I, CN oder $NO_2$ ersetzt sein können, ein aromatisches oder heteroaromatisches Ringsystem mit 5 bis 60 aromatischen Ringatomen, das jeweils durch einen oder mehrere Reste R substituiert sein kann, eine Aryloxygruppe mit 5 bis 60 aromatischen Ringatomen, die durch einen oder mehrere Reste R substituiert sein kann, oder eine Aralkyl- oder Heteroaralkylgruppe mit 5 bis 60 aromatischen Ringatomen, die durch einen oder mehrere Reste R substituiert sein kann;

$Ar^N$ steht bei jedem Auftreten gleich oder verschieden für ein aromatisches oder heteroaromatisches Ringsystem mit 5 bis 60 aromatischen Ringatomen, das jeweils auch durch einen oder mehrere Reste R substituiert sein kann, und wobei zwei Gruppen $Ar^N$ über eine Einfachbindung oder eine aus N(R), O, S, $C(R)_2$, $C(R)_2$-$C(R)_2$, $Si(R)_2$ oder B(R) ausgewählte zweiwertige Brücke miteinander verbunden sein können;

$Ar^S$ steht bei jedem Auftreten gleich oder verschieden für eine Einfachbindung oder für ein aromatisches oder heteroaromatisches Ringsystem mit 5 bis 40 aromatischen Ringatomen, das jeweils auch durch einen oder mehrere Reste R substituiert sein kann;

R steht bei jedem Auftreten gleich oder verschieden für H, D, F, Cl, Br, I, CHO, CN, $N(Ar)_2$, $C(=O)Ar$, $P(=O)(Ar)_2$, $S(=O)Ar$, $S(=O)_2Ar$, $NO_2$, $Si(R')_3$, $B(OR')_2$, $OSO_2R'$, eine geradkettige Alkyl-, Alkoxy- oder Thioalkylgruppe mit 1 bis 40 C-Atomen oder eine verzweigte oder cyclische Alkyl-, Alkoxy- oder Thioalkylgruppe mit 3 bis 40 C-Atomen, wobei jede dieser Gruppen durch einen oder mehrere Reste R' substituiert sein kann, wobei jeweils eine oder mehrere nicht benachbarte $CH_2$-Gruppen durch R'C=CR', C=C, $Si(R')_2$, $Ge(R')_2$, $Sn(R')_2$, C=O, C=S, C=Se, P(=O)(R'), SO, $SO_2$, O, S oder CONR' ersetzt sein können und wobei ein oder mehrere H-Atome durch D, F, Cl, Br, I, CN oder $NO_2$ ersetzt sein können, ein aromatisches oder heteroaromatisches Ringsystem mit 5 bis 60 aromatischen Ringatomen, das jeweils durch einen oder mehrere Reste R' substituiert sein kann, oder eine Aryloxygruppe mit 5 bis 60 aromatischen Ringatomen, die durch einen oder mehrere Reste R' substituiert sein kann, wobei zwei benachbarte Reste R ein mono- oder polycyclisches, aliphatisches Ringsystem oder aromatisches Ringsystem, das durch einen oder mehrere Reste R' substituiert sein kann, bilden können;

Ar ist bei jedem Auftreten gleich oder verschieden ein aromatisches oder heteroaromatisches Ringsystem mit 5 bis 24 aromatischen Ringatomen, das jeweils auch durch einen oder mehrere Reste R' substituiert sein kann;

R' steht bei jedem Auftreten gleich oder verschieden für H, D, F, Cl, Br, I, CN, eine geradkettige Alkyl-, Alkoxy- oder Thioalkylgruppe mit 1 bis 20 C-Atomen oder eine verzweigte oder cyclische Alkyl-, Alkoxy- oder Thioalkylgruppe mit 3 bis 20 C-Atomen, wobei jeweils eine oder mehrere nicht benachbarte $CH_2$-Gruppen durch SO, $SO_2$, O, S ersetzt sein können und wobei ein oder mehrere H-Atome durch D, F, Cl, Br oder I ersetzt sein können, oder ein aromatisches oder heteroaromatisches Ringsystem mit 5 bis 24 C-Atomen; und

n, m, p, q gleich oder verschieden für 0, 1 oder 2 stehen.

2. Verbindung nach einem oder mehreren der vorhergehenden Ansprüche, **dadurch gekennzeichnet, dass** $R^3$, $R^4$, $R^5$ bei jedem Auftreten gleich oder verschieden für eine geradkettige Alkyl- oder Alkoxygruppe mit 1 bis 10 C-Atomen oder eine verzweigte oder cyclische Alkyl- oder Alkoxygruppe mit 3 bis 40 C-Atomen, wobei jede dieser Gruppen durch einen oder mehrere Reste R substituiert sein kann, wobei jeweils eine oder mehrere nicht benachbarte $CH_2$-Gruppen durch RC=CR, C=C, $Si(R)_2$, $Ge(R)_2$, $Sn(R)_2$, C=O, C=S, C=Se, P(=0) (R), SO, $SO_2$, O, S oder CONR ersetzt sein können und wobei ein oder mehrere H-Atome durch D, F, Cl, Br, I, CN oder $NO_2$ ersetzt sein können, ein aromatisches oder heteroaromatisches Ringsystem mit 5 bis 40 aromatischen Ringatomen, das jeweils durch einen oder mehrere Reste R substituiert sein kann, eine Aryloxygruppe mit 5 bis 40 aromatischen Ringatomen, die

durch einen oder mehrere Reste R substituiert sein kann, oder eine Aralkyl- oder Heteroaralkylgruppe mit 5 bis 40 aromatischen Ringatomen, die durch einen oder mehrere Reste R substituiert sein kann; stehen.

3. Verbindung nach einem oder mehreren der vorhergehenden Ansprüche, **dadurch gekennzeichnet, dass** $R^3$, $R^4$, $R^5$ bei jedem Auftreten gleich oder verschieden für eine geradkettige Alkyl- oder Alkoxygruppe mit 1 bis 10 C-Atomen oder eine verzweigte oder cyclische Alkyl- oder Alkoxygruppe mit 3 bis 40 C-Atomen, wobei jede dieser Gruppen durch einen oder mehrere Reste R substituiert sein kann, wobei jeweils eine oder mehrere nicht benachbarte $CH_2$-Gruppen durch RC=CR, C=C, $Si(R)_2$, $Ge(R)_2$, $Sn(R)_2$, C=O, C=S, C=Se, P(=0) (R), SO, $SO_2$, O, S oder CONR ersetzt sein können und wobei ein oder mehrere H-Atome durch D, F, Cl, Br, I, CN oder $NO_2$ ersetzt sein können, ein aromatisches oder heteroaromatisches Ringsystem mit 5 bis 40 aromatischen Ringatomen, das jeweils durch einen oder mehrere Reste R substituiert sein kann, eine Aryloxygruppe mit 5 bis 40 aromatischen Ringatomen, die durch einen oder mehrere Reste R substituiert sein kann, oder eine Aralkyl- oder Heteroaralkylgruppe mit 5 bis 40 aromatischen Ringatomen, die durch einen oder mehrere Reste R substituiert sein kann; stehen.

4. Verbindung nach einem oder mehreren der vorhergehenden Ansprüche, **dadurch gekennzeichnet, dass** $R^3$, $R^4$, $R^5$ bei jedem Auftreten gleich oder verschieden für ein aromatisches oder heteroaromatisches Ringsystem mit 5 bis 24 aromatischen Ringatomen, das jeweils durch einen oder mehrere Reste R substituiert sein kann, stehen.

5. Verbindung nach einem oder mehreren der vorhergehenden Ansprüche, **dadurch gekennzeichnet, dass** die Gruppe G für eine Gruppe der Formel (G-2), (G-3) oder (G-4) steht,

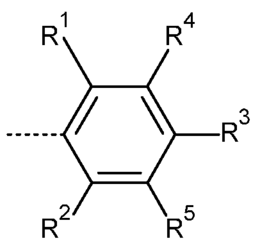
(G-2)

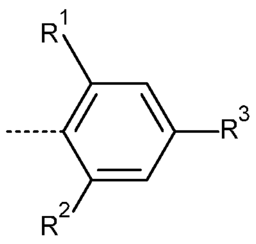
(G-3)

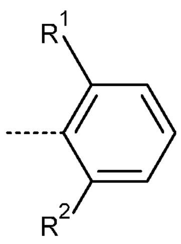
(G-4)

wobei $R^1$ und $R^2$ die gleiche Bedeutung wie in Anspruch 1 haben; und
$R^3$, $R^4$, $R^5$ bei jedem Auftreten gleich oder verschieden für eine geradkettige Alkyl-, Alkoxy- oder Thioalkylgruppe mit 1 bis 40 C-Atomen oder eine verzweigte oder cyclische Alkyl-, Alkoxy- oder Thioalkylgruppe mit 3 bis 40 C-Atomen, wobei jede dieser Gruppen durch einen oder mehrere Reste R substituiert sein kann, wobei jeweils eine oder mehrere nicht benachbarte $CH_2$-Gruppen durch RC=CR, C=C, $Si(R)_2$, $Ge(R)_2$, $Sn(R)_2$, C=O, C=S, C=Se, P(=0) (R), SO, $SO_2$, O, S oder CONR ersetzt sein können und wobei ein oder mehrere H-Atome durch D, F, Cl, Br, I, CN oder $NO_2$ ersetzt sein können, ein aromatisches oder heteroaromatisches Ringsystem mit 5 bis 60 aromatischen Ringatomen, das jeweils durch einen oder mehrere Reste R substituiert sein kann, eine Aryloxygruppe mit 5 bis 60 aromatischen Ringatomen, die durch einen oder mehrere Reste R substituiert sein kann, oder eine Aralkyl- oder Heteroaralkylgruppe mit 5 bis 60 aromatischen Ringatomen, die durch einen oder mehrere Reste R substituiert sein kann, stehen.

6. Verbindung nach einem oder mehreren der vorhergehenden Ansprüche, **dadurch gekennzeichnet, dass** $R^A$ bei jedem Auftreten gleich oder verschieden für F, CN, $N(Ar^N)_2$, C(=O)Ar, $P(=O)(Ar)_2$, S(=O)Ar, $S(=O)_2Ar$, $Si(R)_3$, $B(OR)_2$, $OSO_2R$, eine geradkettige Alkyl-, Alkoxy- oder Thioalkylgruppe mit 1 bis 40 C-Atomen oder eine verzweigte oder cyclische Alkyl-, Alkoxy- oder Thioalkylgruppe mit 3 bis 40 C-Atomen, wobei jede dieser Gruppen durch einen oder mehrere Reste R substituiert sein kann, wobei jeweils eine oder mehrere nicht benachbarte $CH_2$-Gruppen durch RC=CR, C=C, $Si(R)_2$, $Ge(R)_2$, $Sn(R)_2$, C=O, C=S, C=Se, P(=0) (R), SO, $SO_2$, O, S oder CONR ersetzt sein können und wobei ein oder mehrere H-Atome durch D, F, Cl, Br, I, CN oder $NO_2$ ersetzt sein können, ein aromatisches oder heteroaromatisches Ringsystem mit 5 bis 60 aromatischen Ringatomen, das jeweils durch einen oder mehrere Reste R substituiert sein kann, eine Aryloxygruppe mit 5 bis 60 aromatischen Ringatomen, die durch einen oder mehrere Reste R substituiert sein kann, oder eine Aralkyl- oder Heteroaralkylgruppe mit 5 bis 60 aromatischen Ringatomen, die durch einen oder mehrere Reste R substituiert sein kann, steht.

7. Verbindung nach einem oder mehreren der vorhergehenden Ansprüche, **dadurch gekennzeichnet, dass** $R^A$ bei jedem Auftreten gleich oder verschieden ausgewählt ist

- aus $N(Ar^N)_2$; wobei $Ar^N$ die gleiche Bedeutung wie in Anspruch 1 hat;
- aus verzweigten oder cyclischen Alkylgruppen, die durch die folgende allgemeine Formel (RS-a) wiedergegeben werden:

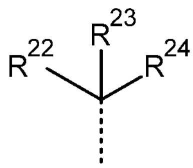

(RS-a)

wobei

$R^{22}$, $R^{23}$, $R^{24}$ bei jedem Auftreten gleich oder verschieden aus H, einer geradkettigen Alkylgruppe mit 1 bis 10 Kohlenstoffatomen oder einer verzweigten oder cyclischen Alkylgruppe mit 3 bis 10 Kohlenstoffatomen ausgewählt sind, wobei die oben aufgeführten Gruppen jeweils durch einen oder mehrere Reste $R^{25}$ substituiert sein können und wobei zwei der Reste $R^{22}$, $R^{23}$, $R^{24}$ oder alle der Reste $R^{22}$, $R^{23}$, $R^{24}$ zu einer (poly) cyclischen Alkylgruppe, die durch einen oder mehrere Reste $R^{25}$ substituiert sein kann, verknüpft sein können;

$R^{25}$ bei jedem Auftreten gleich oder verschieden aus einer geradkettigen Alkylgruppe mit 1 bis 10 Kohlenstoffatomen oder einer verzweigten oder cyclischen Alkylgruppe mit 3 bis 10 Kohlenstoffatomen ausgewählt ist;

mit der Maßgabe, dass bei jedem Auftreten mindestens einer der Reste $R^{22}$, $R^{23}$, $R^{24}$ von H verschieden ist, mit der Maßgabe, dass bei jedem Auftreten mindestens alle der Reste $R^{22}$, $R^{23}$ und $R^{24}$ zusammen mindestens 4 Kohlenstoffatome aufweisen, und mit der Maßgabe, dass bei jedem Auftreten dann, wenn zwei $R^{22}$, $R^{23}$, $R^{24}$ für H stehen, der verbleibende Rest nicht geradkettig ist;

- oder aus verzweigten oder cyclischen Alkoxygruppen, die durch die folgende allgemeine Formel (RS-b) wiedergegeben werden:

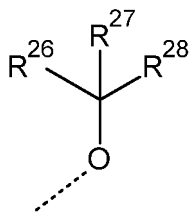

(RS-b)

wobei

$R^{26}$, $R^{27}$, $R^{28}$ bei jedem Auftreten gleich oder verschieden aus H, einer geradkettigen Alkylgruppe mit 1 bis 10 Kohlenstoffatomen oder einer verzweigten oder cyclischen Alkylgruppe mit 3 bis 10 Kohlenstoffatomen ausgewählt sind, wobei die oben aufgeführten Gruppen jeweils durch einen oder mehrere Reste $R^{25}$ gemäß obiger Definition substituiert sein können und wobei zwei der Reste $R^{26}$, $R^{27}$, $R^{28}$ oder alle der Reste $R^{26}$, $R^{27}$, $R^{28}$ zu einer (poly)cyclischen Alkylgruppe, die durch einen oder mehrere Reste $R^{25}$ gemäß obiger Definition substituiert sein kann, verknüpft sein können;

mit der Maßgabe, dass bei jedem Auftreten nur einer der Reste $R^{26}$, $R^{27}$, $R^{28}$ H sein kann;

- oder aus Aralkylgruppen, die durch die folgende allgemeine Formel (RS-c) wiedergegeben werden:

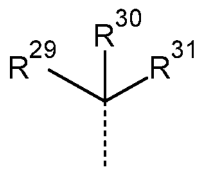

(RS-c)

wobei

$R^{29}$, $R^{30}$, $R^{31}$ bei jedem Auftreten gleich oder verschieden aus H, einer geradkettigen Alkylgruppe mit 1 bis 10 Kohlenstoffatomen oder einer verzweigten oder cyclischen Alkylgruppe mit 3 bis 10 Kohlenstoffatomen ausgewählt sind, wobei die oben aufgeführten Gruppen jeweils durch einen oder mehrere Reste $R^{32}$ substituiert sein können, oder ein aromatisches Ringsystem mit 6 bis 30 aromatischen Ringatomen, das jeweils durch einen oder mehrere Reste $R^{32}$ substituiert sein kann, ausgewählt sind und wobei zwei oder alle der Reste $R^{29}$, $R^{30}$, $R^{31}$ zu einer (poly)cyclischen Alkylgruppe oder einem aromatischen Ringsystem, das bzw. die jeweils durch einen oder mehrere Reste $R^{32}$ substituiert sein kann, verknüpft sein können;
$R^{32}$ bei jedem Auftreten gleich oder verschieden aus einer geradkettigen Alkylgruppe mit 1 bis 10 Kohlenstoffatomen oder einer verzweigten oder cyclischen Alkylgruppe mit 3 bis 10 Kohlenstoffatomen oder einem aromatischen Ringsystemen mit 6 bis 24 aromatischen Ringatomen ausgewählt ist;
mit der Maßgabe, dass bei jedem Auftreten mindestens einer der Reste $R^{29}$, $R^{30}$, $R^{31}$ von H verschieden ist und dass bei jedem Auftreten mindestens einer der Reste $R^{29}$, $R^{30}$, $R^{31}$ ein aromatisches Ringsystem mit mindestens 6 aromatischen Ringatomen ist oder enthält;

- oder aus aromatischen Ringsystemen, die durch die folgende allgemeine Formel (RS-d) wiedergegeben werden:

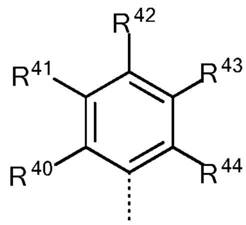

(RS-d)

wobei
$R^{40}$ bis $R^{44}$ bei jedem Auftreten gleich oder verschieden aus H, einer geradkettigen Alkylgruppe mit 1 bis 10 Kohlenstoffatomen oder einer verzweigten oder cyclischen Alkylgruppen mit 3 bis 10 Kohlenstoffatomen, wobei die oben aufgeführten Gruppen jeweils durch einen oder mehrere Reste $R^{32}$ substituiert sein können, oder einem aromatischen Ringsystem mit 6 bis 30 aromatischen Ringatomen, das jeweils durch einen oder mehrere Reste $R^{32}$ substituiert sein kann, ausgewählt sind, und wobei zwei oder mehr der Reste $R^{40}$ bis $R^{44}$ zu einer (poly)cyclischen Alkylgruppe oder einem aromatischen Ringsystem, das bzw. die jeweils durch einen oder mehrere Reste $R^{32}$ gemäß obiger Definition substituiert sein kann, verknüpft sein können.

8. Verbindung nach einem oder mehreren der vorhergehenden Ansprüche, **dadurch gekennzeichnet, dass** $Ar^S$ für Benzol, Biphenyl, Terphenyl, Naphthalin, Fluoren, Indenofluoren, Spirobifluoren, Triazin, Benzochinolin, Benzochinazolin, Dibenzofuran, Dibenzothiophen und Carbazol steht, wobei die oben aufgeführten Gruppen jeweils durch einen oder mehrere Reste R substituiert sein kann.

9. Verbindung nach einem oder mehreren der vorhergehenden Ansprüche, **dadurch gekennzeichnet, dass** m = n = 0.

10. Verbindung nach einem oder mehreren der vorhergehenden Ansprüche, **dadurch gekennzeichnet, dass** p = q = 0.

11. Verbindung nach einem oder mehreren der vorhergehenden Ansprüche, **dadurch gekennzeichnet, dass** sie aus

Verbindungen der Formel (2), (3) oder (4) ausgewählt ist:

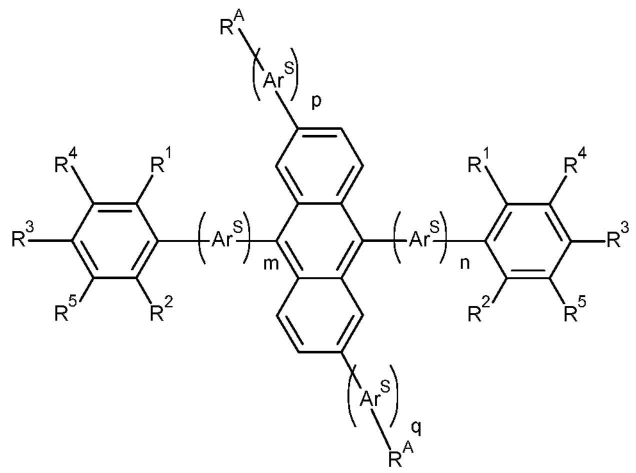

formula (2)

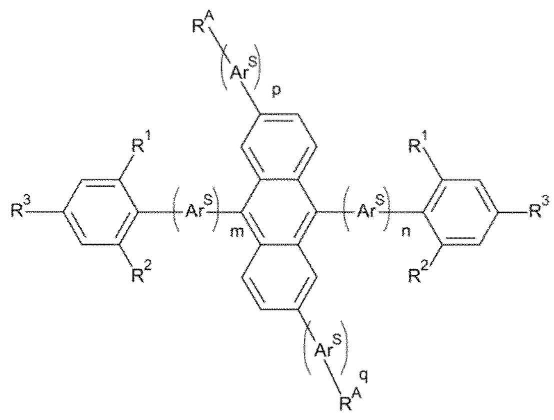

Formel (3)

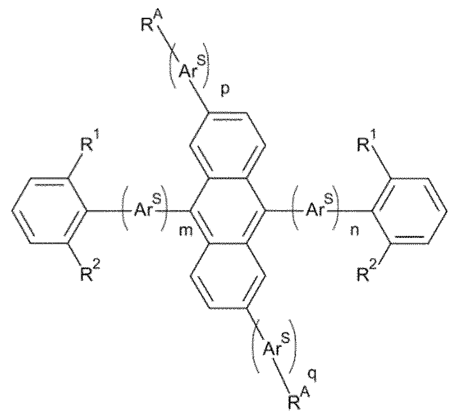

Formel (4)

wobei

$R^3$, $R^4$, $R^5$ bei jedem Auftreten gleich oder verschieden für eine geradkettige Alkyl-, Alkoxy- oder Thioalkylgruppe mit 1 bis 40 C-Atomen oder eine verzweigte oder cyclische Alkyl-, Alkoxy- oder Thioalkylgruppe mit 3 bis 40 C-Atomen, wobei jede dieser Gruppen durch einen oder mehrere Reste R substituiert sein kann, wobei jeweils eine oder mehrere nicht benachbarte $CH_2$-Gruppen durch RC=CR, C=C, $Si(R)_2$, $Ge(R)_2$, $Sn(R)_2$, C=O, C=S, C=Se, P(=0) (R), SO, $SO_2$, O, S oder CONR ersetzt sein können und wobei ein oder mehrere H-Atome durch D, F, Cl, Br, I, CN oder $NO_2$ ersetzt sein können, ein aromatisches oder heteroaromatisches Ringsystem mit 5 bis 60 aromatischen Ringatomen, das jeweils durch einen oder mehrere Reste R substituiert sein kann, eine Aryloxygruppe mit 5 bis 60 aromatischen Ringatomen, die durch einen oder mehrere Reste R substituiert sein kann, oder eine Aralkyl- oder Heteroaralkylgruppe mit 5 bis 60 aromatischen Ringatomen, die durch einen oder mehrere Reste R substituiert sein kann, stehen und
wobei die anderen Symbole und Indizes die gleiche Bedeutung wie in Anspruch 1 haben.

**12.** Verbindung nach einem oder mehreren der vorhergehenden Ansprüche, **dadurch gekennzeichnet, dass** sie aus Verbindungen der Formeln (2-1a) bis (2-4a), (4-1a) bis (4-3a) ausgewählt ist:

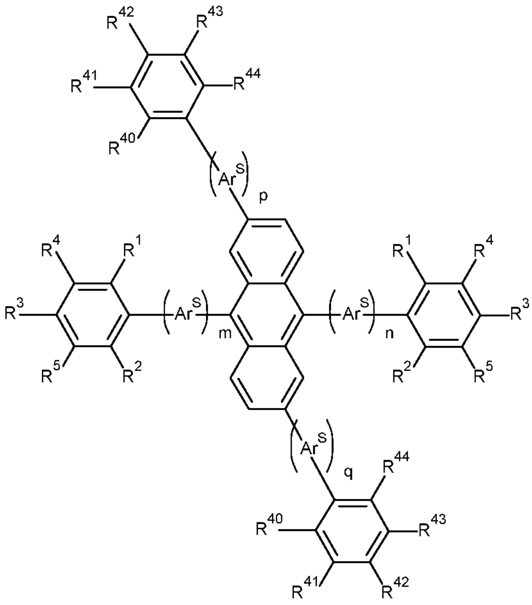

formula (2-1-a)

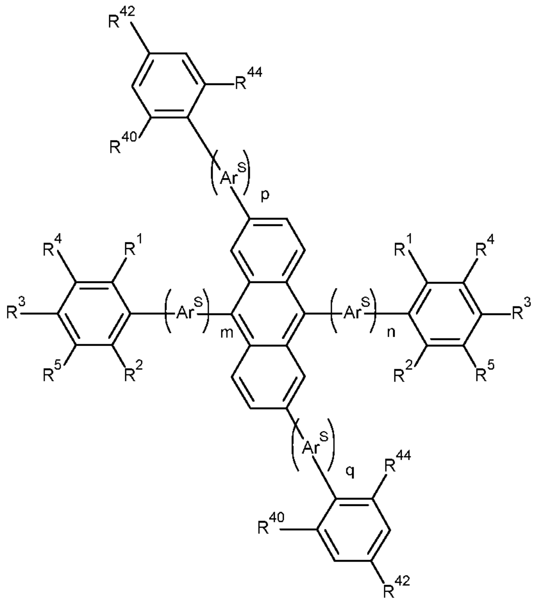

formula (2-2-a)

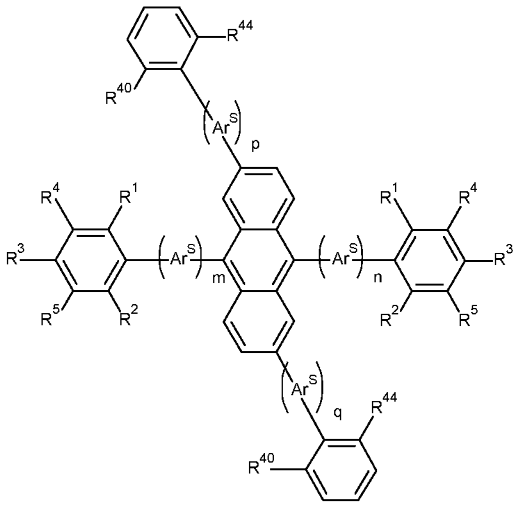

formula (2-3-a)

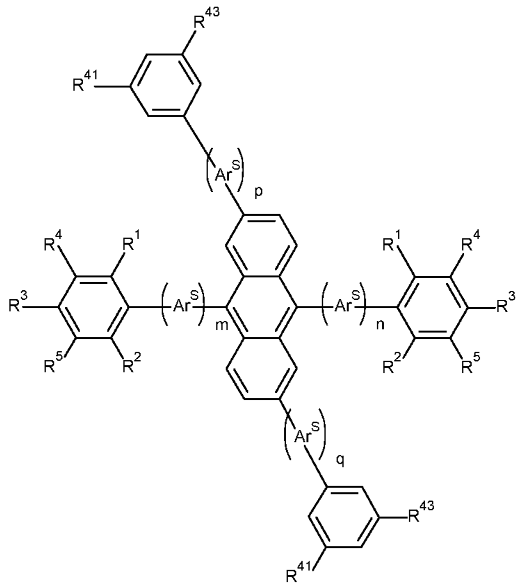

formula (2-4-a)

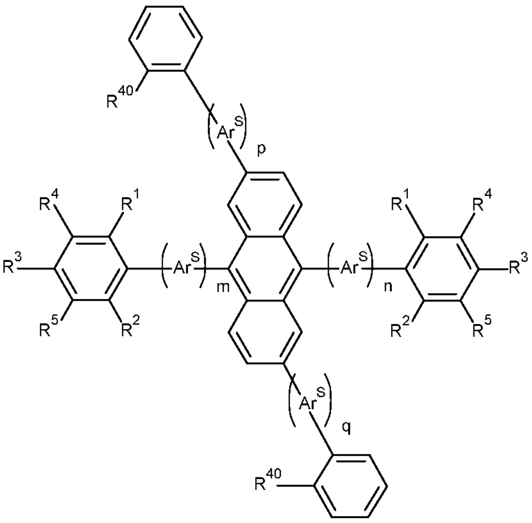

formula (2-5-a)

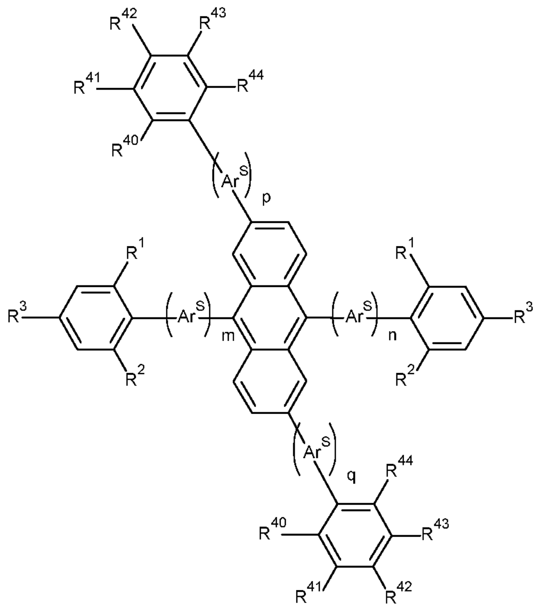

formula (3-1-a)

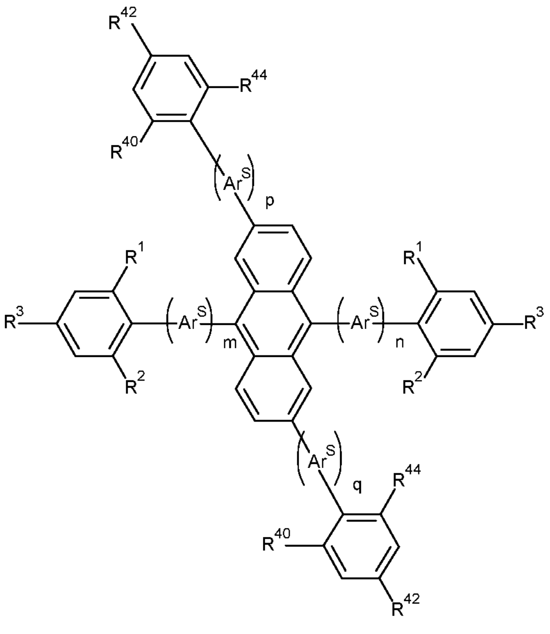

formula (3-2-a)

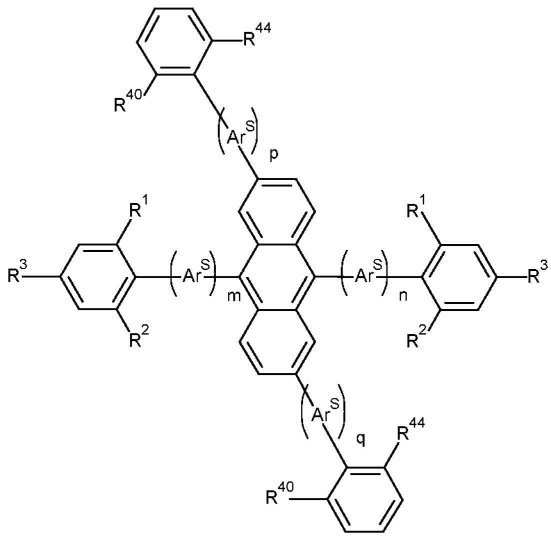

formula (3-3-a)

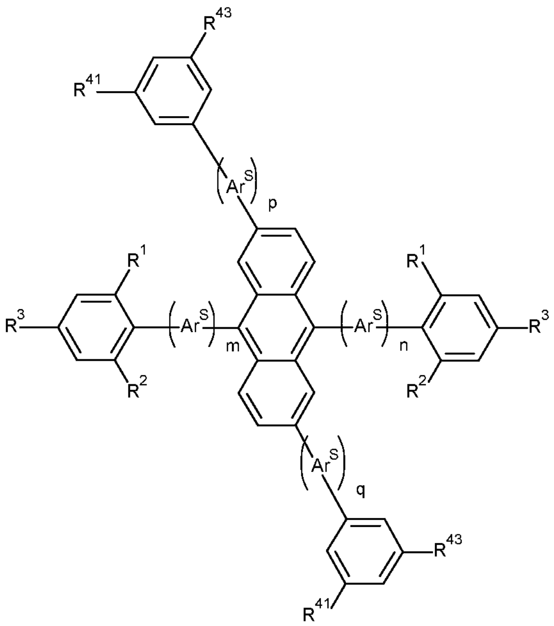

formula (3-4-a)

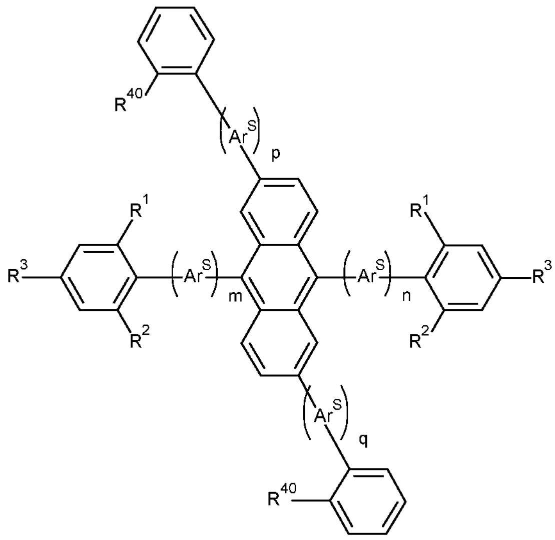

formula (3-5-a)

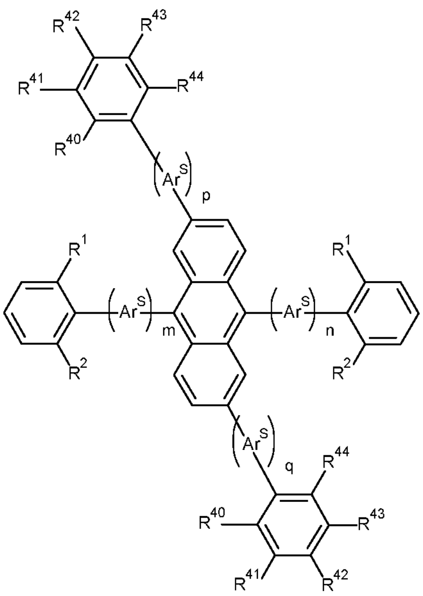

formula (4-1-a)

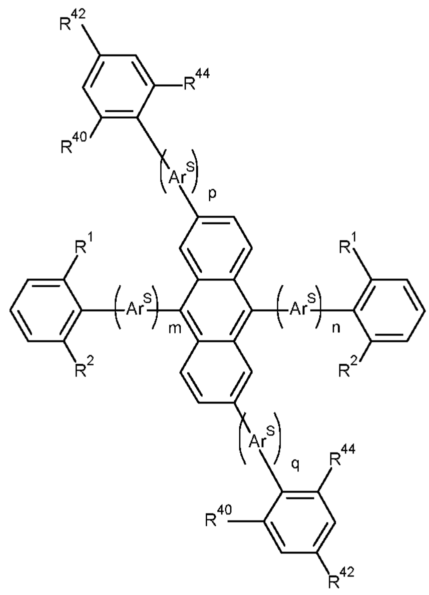

formula (4-2-a)

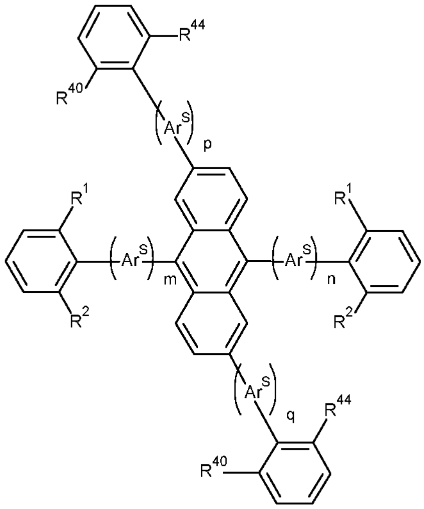

formula (4-3-a)

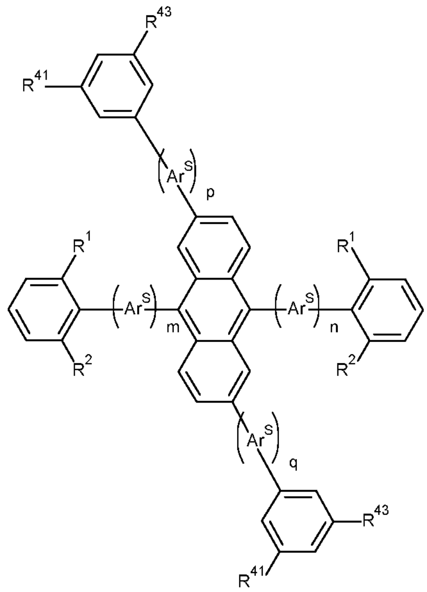

formula (4-4-a)

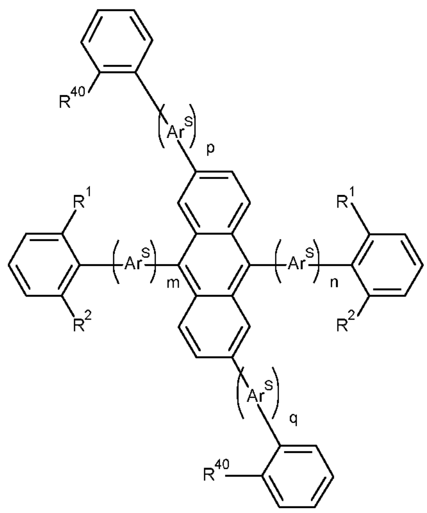

formula (4-5-a)

wobei

$R^3$, $R^4$, $R^5$ bei jedem Auftreten gleich oder verschieden für eine geradkettige Alkyl-, Alkoxy- oder Thioalkylgruppe mit 1 bis 40 C-Atomen oder eine verzweigte oder cyclische Alkyl-, Alkoxy- oder Thioalkylgruppe mit 3 bis 40 C-Atomen, wobei jede dieser Gruppen durch einen oder mehrere Reste R substituiert sein kann, wobei jeweils eine oder mehrere nicht benachbarte $CH_2$-Gruppen durch RC=CR, C=C, $Si(R)_2$, $Ge(R)_2$, $Sn(R)_2$, C=O, C=S, C=Se, P(=0) (R), SO, $SO_2$, O, S oder CONR ersetzt sein können und wobei ein oder mehrere H-Atome durch D, F, Cl, Br, I, CN oder $NO_2$ ersetzt sein können, ein aromatisches oder heteroaromatisches Ringsystem mit 5 bis 60 aromatischen Ringatomen, das jeweils durch einen oder mehrere Reste R substituiert sein kann, eine Aryloxygruppe mit 5 bis 60 aromatischen Ringatomen, die durch einen oder mehrere Reste R substituiert sein kann, oder eine Aralkyl- oder Heteroaralkylgruppe mit 5 bis 60 aromatischen Ringatomen, die durch einen oder mehrere Reste R substituiert sein kann, stehen;

$R^{40}$ bis $R^{44}$ bei jedem Auftreten gleich oder verschieden aus einer geradkettigen Alkylgruppe mit 1 bis 10 Kohlenstoffatomen oder einer verzweigten oder cyclischen Alkylgruppe mit 3 bis 10 Kohlenstoffatomen, wobei die oben aufgeführten Gruppen jeweils durch einen oder mehrere Reste $R^{32}$ substituiert sein können, oder einem aromatischen Ringsystem mit 6 bis 30 aromatischen Ringatomen, das jeweils durch einen oder mehrere Reste $R^{32}$ substituiert sein kann, ausgewählt sind; wobei $R^{32}$ wie in Anspruch 6 definiert ist;

und wobei $R^1$, $R^2$, R, m, n, p und q die gleiche Bedeutung wie in Anspruch 1 haben.

13. Verwendung nach Anspruch 11, **dadurch gekennzeichnet, dass** $R^{40}$ bis $R^{44}$ bei jedem Auftreten gleich oder verschieden aus einem aromatischen Ringsystem mit 6 bis 30 aromatischen Ringatomen, das jeweils durch einen oder mehrere Reste $R^{32}$ substituiert sein kann, ausgewählt sind; wobei $R^{32}$ wie in Anspruch 6 definiert ist.

14. Verbindung nach Anspruch 11 oder 12, **dadurch gekennzeichnet, dass** sie aus Verbindungen der Formeln (2-1-a') bis (4-5-a') ausgewählt ist:

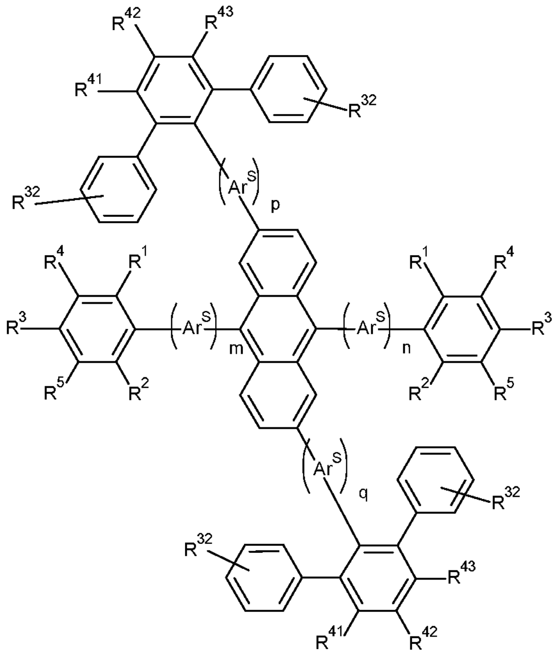

formula (2-1-a´)

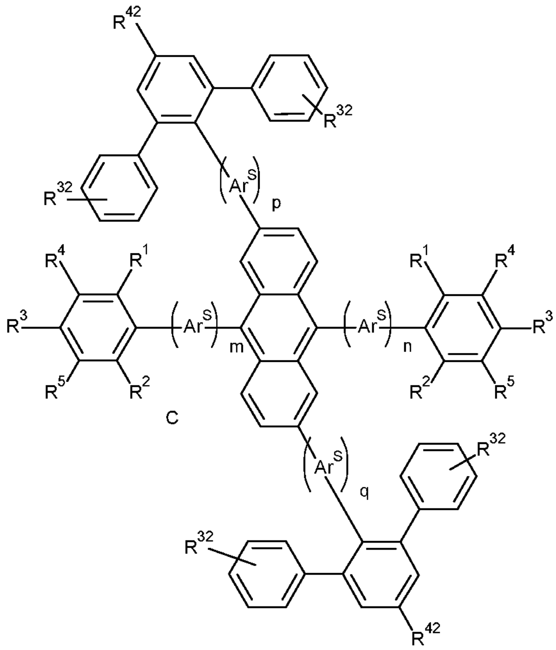

formula (2-2-a´)

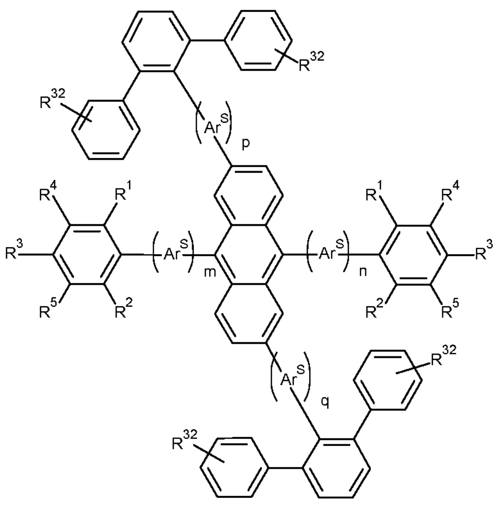

formula (2-3-a´)

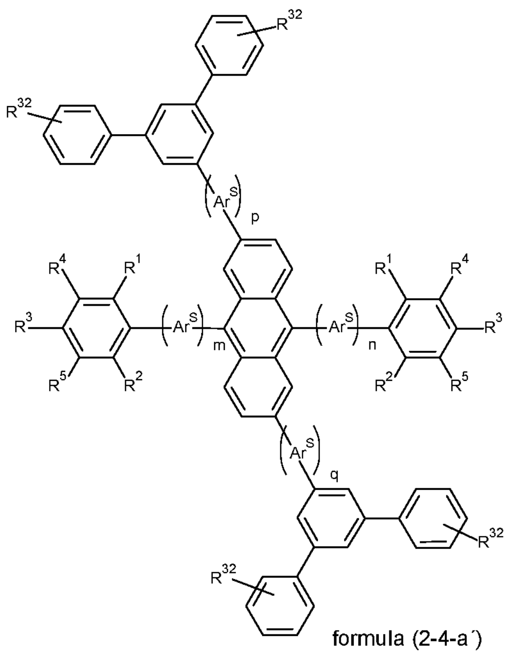

formula (2-4-a´)

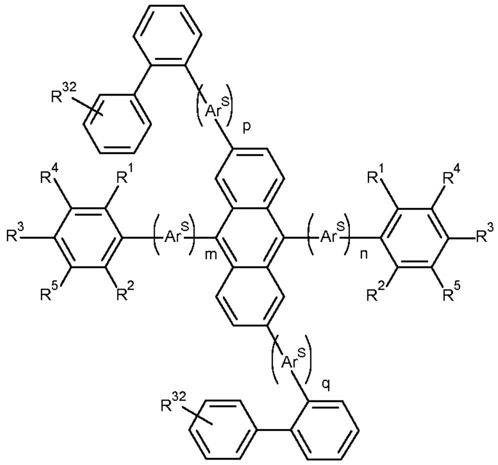

formula (2-5-a´)

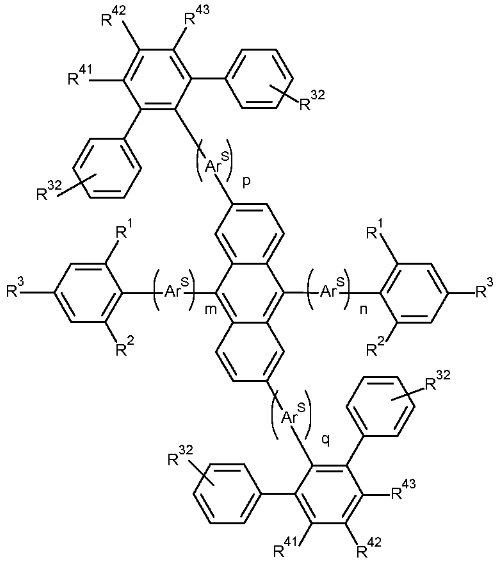

formula (3-1-a´)

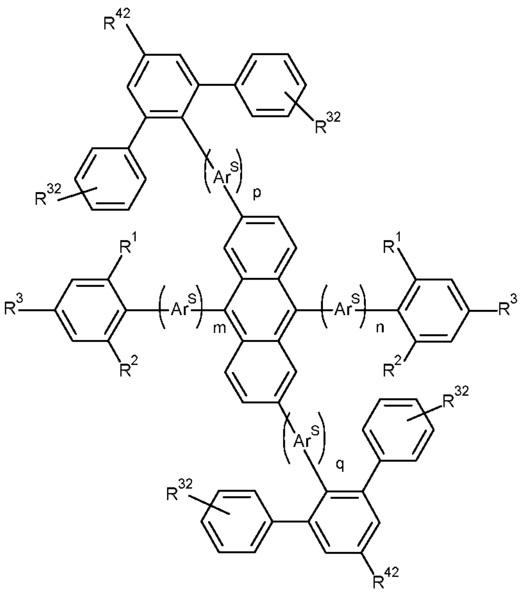

formula (3-2-a´)

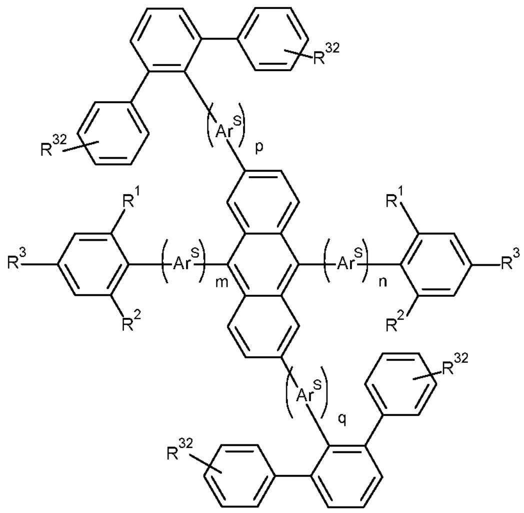

formula (3-3-a´)

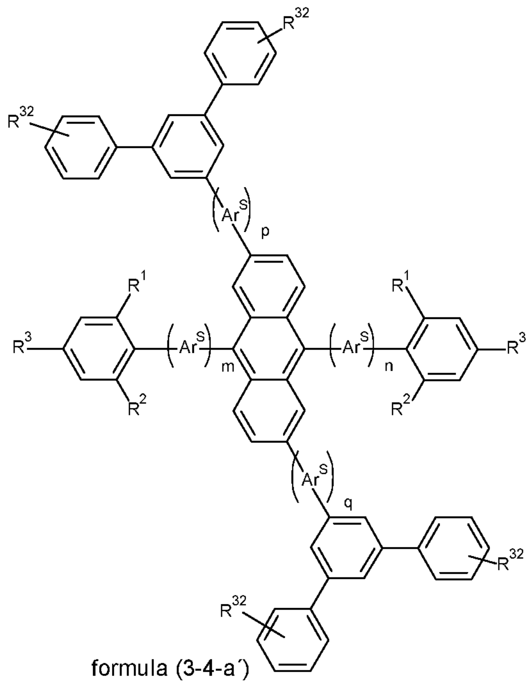

formula (3-4-a´)

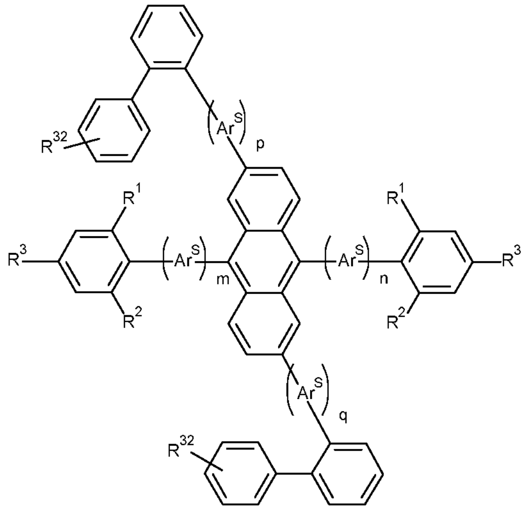

formula (3-5-a´)

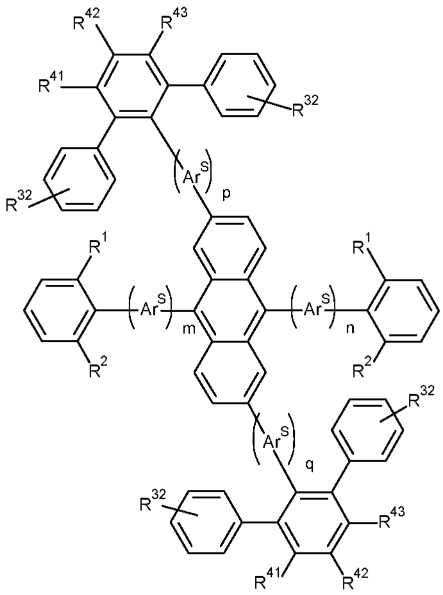

formula (4-1-a´)

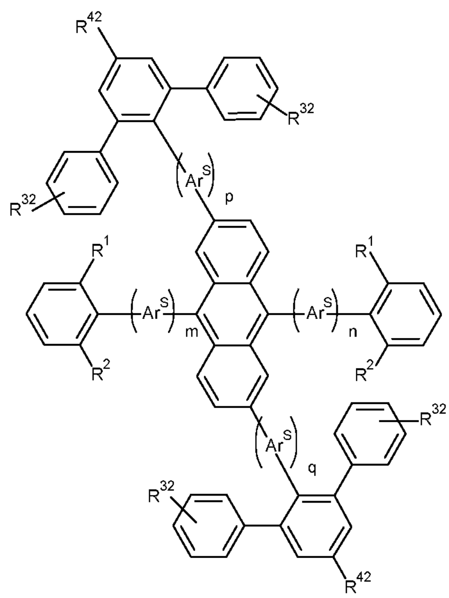

formula (4-2-a´)

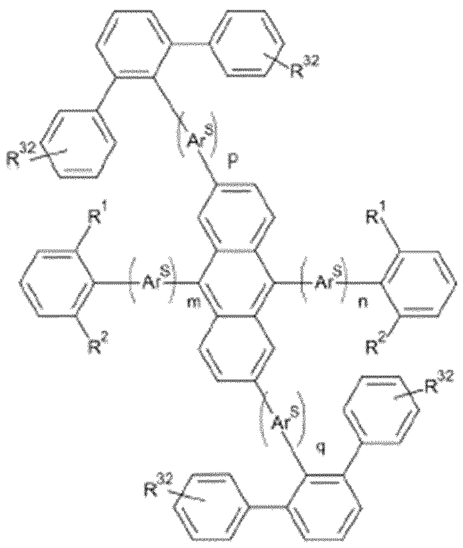

Formel (4-3-a´)

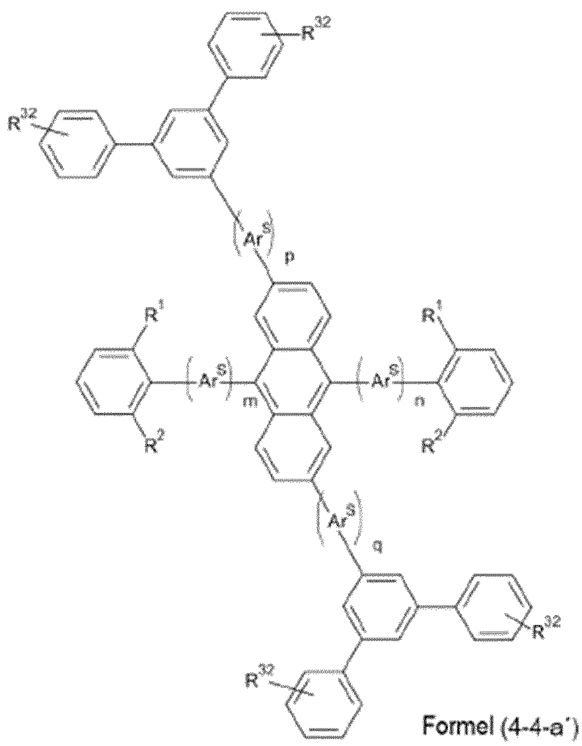

Formel (4-4-a´)

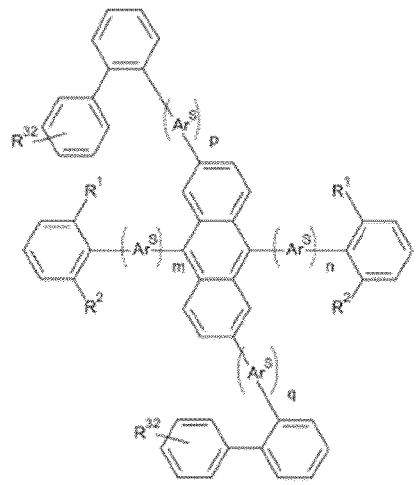

Formel (4-5-a´)

wobei die Symbole und Indizes die gleiche Bedeutung wie in Anspruch 11 haben.

15. Verbindung nach einem oder mehreren der Ansprüche 1 bis 10, **dadurch gekennzeichnet, dass** sie aus Verbindungen der Formeln (2-b) bis (4-b) ausgewählt sind:

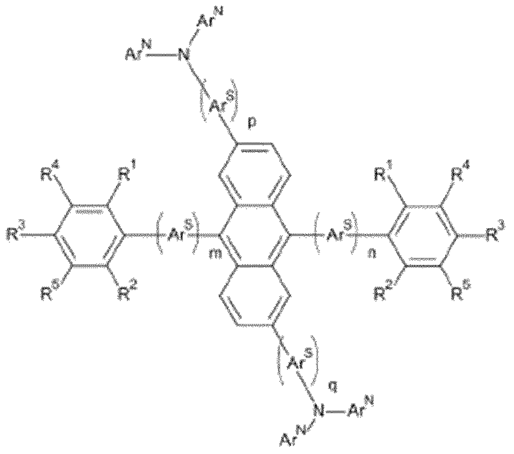

Formel (2-b)

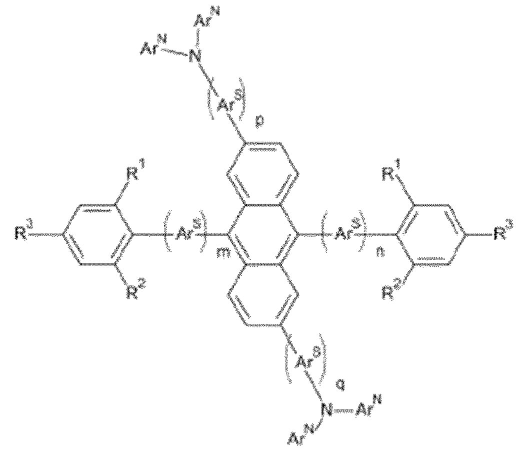

Formel (3-b)

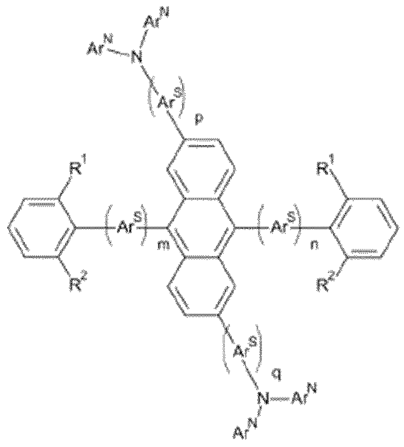

Formel (4-b)

wobei die Symbole und Indizes die gleiche Bedeutung wie in Anspruch 1 haben.

**16.** Polymer, Oligomer oder Dendrimer, enthaltend eine oder mehrere Verbindungen nach einem oder mehreren der Ansprüche 1, wobei die Bindung bzw. die Bindungen zum Polymer, Oligomer oder Dendrimer in beliebigen Positionen in Formel (1), die durch $R^1$ bis $R^5$ oder $R^A$ substituiert ist, lokalisiert sein kann bzw. können.

**17.** Formulierung, umfassend mindestens eine Verbindung nach einem oder mehreren der Ansprüche 1 bis 15 oder mindestens ein Polymer, Oligomer oder Dendrimer nach Anspruch 16 und mindestens ein Lösungsmittel.

**18.** Organische Elektrolumineszenzvorrichtung, umfassend eine Anode, eine Kathode und mindestens eine organische Schicht, die mindestens eine Verbindung nach einem der Ansprüche 1 bis 15 oder mindestens ein Polymer, Oligomer oder Dendrimer nach Anspruch 16 umfasst, **dadurch gekennzeichnet, dass** die Verbindung nach einem oder mehreren der Ansprüche 1 bis 15 oder das Polymer, Oligomer oder Dendrimer nach Anspruch 16 als Emitter in einer emittierenden Schicht eingesetzt wird.

**19.** Organische Elektrolumineszenzvorrichtung nach Anspruch 18, **dadurch gekennzeichnet, dass** die Verbindung nach einem oder mehreren der Ansprüche 1 bis 15 oder das Polymer, Oligomer oder Dendrimer nach Anspruch 16 als fluoreszierender Emitter in einer emittierenden Schicht eingesetzt wird, wobei die emittierende Schicht mindestens eine weitere Komponente, die aus Matrixmaterialien ausgewählt ist, umfasst.

**20.** Organische Elektrolumineszenzvorrichtung nach Anspruch 18 oder 19, **dadurch gekennzeichnet, dass** die Verbindung nach einem oder mehreren der Ansprüche 1 bis 15 oder das Polymer, Oligomer oder Dendrimer nach Anspruch 16 als fluoreszierender Emitter in einer emittierenden Schicht eingesetzt wird, wobei die emittierende Schicht mindestens eine Sensibilisatorverbindung, die aus Verbindungen mit verzögerter Fluoreszenz und aus

phosphoreszierenden Verbindungen ausgewählt ist, umfasst.

21. Organische Elektrolumineszenzvorrichtung nach Anspruch 20, **dadurch gekennzeichnet, dass** die emittierenden Schicht ferner mindestens ein organisches funktionelles Material, das aus Matrixmaterialien ausgewählt ist, umfasst.

## Revendications

1. Composé de formule (1),

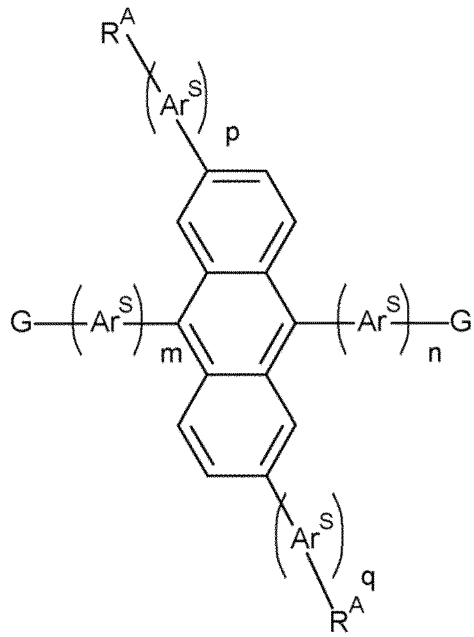

formule (1)

où ce qui suit s'applique aux symboles et indices utilisés :

G est un groupe de formule (G-1),

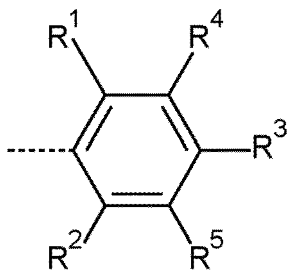

(G-1)

où la liaison pointillée indique la liaison à $Ar^S$ ou, si $Ar^S$ est absent, au benzène central de l'anthracène comme décrit dans la formule (1) ;
$R^1$, $R^2$ désignent, à chaque occurrence, identiques ou différents, un système cyclique aromatique ou hétéroaromatique ayant 5 à 24 atomes de cycle aromatique, qui peut, dans chaque cas, également être substitué par un ou plusieurs radicaux R ;
$R^3$, $R^4$, $R^5$ désignent, à chaque occurrence, identiques ou différents, H, D, F, Cl, Br, I, CHO, CN, $N(Ar)_2$, C(=O)Ar, $P(=O)(Ar)_2$, S (=O)Ar, $S(=O)_2Ar$, $NO_2$, $Si(R)_3$, $B(OR)_2$, $OSO_2R$, un groupe alkyle, alcoxy ou thioalkyle à chaîne linéaire ayant 1 à 40 atomes C ou un groupe alkyle, alcoxy ou thioalkyle ramifié ou cyclique ayant 3 à 40 atomes

C, dont chacun peut être substitué par un ou plusieurs radicaux R, où, dans chaque cas, un ou plusieurs groupes $CH_2$ non adjacents peuvent être remplacés par RC=CR, C=C, $Si(R)_2$, $Ge(R)_2$, $Sn(R)_2$, C=O, C=S, C=Se, P (=O) (R), SO, $SO_2$, O, S ou CONR et où un ou plusieurs atomes H peuvent être remplacés par D, F, Cl, Br, I, CN ou $NO_2$, un système cyclique aromatique ou hétéroaromatique ayant 5 à 60 atomes de cycle aromatique, qui peut, dans chaque cas, être substitué par un ou plusieurs radicaux R, un groupe aryloxy ayant 5 à 60 atomes de cycle aromatique, qui peut être substitué par un ou plusieurs radicaux R, ou un groupe aralkyle ou hétéroaralkyle qui a 5 à 60 atomes de cycle aromatique, qui peut être substitué par un ou plusieurs radicaux R ; où deux radicaux adjacents choisis parmi $R^3$, $R^4$, $R^5$ peuvent former un système cyclique aliphatique, mono- ou polycyclique ou un système cyclique aromatique, qui peut être substitué par un ou plusieurs radicaux R ;

$R^A$ désigne, à chaque occurrence, identique ou différent, F, CN, $N(ArN)_2$, C(=O)Ar, $P(=O) (Ar)_2$, S(=O)Ar, $S(=O)_2Ar$, $Si(R)_3$, $B(OR)_2$, $OSO_2R$, un groupe alkyle, alcoxy ou thioalkyle à chaîne linéaire ayant 1 à 40 atomes C ou un groupe alkyle, alcoxy ou thioalkyle ramifié ou cyclique ayant 3 à 40 atomes C, dont chacun peut être substitué par un ou plusieurs radicaux R, où, dans chaque cas, un ou plusieurs groupes $CH_2$ non adjacents peuvent être remplacés par RC=CR, C=C, $Si(R)_2$, $Ge(R)_2$, $Sn(R)_2$, C=O, C=S, C=Se, P (=O) (R), SO, $SO_2$, O, S ou CONR et où un ou plusieurs atomes H peuvent être remplacés par D, F, Cl, Br, I, CN ou $NO_2$, un système cyclique aromatique ou hétéroaromatique ayant 5 à 60 atomes de cycle aromatique, qui peut, dans chaque cas, être substitué par un ou plusieurs radicaux R, un groupe aryloxy ayant 5 à 60 atomes de cycle aromatique, qui peut être substitué par un ou plusieurs radicaux R, ou un groupe aralkyle ou hétéroaralkyle qui a 5 à 60 atomes de cycle aromatique, qui peut être substitué par un ou plusieurs radicaux R ;

$Ar^N$ désigne, à chaque occurrence, identique ou différent, un système cyclique aromatique ou hétéroaromatique ayant 5 à 60 atomes de cycle aromatique, qui peut, dans chaque cas, également être substitué par un ou plusieurs radicaux R, et dans lequel deux groupes $Ar^N$ peuvent être reliés l'un à l'autre par une simple liaison ou par un pont divalent choisi parmi N(R), O, S, $C(R)_2$, $C(R)_2$-$C(R)_2$, $Si(R)_2$ ou B(R) ;

$Ar^S$ désigne, à chaque occurrence, identique ou différent, une simple liaison ou un système cyclique aromatique ou hétéroaromatique ayant 5 à 40 atomes de cycle aromatique, qui peut, dans chaque cas, également être substitué par un ou plusieurs radicaux R ;

R désigne, à chaque occurrence, identique ou différent, H, D, F, Cl, Br, I, CHO, CN, $N(Ar)_2$, C(=O)Ar, $P(=O)(Ar)_2$, S (=O)Ar, $S(=O)_2Ar$, $NO_2$, $Si(R')_3$, $B(OR')_2$, $OSO_2R'$, un groupe alkyle, alcoxy ou thioalkyle à chaîne linéaire ayant 1 à 40 atomes C ou un groupe alkyle, alcoxy ou thioalkyle ramifié ou cyclique ayant 3 à 40 atomes C, dont chacun peut être substitué par un ou plusieurs radicaux R', où, dans chaque cas, un ou plusieurs groupes $CH_2$ non adjacents peuvent être remplacés par R'C=CR', C=C, $Si(R')_2$, $Ge(R')_2$, $Sn(R')_2$, C=O, C=S, C=Se, P(=O)(R'), SO, $SO_2$, O, S ou CONR' et où un ou plusieurs atomes H peuvent être remplacés par D, F, Cl, Br, I, CN ou $NO_2$, un système cyclique aromatique ou hétéroaromatique ayant 5 à 60 atomes de cycle aromatique, qui peut, dans chaque cas, être substitué par un ou plusieurs radicaux R', ou un groupe aryloxy ayant 5 à 60 atomes de cycle aromatique, qui peut être substitué par un ou plusieurs radicaux R', où deux radicaux R adjacents peuvent former un système cyclique aliphatique, mono- ou polycyclique ou un système cyclique aromatique, qui peut être substitué par un ou plusieurs radicaux R' ;

Ar est, à chaque occurrence, identique ou différent, un système cyclique aromatique ou hétéroaromatique ayant 5 à 24 atomes de cycle aromatique, qui peut, dans chaque cas, également être substitué par un ou plusieurs radicaux R' ;

R' désigne, à chaque occurrence, identique ou différent, H, D, F, Cl, Br, I, CN, un groupe alkyle, alcoxy ou thioalkyle à chaîne linéaire ayant 1 à 20 atomes C ou un groupe alkyle, alcoxy ou thioalkyle ramifié ou cyclique ayant 3 à 20 atomes C, où, dans chaque cas, un ou plusieurs groupes $CH_2$ non adjacents peuvent être remplacés par SO, $SO_2$, O, S et où un ou plusieurs atomes H peuvent être remplacés par D, F, Cl, Br ou I, ou un système cyclique aromatique ou hétéroaromatique ayant 5 à 24 atomes C ; et

n, m, p, q sont, identiques ou différents, 0, 1 ou 2.

2. Composé selon une ou plusieurs des revendications précédentes, **caractérisé en ce que** $R^3$, $R^4$, $R^5$ désignent, à chaque occurrence, identiques ou différents, un groupe alkyle ou alcoxy à chaîne linéaire ayant 1 à 10 atomes C ou un groupe alkyle ou alcoxy ramifié ou cyclique ayant 3 à 40 atomes C, dont chacun peut être substitué par un ou plusieurs radicaux R, où, dans chaque cas, un ou plusieurs groupes $CH_2$ non adjacents peuvent être remplacés par RC=CR, C=C, $Si(R)_2$, $Ge(R)_2$, $Sn(R)_2$, C=O, C=S, C=Se, P (=O) (R), SO, $SO_2$, O, S ou CONR et où un ou plusieurs atomes H peuvent être remplacés par D, F, Cl, Br, I, CN ou $NO_2$, un système cyclique aromatique ou hétéroaromatique ayant 5 à 40 atomes de cycle aromatique, qui peut, dans chaque cas, être substitué par un ou plusieurs radicaux R, un groupe aryloxy ayant 5 à 40 atomes de cycle aromatique, qui peut être substitué par un ou plusieurs radicaux R, ou un groupe aralkyle ou hétéroaralkyle qui a 5 à 40 atomes de cycle aromatique, qui peut être substitué par un ou plusieurs radicaux R.

**3.** Composé selon une ou plusieurs des revendications précédentes, **caractérisé en ce que** $R^3$, $R^4$, $R^5$ désignent, à chaque occurrence, identiques ou différents, un groupe alkyle ou alcoxy à chaîne linéaire ayant 1 à 10 atomes C ou un groupe alkyle ou alcoxy ramifié ou cyclique ayant 3 à 40 atomes C, dont chacun peut être substitué par un ou plusieurs radicaux R, où, dans chaque cas, un ou plusieurs groupes $CH_2$ non adjacents peuvent être remplacés par RC=CR, C≡C, $Si(R)_2$, $Ge(R)_2$, $Sn(R)_2$, C=O, C=S, C=Se, P (=O) (R), SO, $SO_2$, O, S ou CONR et où un ou plusieurs atomes H peuvent être remplacés par D, F, Cl, Br, I, CN ou $NO_2$, un système cyclique aromatique ou hétéroaromatique ayant 5 à 40 atomes de cycle aromatique, qui peut, dans chaque cas, être substitué par un ou plusieurs radicaux R, un groupe aryloxy ayant 5 à 40 atomes de cycle aromatique, qui peut être substitué par un ou plusieurs radicaux R, ou un groupe aralkyle ou hétéroaralkyle qui a 5 à 40 atomes de cycle aromatique, qui peut être substitué par un ou plusieurs radicaux R.

**4.** Composé selon une ou plusieurs des revendications précédentes, **caractérisé en ce que** $R^3$, $R^4$, $R^5$ désignent, à chaque occurrence, identiques ou différents, un système cyclique aromatique ou hétéroaromatique ayant 5 à 24 atomes de cycle aromatique, qui peut, dans chaque cas, également être substitué par un ou plusieurs radicaux R.

**5.** Composé selon une ou plusieurs des revendications précédentes, **caractérisé en ce que** le groupe G désigne un groupe de formule (G-2), (G-3) ou (G-4),

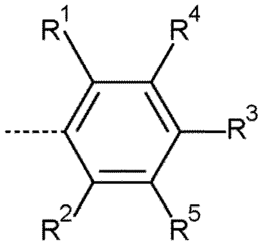
(G-2)

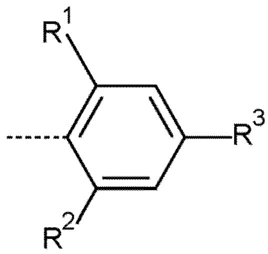
(G-3)

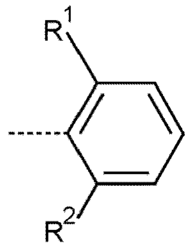
(G-4)

où $R^1$ et $R^2$ ont la même définition que dans la revendication 1 ; et
$R^3$, $R^4$, $R^5$ désignent, à chaque occurrence, identiques ou différents, un groupe alkyle, alcoxy ou thioalkyle à chaîne linéaire ayant 1 à 40 atomes C ou un groupe alkyle, alcoxy ou thioalkyle ramifié ou cyclique ayant 3 à 40 atomes C, dont chacun peut être substitué par un ou plusieurs radicaux R, où, dans chaque cas, un ou plusieurs groupes $CH_2$ non adjacents peuvent être remplacés par RC=CR, C≡C, $Si(R)_2$, $Ge(R)_2$, $Sn(R)_2$, C=O, C=S, C=Se, P (=O) (R), SO, $SO_2$, O, S ou CONR et où un ou plusieurs atomes H peuvent être remplacés par D, F, Cl, Br, I, CN ou $NO_2$, un système cyclique aromatique ou hétéroaromatique ayant 5 à 60 atomes de cycle aromatique, qui peut, dans chaque cas, être substitué par un ou plusieurs radicaux R, un groupe aryloxy ayant 5 à 60 atomes de cycle aromatique, qui peut être substitué par un ou plusieurs radicaux R, ou un groupe aralkyle ou hétéroaralkyle qui a 5 à 60 atomes de cycle aromatique, qui peut être substitué par un ou plusieurs radicaux R.

**6.** Composé selon une ou plusieurs des revendications précédentes, **caractérisé en ce que** $R^A$ désigne, à chaque occurrence, identique ou différent, F, CN, $N(ArN)_2$, C(=O)Ar, $P(=O)(Ar)_2$, S(=O)Ar, $S(=O)_2Ar$, $Si(R)_3$, $B(OR)_2$, $OSO_2R$, un groupe alkyle, alcoxy ou thioalkyle à chaîne linéaire ayant 1 à 40 atomes C ou un groupe alkyle, alcoxy ou thioalkyle ramifié ou cyclique ayant 3 à 40 atomes C, dont chacun peut être substitué par un ou plusieurs radicaux R, où, dans chaque cas, un ou plusieurs groupes $CH_2$ non adjacents peuvent être remplacés par RC=CR, C≡C, $Si(R)_2$, $Ge(R)_2$, $Sn(R)_2$, C=O, C=S, C=Se, P (=O) (R), SO, $SO_2$, O, S ou CONR et où un ou plusieurs atomes H peuvent être remplacés par D, F, Cl, Br, I, CN ou $NO_2$, un système cyclique aromatique ou hétéroaromatique ayant 5 à 60 atomes de cycle aromatique, qui peut, dans chaque cas, être substitué par un ou plusieurs radicaux R, un groupe aryloxy ayant 5 à 60 atomes de cycle aromatique, qui peut être substitué par un ou plusieurs radicaux R, ou un groupe aralkyle ou hétéroaralkyle qui a 5 à 60 atomes de cycle aromatique, qui peut être substitué par un ou plusieurs radicaux R.

**7.** Composé selon une ou plusieurs des revendications précédentes, **caractérisé en ce que** $R^A$ est choisi à chaque occurrence, identique ou différent,

- parmi $N(Ar^N)_2$ ; où $Ar^N$ a la même définition que dans la revendication 1 ;
- parmi des groupes alkyle ramifiés ou cycliques représentés par la formule générale (RS-a) suivante

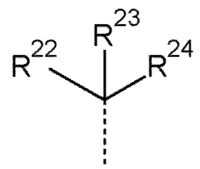

(RS-a)

dans laquelle

$R^{22}$, $R^{23}$, $R^{24}$ sont, à chaque occurrence, identiques ou différents, choisis parmi H, un groupe alkyle à chaîne linéaire ayant 1 à 10 atomes de carbone, ou un groupe alkyle ramifié ou cyclique ayant 3 à 10 atomes de carbone, où les groupes mentionnés ci-dessus peuvent chacun être substitués par un ou plusieurs radicaux $R^{25}$, et où deux des radicaux $R^{22}$, $R^{23}$, $R^{24}$ ou tous les radicaux $R^{22}$, $R^{23}$, $R^{24}$ peuvent être assemblés pour former un groupe alkyle (poly)cyclique, qui peut être substitué par un ou plusieurs radicaux $R^{25}$ ;

$R^{25}$ est, à chaque occurrence, identique ou différent, choisi parmi un groupe alkyle à chaîne linéaire ayant 1 à 10 atomes de carbone, ou un groupe alkyle ramifié ou cyclique ayant 3 à 10 atomes de carbone ;

à condition que, à chaque occurrence, au moins un des radicaux $R^{22}$, $R^{23}$ et $R^{24}$ soit autre que H, à condition que, à chaque occurrence, tous les radicaux $R^{22}$, $R^{23}$ et $R^{24}$ aient conjointement au moins 4 atomes de carbone et à condition que, à chaque occurrence, si deux des radicaux $R^{22}$, $R^{23}$, $R^{24}$ sont H, le radical restant ne soit pas une chaîne linéaire ;

- ou parmi des groupes alcoxy ramifiés ou cycliques représentés par la formule générale (RS-b) suivante

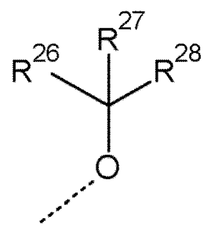

(RS-b)

dans laquelle

$R^{26}$, $R^{27}$, $R^{28}$ sont, à chaque occurrence, identiques ou différents, choisis parmi H, un groupe alkyle à chaîne linéaire ayant 1 à 10 atomes de carbone, ou un groupe alkyle ramifié ou cyclique ayant 3 à 10 atomes de carbone, où les groupes mentionnés ci-dessus peuvent chacun être substitués par un ou plusieurs radicaux $R^{25}$ tels que définis ci-dessus, et où deux des radicaux $R^{26}$, $R^{27}$, $R^{28}$ ou tous les radicaux $R^{26}$, $R^{27}$, $R^{28}$ peuvent être assemblés pour former un groupe alkyle (poly)cyclique, qui peut être substitué par un ou plusieurs radicaux $R^{25}$ tels que définis ci-dessus ;

à condition que, à chaque occurrence, un seul des radicaux $R^{26}$, $R^{27}$ et $R^{28}$ puisse être H ;

- ou parmi des groupes aralkyle représentés par la formule générale (RS-c) suivante

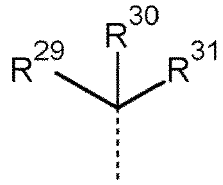

(RS-c)

dans laquelle

$R^{29}$, $R^{30}$, $R^{31}$ sont, à chaque occurrence, identiques ou différents, choisis parmi H, un groupe alkyle à chaîne linéaire ayant 1 à 10 atomes de carbone, ou un groupe alkyle ramifié ou cyclique ayant 3 à 10 atomes de carbone, où les groupes mentionnés ci-dessus peuvent chacun être substitués par un ou plusieurs radicaux $R^{32}$, ou un système cyclique aromatique ayant 6 à 30 atomes de cycle aromatique, qui peut, dans chaque cas, être substitué par un ou plusieurs radicaux $R^{32}$, et où deux ou tous les radicaux $R^{29}$, $R^{30}$, $R^{31}$ peuvent être assemblés pour former un groupe alkyle (poly)cyclique ou un système cyclique aromatique, dont chacun peut être substitué par un ou plusieurs radicaux $R^{32}$ ;

$R^{32}$ est, à chaque occurrence, identique ou différent, choisi parmi un groupe alkyle à chaîne linéaire ayant 1 à 10 atomes de carbone, ou un groupe alkyle ramifié ou cyclique ayant 3 à 10 atomes de carbone, ou un système cyclique aromatique ayant 6 à 24 atomes de cycle aromatique ;

à condition que, à chaque occurrence, au moins un des radicaux $R^{29}$, $R^{30}$ et $R^{31}$ soit autre que H et que, à chaque occurrence, au moins un des radicaux $R^{29}$, $R^{30}$ et $R^{31}$ est ou contient un système cyclique aromatique ayant au moins 6 atomes de cycle aromatique ;

- ou parmi des systèmes cycliques aromatiques représentés par la formule générale (RS-d) suivante

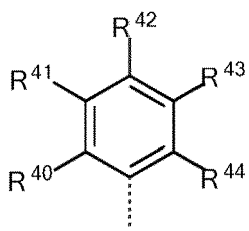

(RS-d)

dans laquelle

$R^{40}$ à $R^{44}$ est, à chaque occurrence, identique ou différent, choisi parmi H, un groupe alkyle à chaîne linéaire ayant 1 à 10 atomes de carbone, ou un groupe alkyle ramifié ou cyclique ayant 3 à 10 atomes de carbone, où les groupes mentionnés ci-dessus peuvent chacun être substitués par un ou plusieurs radicaux $R^{32}$, ou un système cyclique aromatique ayant 6 à 30 atomes de cycle aromatique, qui peut, dans chaque cas, être substitué par un ou plusieurs radicaux $R^{32}$, et où deux ou plus de deux des radicaux $R^{40}$ à $R^{44}$ peuvent être assemblés pour former un groupe alkyle (poly)cyclique ou un système cyclique aromatique, dont chacun peut être substitué par un ou plusieurs radicaux $R^{32}$ tels que définis ci-dessus.

8. Composé selon une ou plusieurs des revendications précédentes, **caractérisé en ce que** $Ar^s$ désigne benzène, biphényle, terphényle, naphtalène, fluorène, indénofluorène, spirobifluorène, triazine, benzoquinoléine, benzoquinazoline, dibenzofurane, dibenzothiophène et carbazole, où chacun des groupes mentionnés ci-dessus peuvent être substitués par un ou plusieurs radicaux R.

9. Composé selon une ou plusieurs des revendications précédentes, **caractérisé en ce que** m = n = 0.

10. Composé selon une ou plusieurs des revendications précédentes, **caractérisé en ce que** p = q = 0.

11. Composé selon une ou plusieurs des revendications précédentes, **caractérisé en ce qu'**il est choisi parmi des composés de formule (2), (3) ou (4),

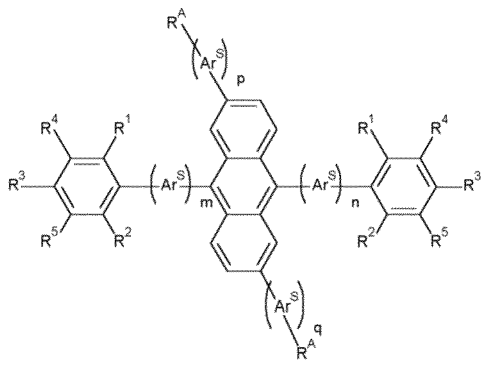

formule (2)

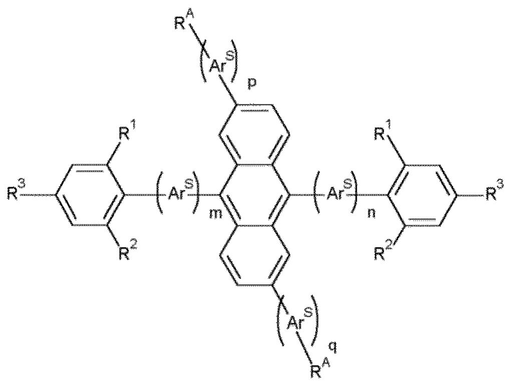

formule (3)

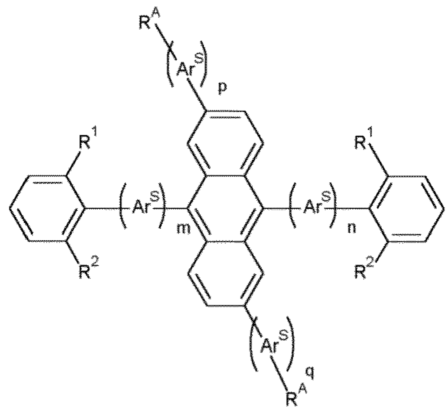

formule (4)

où $R^3$, $R^4$, $R^5$ désignent à chaque occurrence, identiques ou différents, un groupe alkyle, alcoxy ou thioalkyle à chaîne linéaire ayant 1 à 40 atomes C ou un groupe alkyle, alcoxy ou thioalkyle ramifié ou cyclique ayant 3 à 40 atomes C, dont chacun peut être substitué par un ou plusieurs radicaux R, où, dans chaque cas, un ou plusieurs groupes $CH_2$ non adjacents peuvent être remplacés par RC=CR, C≡C, $Si(R)_2$, $Ge(R)_2$, $Sn(R)_2$, C=O, C=S, C=Se, P(=O)(R), SO, $SO_2$, O, S ou CONR et où un ou plusieurs atomes H peuvent être remplacés par D, F, Cl, Br, I, CN ou $NO_2$, un système cyclique aromatique ou hétéroaromatique ayant 5 à 60 atomes de cycle aromatique, qui peut, dans chaque cas, être substitué par un ou plusieurs radicaux R, un groupe aryloxy ayant 5 à 60 atomes de cycle aromatique, qui peut être substitué par un ou plusieurs radicaux R, ou un groupe aralkyle ou hétéroaralkyle qui a 5 à 60 atomes de cycle aromatique, qui peut être substitué par un ou plusieurs radicaux R ; et
où les autres symboles et indices ont la même signification que dans la revendication 1.

**12.** Composé selon une ou plusieurs des revendications précédentes, **caractérisé en ce qu'**il est choisi parmi des composés de formules (2-1a) à (2-4a), (4-1a) à (4-3a),

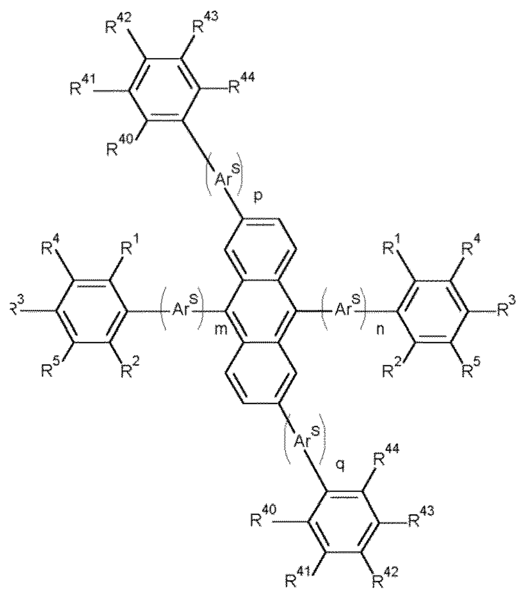

formule (2-1-a)

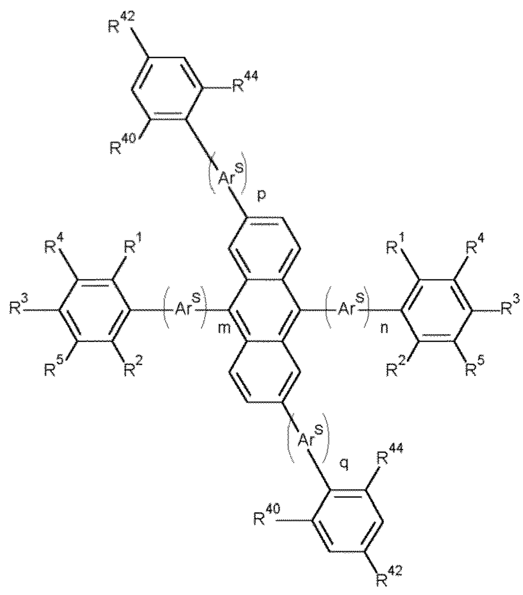

formule (2-2-a)

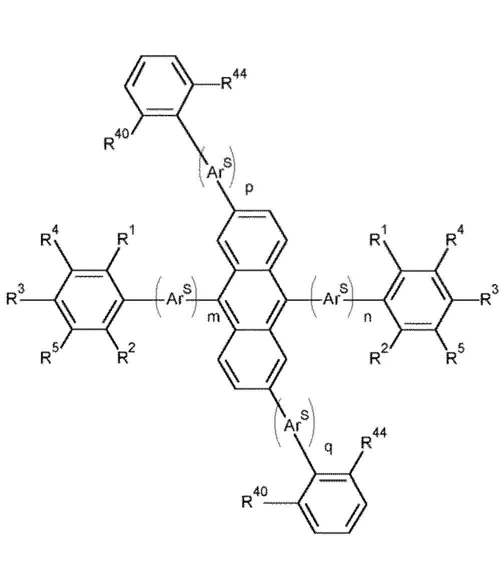

formule (2-3-a)

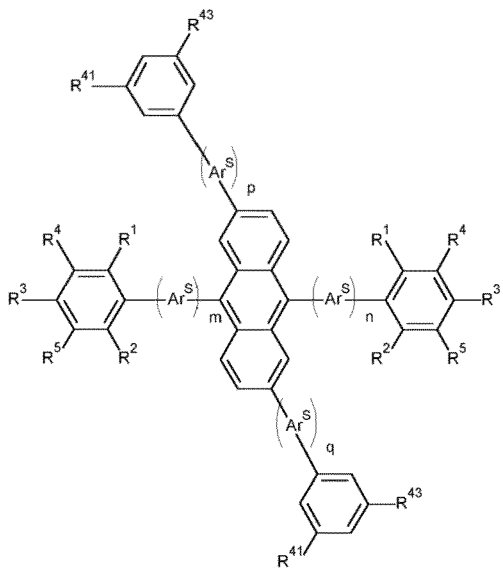

formule (2-4-a)

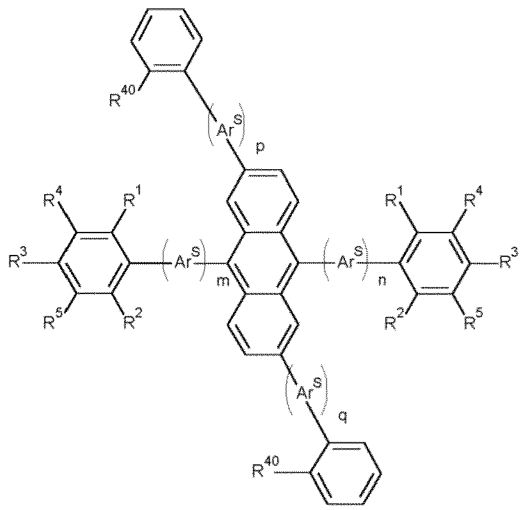

formule (2-5-a)

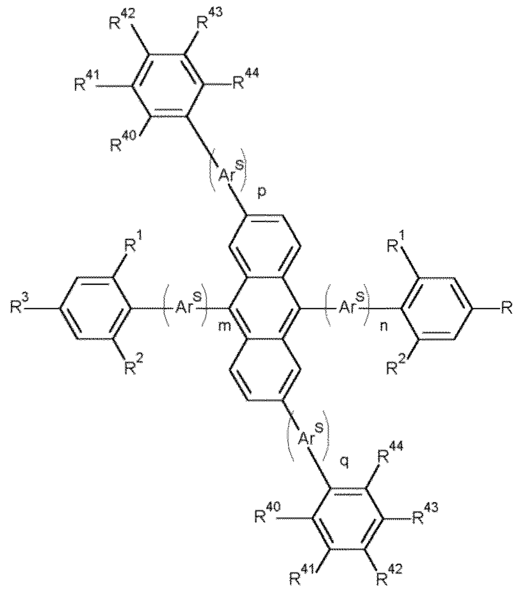

formule (3-1-a)

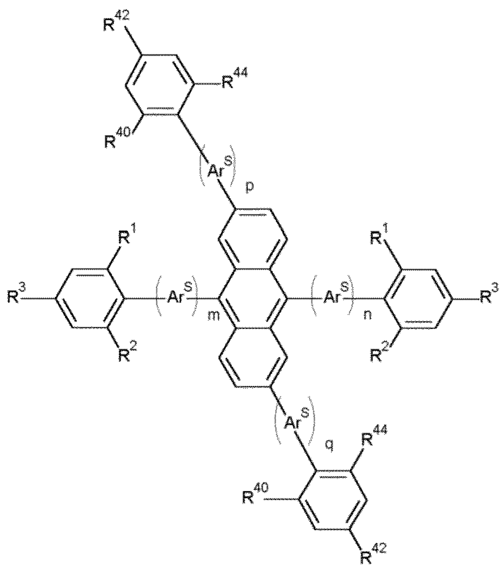

formule (3-2-a)

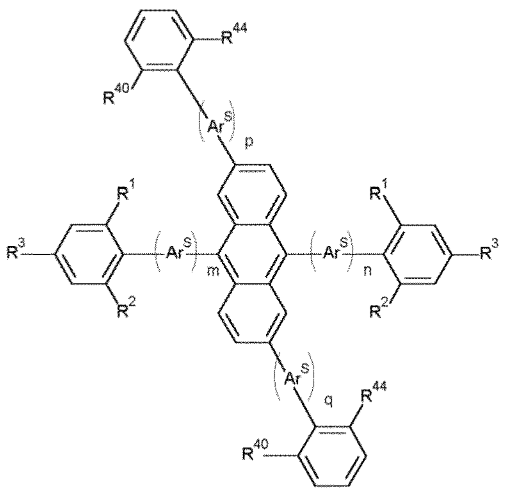

formule (3-3-a)

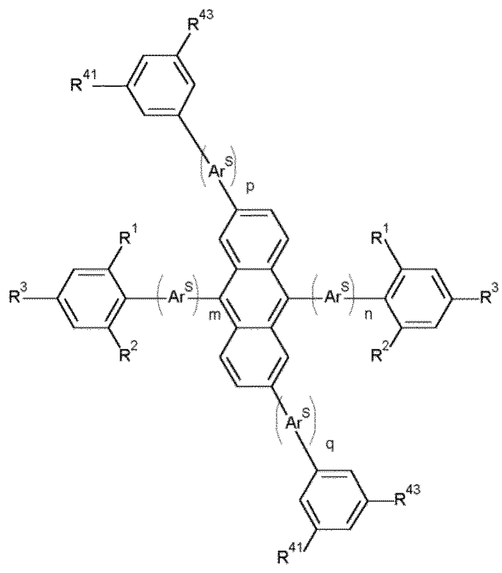

formule (3-4-a)

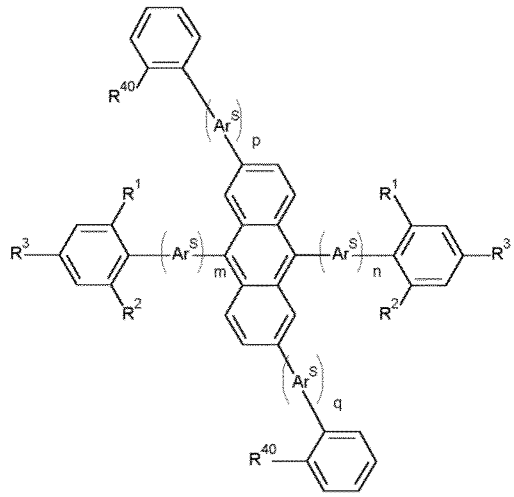

formule (3-5-a)

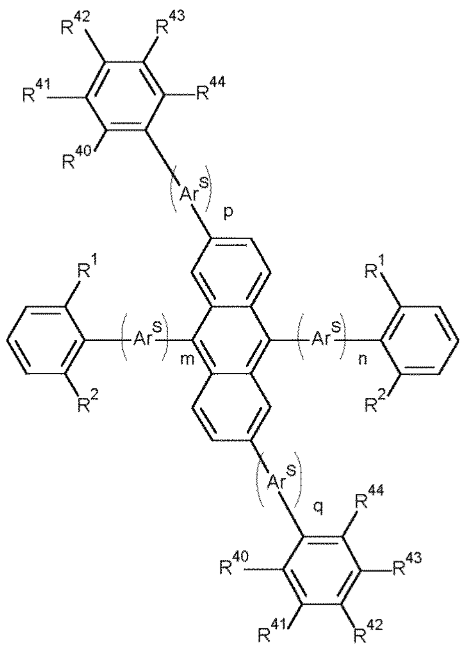

formule (4-1-a)

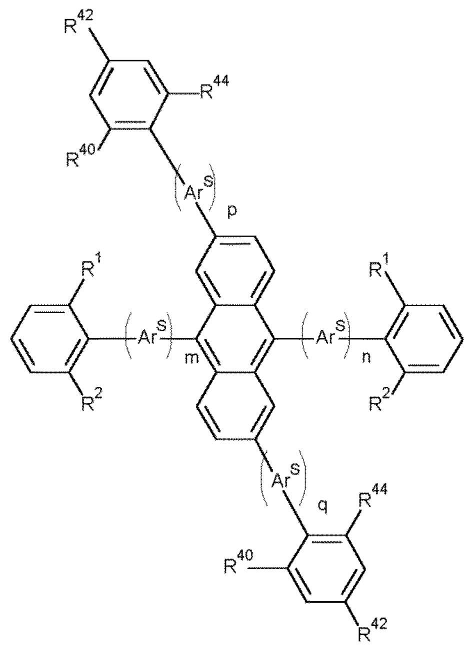

formule (4-2-a)

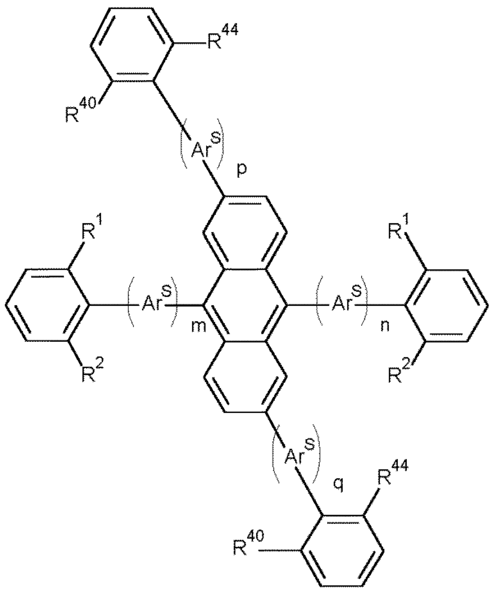

formule (4-3-a)

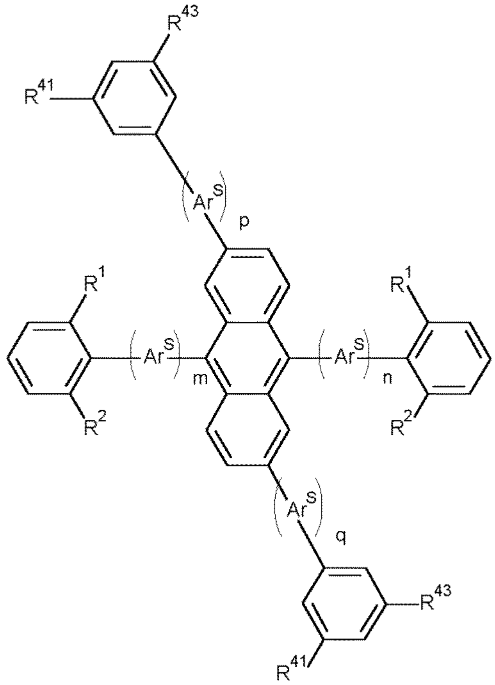

formule (4-4-a)

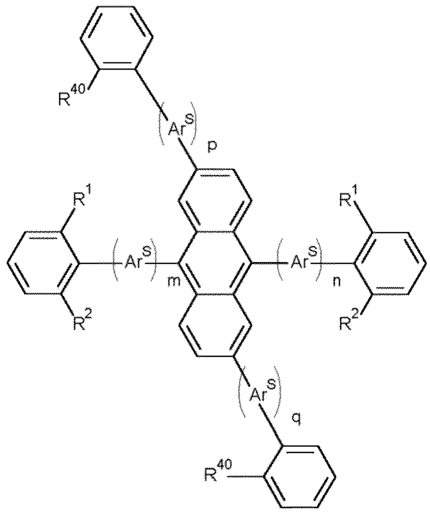

formule (4-5-a)

où

$R^3$, $R^4$, $R^5$ désignent à chaque occurrence, identiques ou différents, un groupe alkyle, alcoxy ou thioalkyle à chaîne linéaire ayant 1 à 40 atomes C ou un groupe alkyle, alcoxy ou thioalkyle ramifié ou cyclique ayant 3 à 40 atomes C, dont chacun peut être substitué par un ou plusieurs radicaux R, où, dans chaque cas, un ou plusieurs groupes $CH_2$ non adjacents peuvent être remplacés par RC=CR, C≡C, $Si(R)_2$, $Ge(R)_2$, $Sn(R)_2$, C=O, C=S, C=Se, P(=O)(R), SO, $SO_2$, O, S ou CONR et où un ou plusieurs atomes H peuvent être remplacés par D, F, Cl, Br, I, CN ou $NO_2$, un système cyclique aromatique ou hétéroaromatique ayant 5 à 60 atomes de cycle aromatique, qui peut, dans chaque cas, être substitué par un ou plusieurs radicaux R, un groupe aryloxy ayant 5 à 60 atomes de cycle aromatique, qui peut être substitué par un ou plusieurs radicaux R, ou un groupe aralkyle ou hétéroaralkyle qui a 5 à 60 atomes de cycle aromatique, qui peut être substitué par un ou plusieurs radicaux R ; $R^{40}$ à $R^{44}$ sont, à chaque occurrence, identiques ou différents, choisis parmi un groupe alkyle à chaîne linéaire ayant 1 à 10 atomes de carbone, ou un groupe alkyle ramifié ou cyclique ayant 3 à 10 atomes de

carbone, où les groupes mentionnés ci-dessus peuvent chacun être substitués par un ou plusieurs radicaux $R^{32}$, ou un système cyclique aromatique ayant 6 à 30 atomes de cycle aromatique, qui peut, dans chaque cas, être substitué par un ou plusieurs radicaux $R^{32}$ ; où $R^{32}$ est tel que défini dans la revendication 6 ;
et où $R^1$, $R^2$, R, m, n, p et q ont la même définition que dans la revendication 1.

**13.** Composé selon la revendication 11, **caractérisé en ce que** $R^{40}$ à $R^{44}$ sont, à chaque occurrence, identiques ou différents, choisis parmi un système cyclique aromatique ayant 6 à 30 atomes de cycle aromatique, qui peut, dans chaque cas, être substitué par un ou plusieurs radicaux $R^{32}$ ; où $R^{32}$ est tel que défini dans la revendication 6.

**14.** Composé selon la revendication 11 ou 12, **caractérisé en ce qu'**il est choisi parmi des composés de formules (2-1-a') à (4-5-a'),

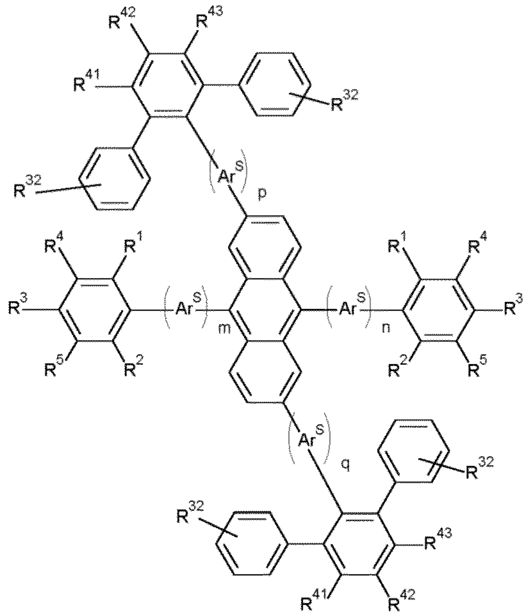

formule (2-1-a´)

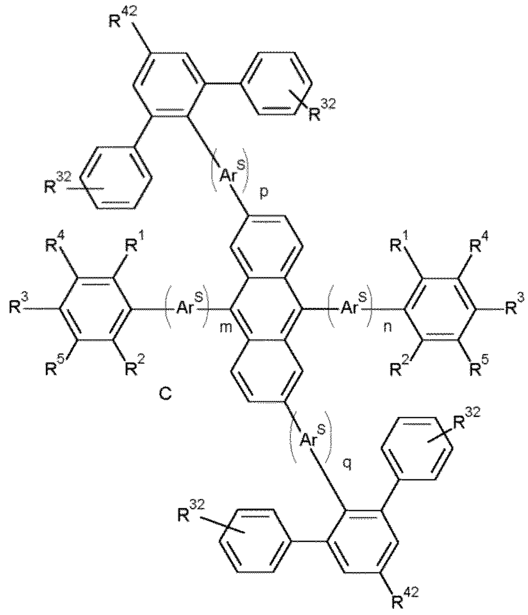

formule (2-2-a´)

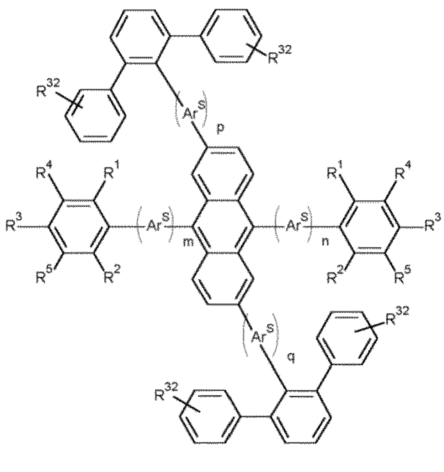

formule (2-3-a´)

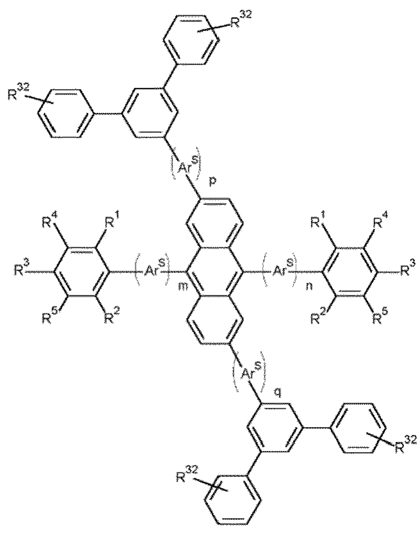

formule (2-4-a´)

formule (2-1-a´) formule (2-2-a´)

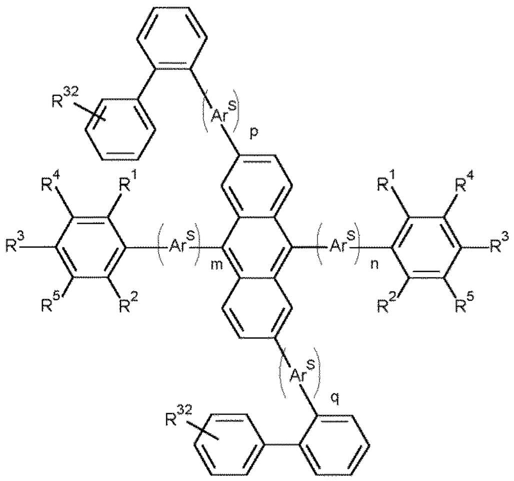

formule (2-5-a´)

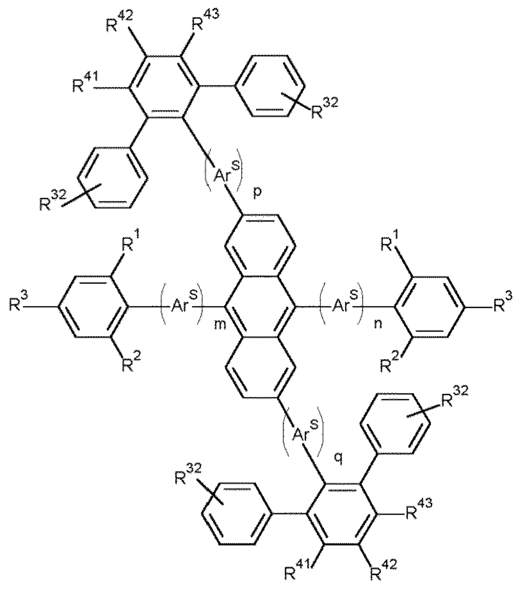

formule (3-1-a´)

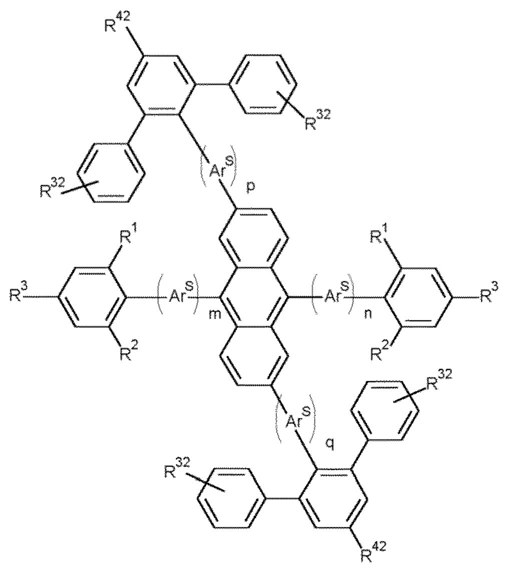

formule (3-2-a´)

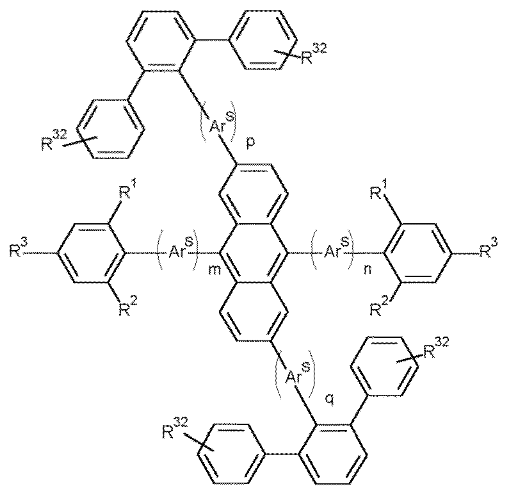

formule (3-3-a´)

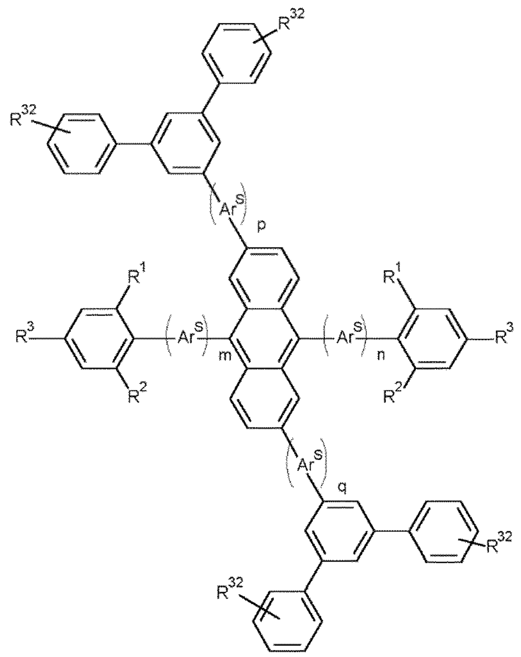

formule (3-4-a´)

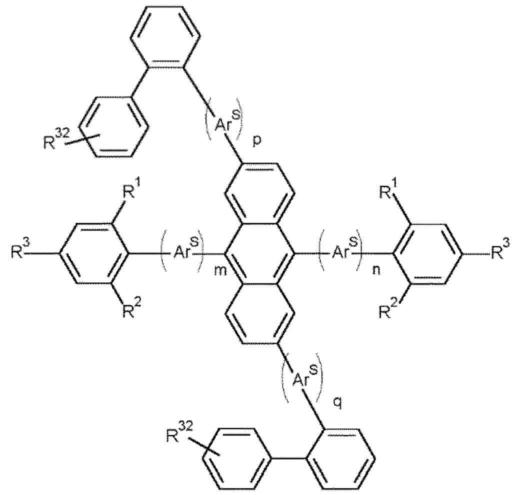

formule (3-5-a´)

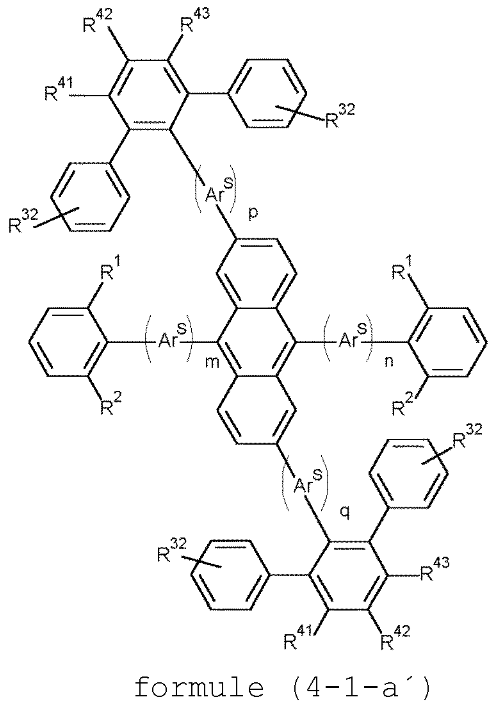

formule (4-1-a´)

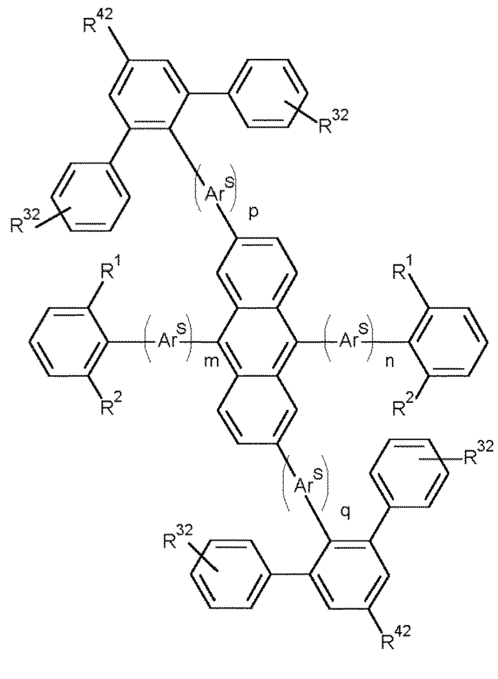

formule (4-2-a´)

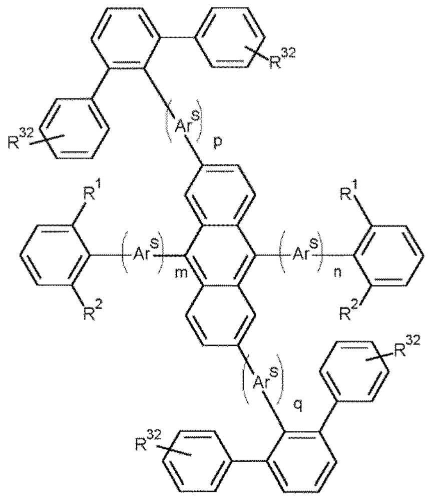

formule (4-3-a´)

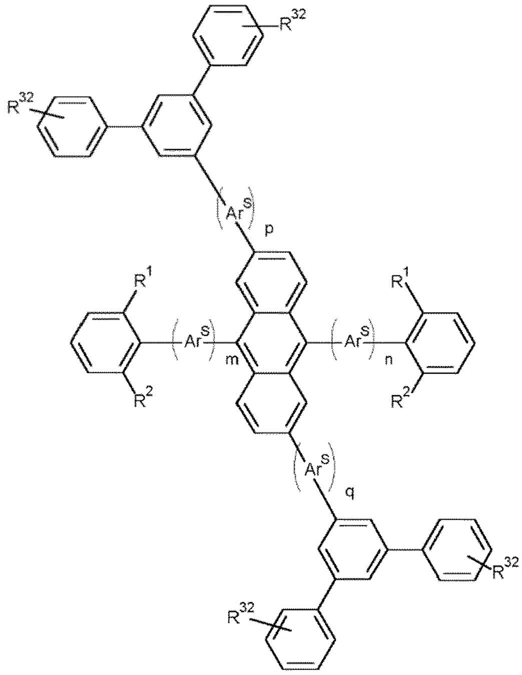

formule (4-4-a´)

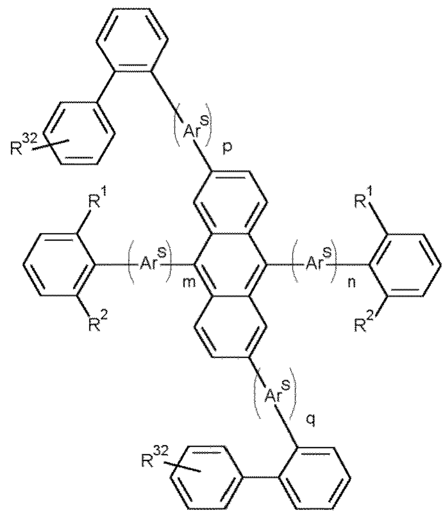

formule (4-5-a´)

où les symboles et indices ont la même signification que dans la revendication 11.

**15.** Composé selon une ou plusieurs des revendications 1 à 10, **caractérisé en ce qu'**il est choisi parmi des composés de formules (2-b) à (4-b),

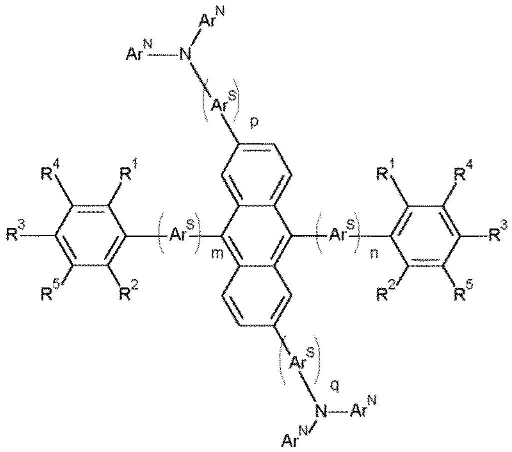

formule (2-b)

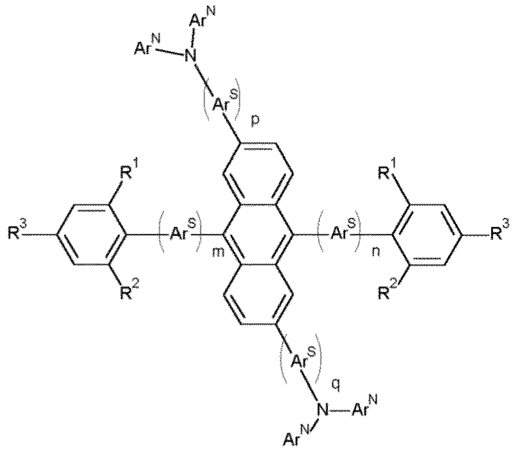

formule (3-b)

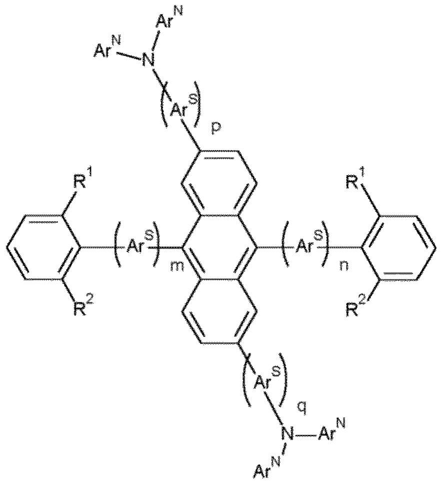

formule (4-b)

où les symboles et indices ont la même signification que dans la revendication 1.

**16.** Polymère, oligomère ou dendrimère contenant un ou plusieurs composés selon une ou plusieurs des revendications 1, où les un ou plusieurs liaisons au polymère, oligomère ou dendrimère peuvent être localisées à des positions quelconques dans la formule (1) qui est substituée par $R^1$ à $R^5$ ou $R^A$.

**17.** Formulation comprenant au moins un composé selon une ou plusieurs des revendications 1 à 15 ou au moins un polymère, oligomère ou dendrimère selon la revendication 16 et au moins un solvant.

**18.** Dispositif électroluminescent organique comprenant une anode, une cathode et au moins une couche organique comprenant au moins un composé selon une ou plusieurs des revendications 1 à 15 ou au moins un polymère, oligomère ou dendrimère selon la revendication 16, **caractérisé en ce que** le composé selon une ou plusieurs des revendications 1 à 15 ou le polymère, oligomère ou dendrimère selon la revendication 16 est utilisé en tant qu'émetteur dans une couche émettrice.

**19.** Dispositif électroluminescent organique selon la revendication 18, **caractérisé en ce que** le composé selon une ou plusieurs des revendications 1 à 15 ou le polymère, oligomère ou dendrimère selon la revendication 16 est utilisé en tant qu'émetteur fluorescent dans une couche émettrice, dans lequel la couche émettrice comprend au moins un composant supplémentaire choisi parmi des matériaux de matrice.

**20.** Dispositif électroluminescent organique selon la revendication 18 ou 19, **caractérisé en ce que** le composé selon

une ou plusieurs des revendications 1 à 15 ou le polymère, oligomère ou dendrimère selon la revendication 16 est utilisé en tant qu'émetteur fluorescent dans une couche émettrice, dans lequel la couche émettrice comprend au moins un composé sensibilisateur choisi parmi des composés qui présentent une fluorescence retardée et parmi des composés phosphorescents.

**21.** Dispositif électroluminescent organique selon la revendication 20, **caractérisé en ce que** la couche émettrice comprend en outre au moins un matériau fonctionnel organique choisi parmi des matériaux de matrice.

**Figure 1: Emission spectra of the compound of example 1 and the compound of the comparative example V-01**

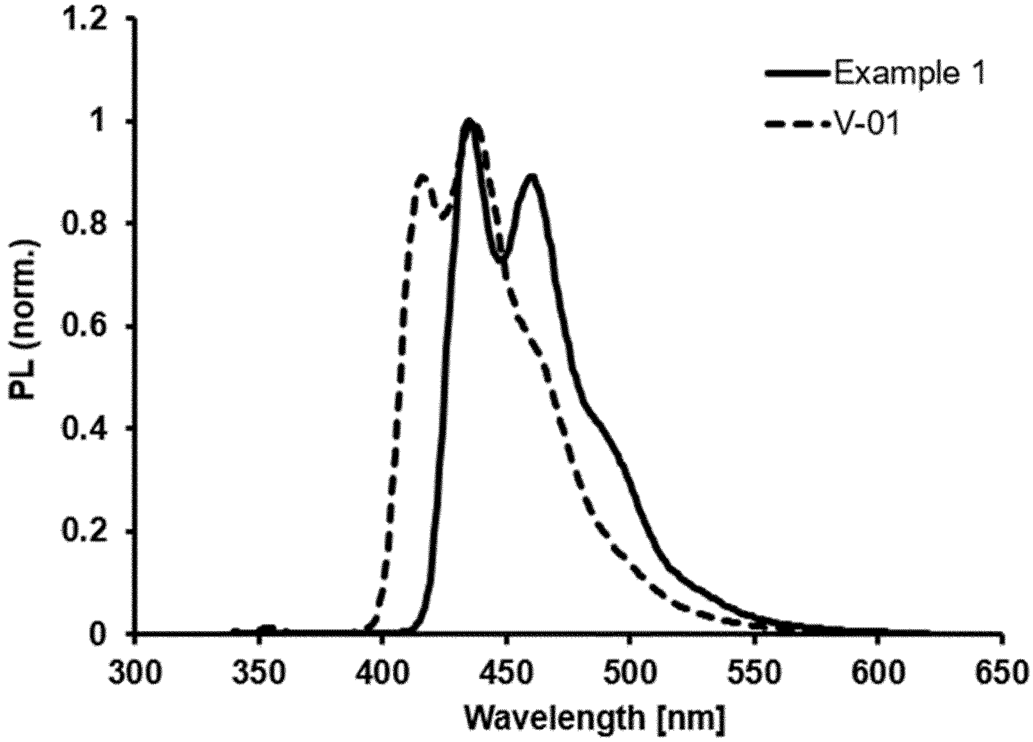

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4539507 A
- WO 2007105917 A1
- US 2009256468 A1
- US 2017200905 A1
- WO 2015135624 A
- WO 2005011013 A
- JP 2000053957 A
- WO 2003060956 A
- WO 2004028217 A
- WO 2004080975 A
- WO 2010072300 A
- WO 06122630 A
- WO 06100896 A
- EP 1661888 A
- WO 01049806 A
- US 5061569 A
- WO 9509147 A
- WO 08006449 A
- WO 07140847 A
- WO 2012034627 A
- WO 2013120577 A
- EP 2875092 A
- EP 2875699 A
- EP 2875004 A
- WO 2013083216 A
- WO 2012150001 A
- WO 2013154064 A
- WO 2013133359 A
- WO 2013161437 A
- WO 2013081088 A
- WO 2013011954 A
- JP 2013116975 A
- US 20120241732 A
- WO 2015091716 A
- WO 200070655 A
- WO 200141512 A
- WO 200202714 A
- WO 200215645 A
- EP 1191612 A
- WO 2005033244 A
- WO 2005019373 A
- US 20050258742 A
- WO 2006056418 A
- WO 2007115970 A
- WO 2007115981 A
- WO 2008000727 A
- WO 2009050281 A
- WO 2009050290 A
- WO 2011051404 A
- WO 2011073149 A
- WO 2012121936 A
- US 20120305894 A
- WO 2012170571 A
- WO 2012170461 A
- WO 2012170463 A
- WO 2006121811 A
- WO 2007095118 A
- WO 2008156879 A
- WO 2010068876 A
- WO 2011106344 A
- WO 2012172482 A
- EP 3126371 A
- WO 2015014835 A
- WO 2015014944 A
- WO 2016020516 A
- US 20160072081 A
- WO 2010086089 A
- WO 2011044988 A
- WO 2014008982 A
- WO 2014023377 A
- WO 2014094961 A
- WO 2010069442 A
- WO 2012163471 A
- WO 2013020631 A
- US 20150243912 A
- WO 2008000726 A
- WO 2010015307 A
- WO 2010054731 A
- WO 2010054728 A
- WO 2010099852 A
- WO 2011032626 A
- WO 2011157339 A
- WO 2012007086 A
- WO 2015036074 A
- WO 2015104045 A
- WO 2015117718 A
- WO 2016015815 A
- WO 2004081017 A
- WO 2005042550 A
- US 20050170206 A
- WO 2009146770 A
- WO 2010102709 A
- WO 2011066898 A
- WO 2016124304 A
- WO 2017032439 A
- WO 2018019688 A
- EP 3184534 A
- WO 2018011186 A

- WO 2011045337 A
- US 20150171350 A
- WO 2016079169 A
- WO 2018019687 A
- WO 2018041769 A
- WO 2018054798 A
- WO 2018069196 A
- WO 2018069197 A
- WO 2018069273 A
- WO 2010031485 A
- US 2013150581 A
- WO 2013017675 A
- WO 2013007707 A
- WO 2013001086 A
- WO 2012156378 A
- WO 2013072508 A
- EP 2543672 A
- WO 2004013080 A
- WO 2004093207 A
- WO 2006005627 A
- WO 2010006680 A
- WO 2005039246 A
- US 20050069729 A
- JP 2004288381 A
- EP 1205527 A
- WO 2008086851 A
- US 20090134784 A
- WO 2007063754 A
- WO 2008056746 A
- WO 2010136109 A
- WO 2011000455 A
- EP 1617710 A
- EP 1617711 A
- EP 1731584 A
- JP 2005347160 A
- WO 2007137725 A
- WO 2005111172 A
- WO 2006117052 A
- WO 2010054729 A
- WO 2010054730 A
- WO 2010015306 A
- EP 652273 A
- WO 2009062578 A
- US 20090136779 A
- WO 2010050778 A
- WO 2011042107 A
- WO 2011088877 A
- EP 1968131 A
- EP 2276085 A
- EP 2213662 A
- EP 1722602 A
- EP 2045848 A
- DE 102007031220
- US 8044390 B
- US 8057712 B
- WO 2009003455 A
- WO 2010094378 A
- WO 2011120709 A
- US 20100096600 A
- US 2010102709 A1
- WO 2015007729 A1

**Non-patent literature cited in the description**


- **J. MATER.** *Chem. C,* 2015, vol. 3, 913
- **H. UOYAMA et al.** *Nature,* 2012, vol. 492, 234
- **ZHAO et al.** Horizontal molecular orientation in solution-processed organic light-emitting diodes. *Appl. Phys. Lett.,* 2015, vol. 106063301
- **D. M. KOLLER et al.** *Nature Photonics,* 2008, vol. 1-4
- **Y. SHIROTA et al.** *Chem. Rev.,* 2007, vol. 107 (4), 953-1010
- **TANAKA et al.** *Chemistry of Materials,* 2013, vol. 25 (18), 3766
- **LEE et al.** *Journal of Materials Chemistry C,* 2013, vol. 1 (30), 4599
- **ZHANG et al.** *Nature Photonics advance online publication,* 2014, vol. 1
- **SEREVICIUS et al.** *Physical Chemistry Chemical Physics,* 2013, vol. 15 (38), 15850
- **LI et al.** *Advanced Materials,* 2013, vol. 25 (24), 3319
- **YOUN LEE et al.** *Applied Physics Letters,* 2012, vol. 101 (9), 093306
- **NISHIMOTO et al.** *Materials Horizons,* 2014, vol. 1, 264
- **VALCHANOV et al.** *Organic Electronics,* 2013, vol. 14 (11), 2727
- **NASU et al.** *ChemComm,* 2013, vol. 49, 10385
- *Chem. Rev.,* 2007, vol. 107, 1233
- **M. S. ARNOLD et al.** *Appl. Phys. Lett.,* 2008, vol. 92, 053301
- *CHEMICAL ABSTRACTS,* 122-39-4
- *CHEMICAL ABSTRACTS,* 479-33-4
- *CHEMICAL ABSTRACTS,* 140-CT